(19) Europäisches Patentamt / European Patent Office / Office européen des brevets

(11) **EP 4 714 444 A1**

(12) **EUROPEAN PATENT APPLICATION**
published in accordance with Art. 153(4) EPC


(43) Date of publication:
**25.03.2026 Bulletin 2026/13**

(21) Application number: **24810168.5**

(22) Date of filing: **28.04.2024**

(51) International Patent Classification (IPC):
***A61K 31/4192*** (2006.01) ***C07D 207/325*** (2006.01)
***C07D 233/60*** (2006.01) ***C07D 249/04*** (2006.01)
***A61P 25/30*** (2006.01) ***A61P 25/28*** (2006.01)
***A61P 25/22*** (2006.01) ***A61P 25/00*** (2006.01)
***A61P 29/00*** (2006.01)

(52) Cooperative Patent Classification (CPC):
**A61K 31/4192; A61P 25/00; A61P 25/22; A61P 25/28; A61P 25/30; A61P 29/00; C07D 207/325; C07D 233/60; C07D 249/04**

(86) International application number:
**PCT/CN2024/090415**

(87) International publication number:
**WO 2024/239921 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.05.2023 CN 202310569199**
**20.10.2023 CN 202311363637**



(71) Applicants:
- **Changchun Institute Of Applied Chemistry Chinese Academy Of Sciences**
  **Changchun, Jilin 130022 (CN)**
- **Shenzhen University**
  **Shenzhen, Guangdong 518000 (CN)**

(72) Inventors:
- **WANG, Xiaohui**
  **Changchun, Jilin 130022 (CN)**
- **JIN, Sha**
  **Changchun, Jilin 130022 (CN)**
- **DOU, Xiubo**
  **Changchun, Jilin 130022 (CN)**

(74) Representative: **Evans, Sophie Elizabeth et al**
**Kilburn & Strode LLP**
**Lacon London**
**84 Theobalds Road**
**London WC1X 8NL (GB)**


(54) **PREPARATION FOR AND USE OF CANNABIDIOL DERIVATIVE DRUGS**


(57) The present invention provides cannabidiol derivatives that are useful for treating opioid addiction, neurodegenerative diseases, and neuroinflammation. The cannabidiol derivatives exhibit low toxicities relative to cannabidiol, are non-addictive, and do not inhibit respiration or damage short-term memory.


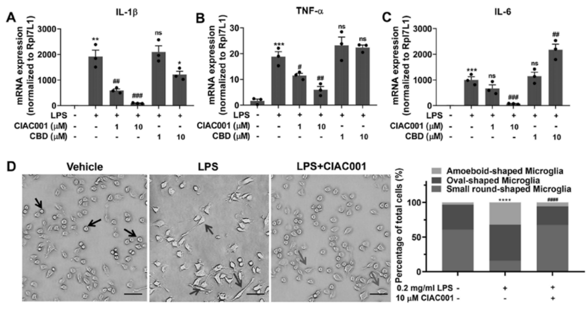


FIG. 1

EP 4 714 444 A1

## Description

## BACKGROUND OF THE INVENTION

### FIELD OF THE INVENTION

The present disclosure generally relates to neurodegenerative and opioid addiction treatments and more specifically to cannabidiol analogues with anti-neuroinflammatory and PKM2 modulator activities useful for such treatments.

### BACKGROUND INFORMATION

Opioids are a class of compounds that encompass psychoactive substances derived from opium poppies and synthetic analogues thereof. Opioid addiction is a chronically relapsing disorder responsible for critical public health problems. Currently, there is a lack of effective drug treatment for opioid addiction. According to data from the World Health Organization, opioids are responsible for 69,000 annual deaths. It is estimated that 15 million people are dependent on opioids. Traditional methods for opioid addiction treatment include medicament maintenance treatments, withdrawal reaction treatments, comprehensive detoxification, and physical therapy. However, each of these treatments has notable limitations.

Medicament maintenance treatments typically involve administration of weak opioid receptor agonists such as methadone or buprenorphine, MOR antagonists such as naltrezone or naloxone, or opium tincture at progressively lowered doses to achieve drug rehabilitation. However, such treatments typically exhibit limited treatment efficacy and pronounced side effects. In particular, it is now well documented that morphine creates neuroinflammation and leads to the release of neuroexcitatory, proinflammatory factors in the CNS, which have been linked to dependence and reward associated with drug abuse. Furthermore, weak opioid receptor agonists themselves are also addictive, making complete recovery challenging for many opioid addicts.

Withdrawal reaction treatments often involve withdrawal induction paired with withdrawal symptom management. Commonly used drugs include clonidine ($\alpha$2-agonist) that can effectively suppress symptoms such as vomiting, diarrhea, elevated blood pressure and accelerated respiration. However, these drugs often also cause additional undesirable effects such as anxiety and insomnia, and are therefore often insufficient for sustaining abstinence from opioid use.

Comprehensive detoxification methods typically utilize scopolamine (an M-blocking agent) to antagonize the vagus nerve, control withdrawal symptoms of addiction to morphine, reduce or reverse morphine tolerance, and promote drug excretion. However, this agent can only suppress physical dependence, namely, withdrawal reactions, caused by morphine, such that comprehensive detoxification methods typically have little to no effect on psychological dependence.

Physical therapy regiments for opioid addiction treatment typically involve exercise, acupuncture, and other physical methods, but generally exhibit limited effects on opioid addiction.

Therefore, there is an unmet need for low side effect treatments for physical and psychological opioid dependence.

## SUMMARY OF THE INVENTION

The present disclosure is based on the seminal discovery of a class of compounds with lower toxicities and side effects but similar anti-neuroinflammatory activity as cannabidiol. While cannabidiol exhibits anti-neuroinflammation activity, this compound also exerts appreciable neurotoxic effects and cognitive impairment, limiting the scope of its therapeutic applications. To address this problem, a function-oriented synthesis strategy was employed to optimize the side chain pharmacophore to improve its therapeutic index. This strategy generated numerous CBD analogues with improved therapeutic indices reflecting high activities and lowered toxicities relative to CBD. For example, an illustrative compound of the invention, Compound 19a (CIAC001), has a therapeutic index that is about 30-fold greater than CBD.

It was further surprisingly determined that these compounds are effective for treating opioid addiction. In particular, animal studies showed that these compounds inhibit chronic morphine-induced inflammation and microglia activation in the medial prefrontal cortex (mPFC), block morphine-induced withdrawal reactions, block morphine-induced behavioral sensitization, lack a rewarding effect that can lead to addiction, and have no apparent activity towards CB1/CB2 receptors.

Leveraging these discoveries, in one embodiment, the present disclosure provides a method of treating or preventing opioid addiction, withdrawal, or overdose in a subject in need thereof comprising administering a compound of Formula I:

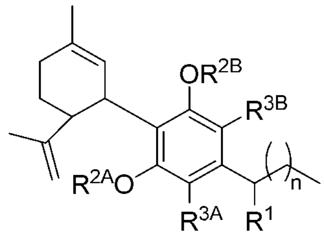

(I)

or a salt or solvate thereof to the subject, wherein:

$R^1$ is selected from -H,

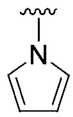 , 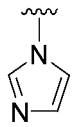 , 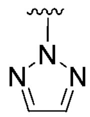 , 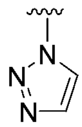 , 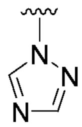 , 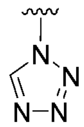 , 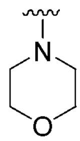 , or 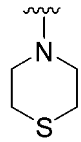 ;

$R^{2A}$ and $R^{2B}$ are each independently selected from -H, $C_1$-$C_4$ alkyl, $C_1$-$C_4$ alkenyl, or $C_1$-$C_4$, wherein $R^{2A}$ and $R^{2B}$ are each optionally substituted by diazirine;

$R^{3A}$ and $R^{3B}$ are each independently selected from -H, halogen, -COOH, or $-NH_2$; and subscript n is 0 to 12; thereby treating or preventing the opioid addiction, withdrawal, or overdose in the subject.

In some aspects, the compound of Formula I is a compound of Formula Ia:

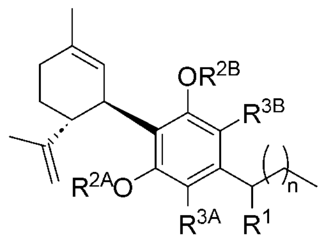

(Ia).

In some aspects, $R^1$ is selected from H,

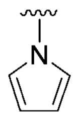 , 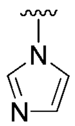 , 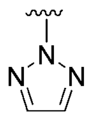 or 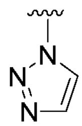 .

In some aspects, $R^1$ is

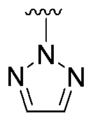 .

In some aspects, $R^{2A}$ and $R^{2B}$ are each independently selected from -H or $C_1$-$C_4$ alkyl. In some aspects, $R^{2A}$ and $R^{2B}$ are each independently selected from -H or $-CH_3$. In some aspects, $R^{2A}$ and $R^{2B}$ are -H. In some aspects, n is 0 or 1. In some aspects, n is 0.

In some aspects, the compound of Formula I is

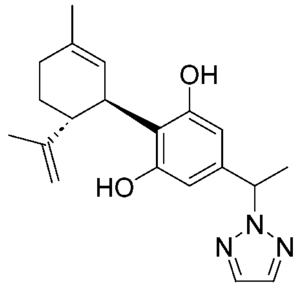

.

In some aspects, the method includes treating or preventing the opioid addiction. In some aspects, the method includes treating or preventing the opioid withdrawal.

In some aspects, the compound of Formula I is administered at a dose of about 0.5 μg/kg, about 2 μg/kg, about 20 μg/kg, about 200 μg/kg, about 2 mg/kg, about 20 mg/kg, or about 100 mg/kg. In some aspects, a $C_{MAX}$ of the compound of Formula I in a brain of the subject is about 1-1000 ng/mg. In some aspects, a brain-to-plasma ratio of the compound of Formula I is at least about 10:1, at least about 25:1, at least about 50:1, at least about 100:1, or at least about 200:1 one hour after the administration.

In some aspects, the opioid is fentanyl, buprenorphine, codeine, dextromoramide, dihydrocodeine, enkephalin, heroin, hydrocodone, hydromorphone, meperidine, methadone, morphine, nicomorphine, opium, oxycodone, oxymorphone, pentazinine, pentazocine, a derivative thereof, a precursor thereof, or pharmacologically acceptable salt or solvate thereof. In particular aspects, the opioid is morphine. In some aspects, the subject is addicted to the opioid.

In some aspects, the subject exhibits a symptom of opioid addiction selected from withdrawal reactions, behavioral sensitization, or conditioned place preference.

In some aspects, the compound of Formula I is administered orally, buccally, sublingually, rectally, vaginally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intrauterineally, intravitreally, transmucosally, or via an inhaler. In some aspects, the compound of Formula I is administered with a pharmaceutically acceptable excipient. In some aspects, the method also includes administering an opioid antagonist to the subject. In particular aspects, the opioid antagonist is naloxone or naltrexone.

In some aspects, the compound of Formula I is administered at a dose sufficient to inhibit opioid induced overexpression of IL-1β, TNFα, IL-6, or a combination thereof in the subject. In some aspects, the compound of Formula I is administered at a dose sufficient to modulate PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, or elongation factor 1-gamma in the subject In some aspects, the compound of Formula I is administered at a dose sufficient to modulate PKM in the subject. In some aspects, the PKM is PKM2. In other aspects, the compound of Formula I is administered at a dose sufficient to increase a ratio of M2 to M1 microglia in a brain of the subject. In some aspects, the compound of Formula I does not cause a toxic stress response in the subject.

In another embodiment, the present disclosure provides a method of modulating PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, elongation factor 1-gamma in a subject in need thereof comprising administering a compound of Formula I:

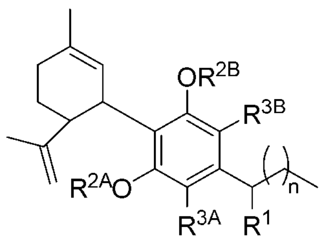

(I)

or a salt or solvate thereof to the subject, wherein:

$R^1$ is selected from -H,

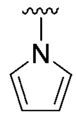 , 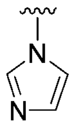 , 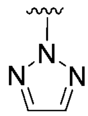 , 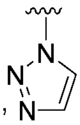 , 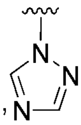 , 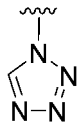 , 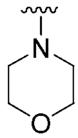 , or 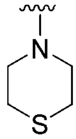 ;

$R^{2A}$ and $R^{2B}$ are each independently selected from-H, $C_1$-$C_4$ alkyl, $C_1$-$C_4$ alkenyl, or $C_1$-$C_4$, wherein $R^{2A}$ and $R^{2B}$ are each optionally substituted by diazirine;

$R^{3A}$ and $R^{3B}$ are each independently selected from -H, halogen, -COOH, or -$NH_2$; and

subscript n is 0 to 12;

thereby modulating PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, elongation factor 1-gamma in the subject.

In some aspects, the method includes modulating PKM in the subject. In particular aspects, the PKM is PKM2.

In a further embodiment, the present disclosure provides a method of increasing a ratio of M2 to M1 microglia in a brain of a subject that includes administering a compound of Formula I to the subject:

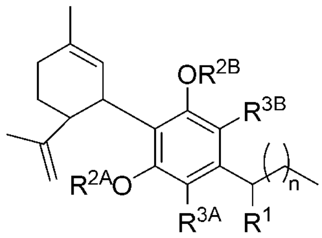

(I)

or a salt or solvate thereof to the subject, wherein:

$R^1$ is selected from -H,

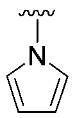 , 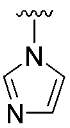 , 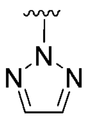 , 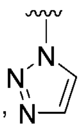 , 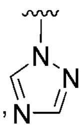 , 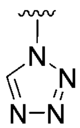 , 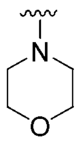 , or 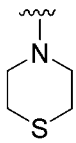 ;

$R^{2A}$ and $R^{2B}$ are each independently selected from-H, $C_1$-$C_4$ alkyl, $C_1$-$C_4$ alkenyl, or $C_1$-$C_4$, wherein $R^{2A}$ and $R^{2B}$ are each optionally substituted by diazirine;

$R^{3A}$ and $R^{3B}$ are each independently selected from -H, halogen, -COOH, or -$NH_2$; and subscript n is 0 to 12;

thereby increasing M2 to M1 microglia ratios in the brain of the subject.

In an additional aspect, the present disclosure provides a method of treating a neurodegenerative disease or disorder in a subject in need thereof comprising administering a compound of Formula I:

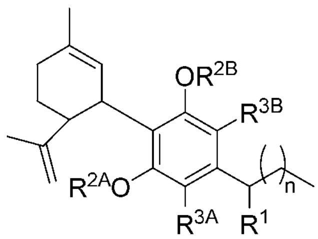

(I)

or a salt or solvate thereof to the subject, wherein:

$R^1$ is selected from -H,

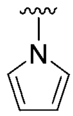, 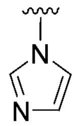, 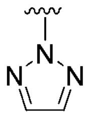, 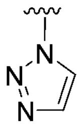, 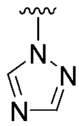, 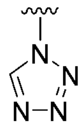, 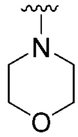, or 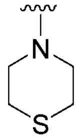;

$R^{2A}$ and $R^{2B}$ are each independently selected from -H, $C_1$-$C_4$ alkyl, $C_1$-$C_4$ alkenyl, or $C_1$-$C_4$, wherein $R^{2A}$ and $R^{2B}$ are each optionally substituted by diazirine;

$R^{3A}$ and $R^{3B}$ are each independently selected from -H, halogen, -COOH, or -$NH_2$; and subscript n is 0 to 12; thereby treating the disease or disorder in the subject.

In certain aspects, the compound of Formula I is a compound of Formula Ia:

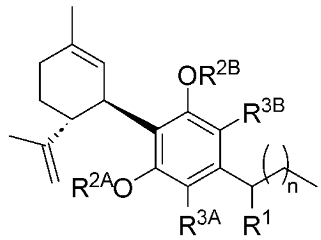

(Ia).

In specific aspects, $R^1$ is selected from H,

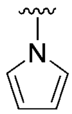, 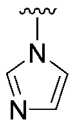, 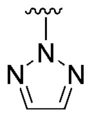 or 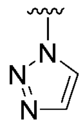.

In select aspects, $R^1$ is

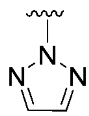.

In certain aspects, $R^{2A}$ and $R^{23}$ are each independently selected from -H or $C_1$-$C_4$ alkyl. In additional aspects, $R^{2A}$ and $R^{2B}$ are each independently selected from -H or -$CH_3$. In further aspects, $R^{2A}$ and $R^{2B}$ are each -H.

In other aspects, $R^{3A}$ and $R^{3B}$ are each independently selected from -H, -Cl,-Br, or -COOH. In additional aspects, $R^{3A}$ and $R^{3B}$ are each -H. In further aspects, n is 0 or 1. In another aspect, n is 0.

In a particular aspect, the compound of Formula I is

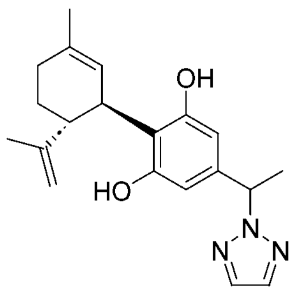.

In certain aspects, the disease or disorder is selected from Alzheimer's disease, Parkinson's disease, or amyotrophic lateral sclerosis. In further aspects, the compound of Formula I inhibits neuroinflammation in the subject. In select aspects, the compound of Formula I has an $IC_{50}$ of at most about 200 μM, at most about 150 μM, at most about 100 μM, at most about 75 μM, at most about 50 μM, at most about 40 μM, at most about 30 μM, at most about 20 μM, at most about 10 μM, at most about 5 μM, at most about 2.5 μM, or at most about 1 μM for inhibition of LPS-induced nitric

oxide overproduction in microglial cells. In specific aspects, the compound of Formula I has a cellular toxicity $IC_{50}$ of at least about 1 μM, at least about 2.5 μM, at least about 5 μM, at least about 10 μM, at least about 20 μM, at least about 30 μM, at least about 40 μM, at least about 50 μM, at least about 75 μM, at least about 100 μM, at least about 150 μM, at least about 200 μM in microglial cells.

In additional aspects, the compound of Formula I is administered at a dose sufficient to diminish expression of IL-1β in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold. In further aspects, the compound of Formula I is administered at a dose sufficient to diminish expression of TNFα in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold. In other aspects, the compound of Formula I is administered at a dose sufficient to diminish expression of IL-6 in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold.

In a specific aspect, the compound of Formula I is administered at a dose of about 0.5 μg/kg, about 2 μg/kg, about 20 μg/kg, about 200 μg/kg, about 2 mg/kg, about 20 mg/kg, or about 100 mg/kg. In another aspect, a $C_{MAX}$ of the compound of Formula I in a brain of the subject is about 1-1000 ng/mg. In a further aspect, a brain-to-plasma ratio of the compound of Formula I is at least about 10:1, at least about 25:1, at least about 50:1, at least about 100:1, or at least about 200:1 one hour after the administration.

In an additional aspect, the compound of Formula I is administered orally, buccally, sublingually, rectally, vaginally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intrauterineally, intravitreally, transmucosally, or via an inhaler. In a certain aspect, the compound of Formula I is administered with a pharmaceutically acceptable excipient.

In a further aspect, the compound of Formula I is administered at a dose sufficient to modulate PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, or elongation factor 1-gamma in the subject. In a particular aspect, the compound of Formula I is administered at a dose sufficient to modulate PKM in the subject. In a specific aspect, the PKM is PKM2.

In another aspect, the compound of Formula I is administered at a dose sufficient to increasing a ratio of M2 to M1 microglia in a brain of the subject. In an additional aspect, the compound of Formula I does not cause a toxic stress response in the subject.

## BRIEF DESCRIPTION OF THE DRAWINGS

**FIGS. 1A-D** show a set of bar graphs of microglia mRNA expression and morphology as well as representative images of microglia. **FIGS. 1A-C** are plot of IL-1β, TNF-α, and IL-6 mRNA expression, respectively, in BV-2 cells treated with varying amounts of LPS, CIAC001, and CBD. **FIG. 1D** is a set of images of vehicle, LPS, and LPS and CIAC001 treated microglial cells, as well as a plot showing the percentages of amoeboid-shaped, oval-shaped, and small round-shaped microglia in each treatment group.

**FIGS. 2A-H** show plots summarizing mouse behavior and expression profiles along with images from microglial stains of mice treated with various combinations of morphine, CIAC001, and CBD. **FIG. 2A** is a plot summarizing morphine withdrawal jump test results. **FIG. 2B** is a plot of behavior sensitization test results during induction phases. **FIG. 2C** is a plot of behavior sensitization test results during expression phase. **FIG. 2D** is a plot of CPP scores. **FIGS. 2E-G** is a plot of IL-1β mRNA expression in mPFC, NAc, and VTA of the mice. **FIG. 2H** is a set of images of microglial marker Iba1 in mPFC, as well as a bar graph summarizing the number of Iba1 positive cells per $mm^2$.

**FIGS. 3A-F** show a set of plots summarizing safety analyses of CIAC001 performed with mice. **FIG. 3A** is a plot of respiratory rates for vehicle, THC, and CIAC001 treated mice. **FIG. 3B** is a plot summarizing the amount of time mice spent in a "new arm" during 2 minute Y-maze tests. **FIG. 3C** is a plot summarizing CPP scores for mice dosed with CIAC001 on the first test day and following 6 days of training. **FIG. 3D** is a plot of locomotor activity for mice dosed with CIAC001 on the first test day of CPP and following 6 days of training in CPP. **FIG. 3E** is a plot of grooming and rubbing frequencies for mice dosed with CIAC001 on the first test day of CPP and following 6 days of training in CPP. **FIG. 3F** is a plot of mouse body weight changes over 7 continuous days of CIAC001 or vehicle administration.

**FIGS. 4A-C** show a schematic for a CIAC001 pull-down assay, images of silve staining of enriched protein based on pull-down probes, and a Venn diagram showing proteins identified by LC-MS/MS from the pull-down assay. **FIG. 4A** is

a schematic of a pull-down assay designed to identify proteins that bind CIAC001. **FIG. 4B** is a silver stain of enriched protein collected from the pull-down assay outlined in **FIG. 4A. FIG. 4C** is a Venn Diagram summarizing proteins collected from the assay outlined in **FIG. 4A** and identified by liquid chromatography, tandem mass spectrometry.

**FIGS. 5A-F** show a series of plots, Western blot results, and a PKM2 structure. **FIG. 5A** is a series of Western blot/pull-down results of PKM2 with Compounds 19e and 19f, as well as a plot of relative PKM2 levels identified *in situ* and *in vitro.* **FIG. 5B** is an image and plot of results from a cellular thermal shift assay characterizing the interaction of endogenous PKM2 with CIAC001. **FIG. 5C** is a set of plots of PKM2 intrinsic fluorescence as a function of CIAC001 concentration. **FIG. 5D** is a structure of PKM2 that shows CIAC001 and Compound 19b binding. **FIG. 5E** is a structure of a PKM2 binding site with CIAC001 bound. **FIG. 5F** is a structure of a PKM2 binding site with Compound 19b bound.

**FIGS. 6A-E** show a set of plots and Western blot results of BV-2 cell expression following treatment with CIAC or Compound 19b. **FIG. 6A** is a plot of BV-2 cell ATP levels following vehicle, CIAC001, or compound 19b treatment. **FIG. 6B** is a set of Western blot results and a plot showing levels of PKM2 tetramers and monomers in cells treated with vehicle, LPS, or LPS and CIAC001. **FIG. 6C** is a set of Western blot results showing PKM2, H3, and GAPDH levels in the cytoplasm and nuclei of cells treated with vehicle, LPS, or LPS and CIAC001. **FIG. 6D** is a plot a relative LD levels in cells treated with vehicle, LPS, LPS and CIAC001, or LPS and Compound 19b. **FIG. 6E** is a set of Western blot results and a plot of HIF-1$\alpha$ levels in cells treated with vehicle, morphine, or morphine and CIAC001.

**FIG. 7** shows a series of bar graphs M1 and M2 phenotype marker mRNA expression in CIAC001 and LPS treated cells.

**FIGS. 8A-F** show a set of illustrations of animal behavior assays. **FIG. 8A** is an illustration of an animal behavior assay for measuring jump number changes in mice treated with naloxone to precipitate withdrawal syndrome and CIAC001. **FIG. 8B** is an illustration of an experimental schedule to interrogate the effects of CIAC001 on morphine-induced behavioral sensitization. **FIG. 8C** is a schematic illustration of a conditional place preference (CPP) test for mice treated with CIAC001. **FIG. 8D** is a timeline of Y-maze, respiratory rate measurement and open field test assays for evaluating the safety of CIAC001. **FIG. 8E** is a schematic illustration of a Y-maze experiment for evaluating short-term memory impairment induced by CIAC001 or THC. **FIG. 8F** is a timeline for a CPP experiment for evaluating the rewarding effect of CIAC001.

**FIGS. 9A-B** show images of microglia stains and a plot summarizing microglia densities in mice administered vehicle, morphine, or morphine with CIAC001. **FIG. 9A** is a set of representative images of vehicle, morphine, and morphine and CIAC001 treated nucleic accumbent (NAc) and ventral tegmental area (VTA) sections showing Iba1 positive microglia. Nuclei were stained with DAPI. **FIG. 9B** is a bar graph summarizing the average number of microglia per $mm^2$ in NAc and VTA of the mice administered vehicle, morphine, or morphine with CIAC001.

**FIG. 10** shows a pair of plots that provide pharmacokinetic profiles of CBD and CIAC001 in brain tissue (left) and plasma (right).

**FIGS. 11A-C** show plots with RMSD (Å) results and H-bond numbers from PKM2 molecular dynamics simulations with and without CIAC001, as well as a molecular dynamics simulation-generated structure of PKM2 bound to CIAC001. **FIG. 11A** is a plot of backbone RMSDs of PKM2 with and without CIAC001 generated from 100 nanosecond simulations. **FIG. 11B** is a plot of the number of hydrogen bonds per frame between dimer AB and dimer CD of PKM2 with and without CIAC001 in the last 50 nanoseconds of 100 nanosecond molecular dynamics simulations. **FIG. 11C** is a molecular dynamics simulation-generated structure of PKM2 bound to CIAC001 showing hydrogen bond interactions between dimer AB and dimer CD.

**FIG. 12** shows a bar graph of relative LDHA mRNA content of BV-2 cells 3 hours after vehicle, LPS, or LPS and CIAC001 treatment.

**FIG. 13** shows a set of plots of concentration-effect curves of CIAC001 and CBD on forskolin-induced cAMP accumulation in HEK293 cells transfected with CB1 (left) or CB2 (right).

**FIGS. 14A-D** show plots of memory and learning assays on wild-type and Alzheimer's disease model mice. **FIG. 14A** is a plot of Morris water maze results on mice administered vehicle or CBD. **FIG. 14B** is a plot of Morris water maze results on mice administered vehicle or CIAC001. **FIG. 14C** is a plot of Y-maze test results on mice administered vehicle or CBD. **FIG. 14C** is a plot of Y-maze test results on mice administered vehicle or CIAC001.

**FIGS. 15A-E** show plots of anxiety assays on wild-type and Alzheimer's disease model mice administered vehicle or CIAC001. **FIG. 15A** is a plot of high plus maze test results summarizing the number of times mice entered a new arm. **FIG. 15B** is a plot of high plus maze test results summarizing the amount of time mice spent in an open arm. **FIG. 15C** is a plot of open field assay results summarizing the number of grids traversed by mice in each treatment group. **FIG. 15D** is a plot of open field assay results summarizing the amount of time mice in each treatment group spent standing. **FIG. 15E** is a plot of open field assay results summarizing the number of excretions by mice in each treatment group.

**FIGS. 16A-F** are a set of plots and Western blot results on Alzheimer's disease mouse models administered varying concentrations of CIAC001. **FIG. 16A** is a plot of *in vivo* potential of hippocampus measurements. **FIG. 16B** is a bar graph that summarizes the *in vivo* potential of hippocampus measurements displayed in **FIG. 16A. FIG. 16C** is a set of Western blot results that show Tau phosphorylation in the various treatment groups. **FIG. 16D** is a set of Western blot

results that show TrkB phosphorylation, Syn1, and Aβ levels in the treatment groups. **FIG. 16E** is a plot of gray value quantification values and ratios of Tau5, p-Tau (T231), p-Tau (S202), and p-Tau (S404) proteins. **FIG. 16F** is a plot of gray value quantification values and ratios of p-TrkB (Y705), Syn1, Aβ1-42 and Aβ1-16 proteins.

## DETAILED DESCRIPTION OF THE INVENTION

Before the present compounds, compositions and methods are described, it is to be understood that this invention is not limited to particular compounds, compositions, methods, and experimental conditions described, as such compounds, compositions, methods, and conditions may vary. It is also to be understood that the terminology used herein is for purposes of describing particular embodiments only, and is not intended to be limiting, since the scope of the present invention will be limited only in the appended claims.

All publications, patents, and patent applications mentioned in this specification are herein incorporated by reference to the same extent as if each individual publication, patent, or patent application was specifically and individually indicated to be incorporated by reference.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, it will be understood that modifications and variations are encompassed within the spirit and scope of the instant disclosure. The preferred methods and materials are now described.

The present invention is based on the seminal discovery of cannabidiol derivatives that are effective for treating neurodegenerative disorders and opioid addiction. These cannabidiol derivatives are non-addictive, do not inhibit respiration or damage short-term memory, and have minor side effects and toxicities, enabling safe use for a range of subjects and conditions.

In one embodiment, the present disclosure provides a method of treating or preventing opioid addiction, withdrawal, or overdose in a subject in need thereof comprising administering a compound of Formula I:

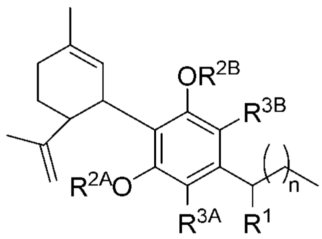

(I)

or a salt or solvate thereof to the subject, wherein:

$R^1$ is selected from -H,

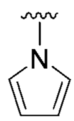 , 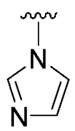 , 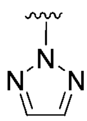 , 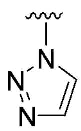 , 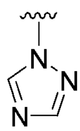 , 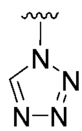 , 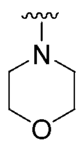 , or 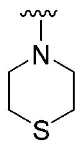 ;

$R^{2A}$ and $R^{2B}$ are each independently selected from -H, $C_1$-$C_4$ alkyl, $C_1$-$C_4$ alkenyl, or $C_1$-$C_4$, wherein $R^{2A}$ and $R^{2B}$ are each optionally substituted by diazirine;

$R^{3A}$ and $R^{3B}$ are each independently selected from -H, halogen, -COOH, or $-NH_2$; and subscript n is 0 to 12; thereby treating or preventing the opioid addiction, withdrawal, or overdose in the subject.

In one aspect, the compound of Formula I is a compound of Formula Ia:

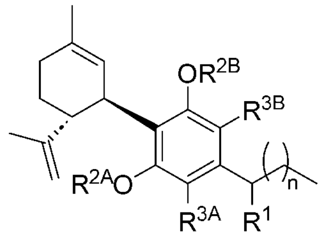

(Ia).

In another aspect, $R^1$ is selected from H,

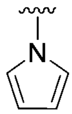 , 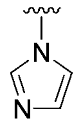 , 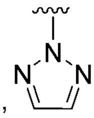 or 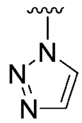 .

In some aspects, $R^1$ is

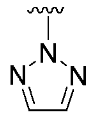 .

In some aspects, $R^{2A}$ and $R^{2B}$ are each independently selected from -H or $C_1$-$C_4$ alkyl. In other aspects, $R^{2A}$ and $R^{2B}$ are each independently selected from -H or $-CH_3$. In further aspects, $R^{2A}$ and $R^{2B}$ are -H. In some aspects, n is 0 or 1. In other aspects, n is 0.

In particular aspects, the compound of Formula I is

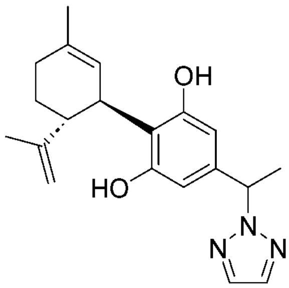 .

In some aspects, the method includes treating or preventing the opioid addiction. In other aspects, the method includes treating or preventing the opioid withdrawal. Opioid withdrawal, one of the critical syndromes of opioid addiction, is a key challenge for preventing drug cravings and preventing relapse. While medications such as clonidine are commonly employed to combat symptoms of opioid withdrawal, these drugs also exhibit acute side effects and have limited effects on residual opioid addiction. Contrasting these medications, the compounds of the present disclosure exhibit low side effects and pronounced efficacies for treating opioid addiction, and accordingly provide a desirable alternative to existing treatments for opioid withdrawal. The compounds of the present disclosure may be used to prevent and/or treat opioid withdrawal symptoms, and are suitable for administration prior to, during, or subsequently to the onset of opioid withdrawal. In many aspects, the subject has opioid withdrawal syndrome. In certain aspects, the subject has acute opioid withdrawal syndrome, wherein the low toxicities of the compounds of the present disclosure optionally enable high doses to counteract severe withdrawal symptoms.

Compounds of Formula I are suitable for administration at a broad range of doses. For example, a compound of Formula I can be administered at a dose of about 0.5 μg/kg, about 2 μg/kg, about 20 μg/kg, about 200 μg/kg, about 2 mg/kg, about 20 mg/kg, or about 100 mg/kg. In particular aspects disclosed herein, the compound of Formula I is administered at a dose of about 0.5 mg/kg to 50 mg/kg, about 0.5 to 10 mg/kg, about 5 to 50 mg/kg, or about 10 to 100 mg/kg. In other aspects, the compound of Formula I is administered at a dose of about 20 μg/kg to 500 μg/kg, about 20 μg/kg to 200 μg/kg, about 100 μg/kg to 500 μg/kg, or about 250 μg/kg to 1 mg/kg. The compound of Formula I can be administered multiple times daily (e.g., twice, three times, four times, five times, or six times daily), on a daily basis, on a weekly basis, on a monthly basis, or at any frequency in between (e.g., once every two, three, or four days). As it was surprisingly shown herein that certain compounds of Formula I have brain and plasma half-lives of about 1 hour, in

particular embodiments, the compound of Formula I is administered between one and twelve, between one and six, or between two and four times daily.

In some aspects, a $C_{MAX}$ of the compound of Formula I in a brain of the subject is about 1-1000 ng/mg. In some aspects, a brain-to-plasma ratio of the compound of Formula I is at least about 10:1, at least about 25:1, at least about 50:1, at least about 100:1, or at least about 200:1 one hour after the administration.

The compounds of the present disclosure are suitable for addiction and withdrawal treatment for a wide range of opioids. As non-limiting examples, in some aspects, the opioid is fentanyl, buprenorphine, codeine, dextromoramide, dihydrocodeine, enkephalin, heroin, hydrocodone, hydromorphone, meperidine, methadone, morphine, nicomorphine, opium, oxycodone, oxymorphone, pentazinine, pentazocine, a derivative thereof, a precursor thereof, or pharmacologically acceptable salt or solvate thereof. In particular aspects, the opioid is morphine. In particular aspects, the opioid is heroin. In further aspects, the opioid is fentanyl. In numerous aspects disclosed herein, the subject is addicted to the opioid. In specific aspects, the subject exhibits a symptom of opioid addiction selected from withdrawal reactions, behavioral sensitization, or conditioned place preference.

Compounds of Formula I are amenable to a range of administration routes. As non-limiting examples, these compounds can be administered orally, buccally, sublingually, rectally, vaginally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intrauterineally, intravitreally, transmucosally, or via an inhaler.

In some aspects, the compound of Formula I is administered with a pharmaceutically acceptable excipient, carrier, and/or stabilizer. Examples of such ingredients are described in detail in Remington's Pharmaceutical Sciences, 18th Edition (1990). Pharmaceutically acceptable carriers, excipients, or stabilizers may include buffers such as phosphate, acetate, and maleate; antioxidants such as ascorbic acid, ascorbyl palmitate, citric acid, methionine, tartaric acid, and vitamin E; preservatives such as octadecyldimethylbenzyl ammonium chloride, hexamethonium chloride, benzalkonium chloride, benzethonium chloride, and phenol; alkyl parabens such as methyl and propyl paraben; resorcinol; low molecular weight polypeptides (e.g., peptides with about 10 or fewer amino acid residues); proteins such as serum albumin and collagen; amino acids such as glycine, glutamate, and lysine; monosaccharides, disaccharides, and other carbohydrates including glucose, mannose, or dextrins; chelating agents such as EDTA; hydrophilic polymers such as polyvinylpyrrolidone and polyethylene glycol; sugars such as sucrose, mannitol, galactose, and trehalose; and/or non-ionic surfactants such as glyceryl monostearate and sorbitan monopalmitate. Examples of pharmaceutically acceptable carriers include, but are not limited to ointments, micellular compositions, colloids, creams, gels, and emulsions. Examples of diluents include, but are not limited to, water, vegetable oils (such as soybean, sunflower, and palm oils), animal fats (such as fish oil and tallow), milk fats, and organic solvents (such as dimethyl sulfoxide).

In some aspects, the method also includes administering an opioid antagonist to the subject. In particular aspects, the opioid antagonist is naloxone or naltrexone. Without being bound by theory, the opioid addiction and withdrawal treatment efficacies of the compounds of the present disclosure can enable these compounds can act synergistically with the opioid antagonist to diminish the symptoms of opioid withdrawal and can lower dosing requirements for the opioid antagonist.

While cannabidiol exhibits anti-neuroinflammatory activity, its use for this purpose, including in the context of opioid treatment, is limited by its toxicity. Contrasting cannabidiol, the compounds of the present contrast cannabinoids by exhibiting low toxicities and anti-neuroinflammatory activities. Accordingly, in many aspects, the compound of Formula I does not cause a toxic stress response in the subject.

Similarly, the compound of Formula I can be administered at a dose sufficient to inhibit opioid induced overexpression of IL-1$\beta$, TNF$\alpha$, IL-6, or a combination thereof in the subject (e.g., to a degree not achievable with cannabidiol). The compound of Formula I can also be administered at a dose sufficient to modulate PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, or elongation factor 1-gamma in the subject In particular aspects, the compound of Formula I is administered at a dose sufficient to modulate PKM in the subject. In some such aspects, the PKM is PKM2. In further aspects, the compound of Formula I is administered at a dose sufficient to increase a ratio of M2 to M1 microglia in a brain of the subject.

As used herein, the term "modulate" refers to increasing or decreasing an activity of a target. For example, in reference to a receptor protein, 'modulate' can denote an increase or decrease in one or more of signaling activity, cognate ligand binding affinity, ligand binding kinetics, or coreceptor binding. In reference to an enzyme, 'modulate' can denote an increase or decrease in enzymatic activity. In some aspects, 'modulate' denotes an increase in activity (e.g., agonist behavior by a compound of the present disclosure). In other aspects, 'modulate' denotes a decrease in activity (e.g., antagonist behavior by a compound of the present disclosure).

In another embodiment, the present disclosure provides a method of modulating PKM, 60 kDa heat shock protein, alpha-enolase, elongation factor 1-1 alpha, RNA helicase, heat shock protein 90-beta, ubiquitin carboxyl-terminal

hydrolase, T-complex protein 1 subunit, alpha, phosphoglycerate kinase 1, elongation factor Tu serine, elongation factor Tu cysteine, peptidase inhibitor, clade B member 6a, elongation factor 1-gamma in a subject in need thereof comprising administering a compound of Formula I:

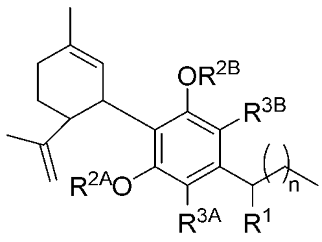

(I)

or a salt or solvate thereof to the subject, wherein:

$R^1$ is selected from -H,

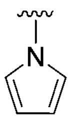 , 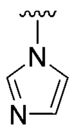 , 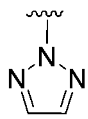 , 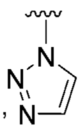 , 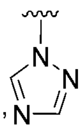 , 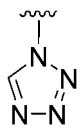 , 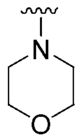 , or 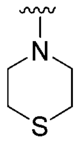 ;

$R^{2A}$ and $R^{2B}$ are each independently selected from-H, $C_1$-$C_4$ alkyl, $C_1$-$C_4$ alkenyl, or $C_1$-$C_4$, wherein $R^{2A}$ and $R^{2B}$ are each optionally substituted by diazirine;
$R^{3A}$ and $R^{3B}$ are each independently selected from -H, halogen, -COOH, or -$NH_2$; and subscript n is 0 to 12;
thereby modulating PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, elongation factor 1-gamma in the subject.

The compound of Formula I is as defined herein. For example, in some aspects, $R^1$ is

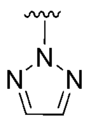
.

In particular aspects, the method includes modulating PKM in the subject. In some such aspects, the PKM is PKM2.

It was further surprisingly demonstrated herein that the compounds of the present disclosure are effective for modulating pyruvate kinase M2 (PKM2). PKM2 is a glycolysis rate-limiting enzyme and transcriptional coactivator distributed in tissues such as brain and liver, and plays an essential role in metabolic homeostasis and inflammation. PKM2 interacts with transcription factors such as STAT3,15 Hif-1$\alpha$, and ATF2, and promotes the production of IL-1$\beta$ and other proinflammatory factors. Therefore, the use of compounds of the present disclosure to target PKM2 is a novel strategy for treating a range of conditions and disorders, including neurodegeneration, neuroinflammation, and opioid use disorder.

In a further aspect, the present disclosure provides a method of increasing a ratio of M2 to M1 microglia in a brain of a subject that includes administering a compound of Formula I to the subject:

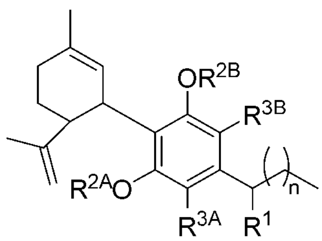

(I)

or a salt or solvate thereof to the subject, wherein:

$R^1$ is selected from -H,

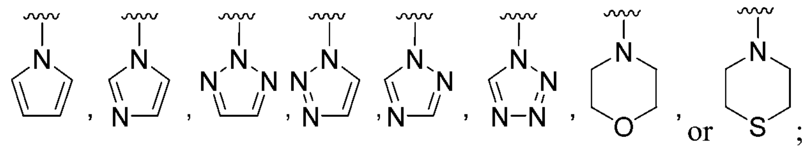

, , , , , , , or ;

$R^{2A}$ and $R^{2B}$ are each independently selected from-H, $C_1$-$C_4$ alkyl, $C_1$-$C_4$ alkenyl, or $C_1$-$C_4$, wherein $R^{2A}$ and $R^{2B}$ are each optionally substituted by diazirine;
$R^{3A}$ and $R^{3B}$ are each independently selected from -H, halogen, -COOH, or $-NH_2$; and subscript n is 0 to 12;
thereby increasing M2 to M1 microglia ratios in the brain of the subject.

The compound of Formula I can be as defined herein (e.g., n can be 0 as detailed above).

In an additional aspect, the present disclosure provides a method of treating a neurodegenerative disease or disorder in a subject in need thereof comprising administering a compound of Formula I:

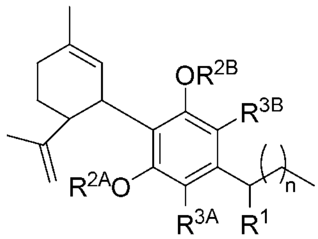

(I)

or a salt or solvate thereof to the subject, wherein:

$R^1$ is selected from -H,

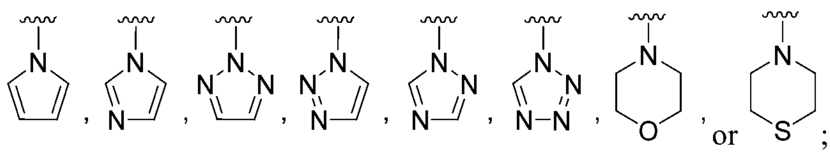

, , , , , , , or ;

$R^{2A}$ and $R^{2B}$ are each independently selected from -H, $C_1$-$C_4$ alkyl, $C_1$-$C_4$ alkenyl, or $C_1$-$C_4$, wherein $R^{2A}$ and $R^{2B}$ are each optionally substituted by diazirine;
$R^{3A}$ and $R^{3B}$ are each independently selected from -H, halogen, -COOH, or $-NH_2$; and subscript n is 0 to 12;
thereby treating the disease or disorder in the subject.

In certain aspects, the compound of Formula I is a compound of Formula Ia:

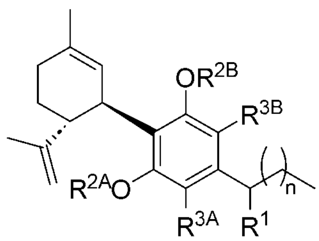

(Ia).

In specific aspects, $R^1$ is selected from H,

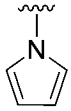 , 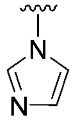 , 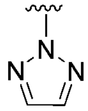 or 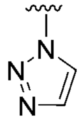 .

In select aspects, $R^1$ is

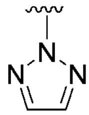 .

In certain aspects, $R^{2A}$ and $R^{2B}$ are each independently selected from -H or $C_1$-$C_4$ alkyl. In additional aspects, $R^{2A}$ and $R^{2B}$ are each independently selected from -H or $-CH_3$. In further aspects, $R^{2A}$ and $R^{2B}$ are each -H.

In other aspects, $R^{3A}$ and $R^{3B}$ are each independently selected from -H, -Cl,-Br, or -COOH. In additional aspects, $R^{3A}$ and $R^{3B}$ are each -H. In further aspects, n is 0 or 1. In another aspect, n is 0.

In a particular aspect, the compound of Formula I is

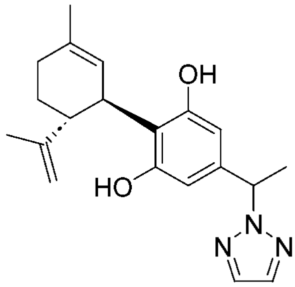 .

The compounds of the present disclosure exhibit improved pharmacokinetics (in particular increased half-lives and brain-to-plasma ratios), lower side effects (including diminished cognitive impairment), decreased toxicities, and similar anti-neuroinflammatory activities relative to cannabidiol. While cannabidiol exhibits anti-neuroinflammatory activity, its toxicity prevents its use in treating neurodegenerative disorders. It was surprisingly discovered herein that shortening and functionalizing the CBD side chain diminishes CBD side effects (e.g., cognitive impairment) and toxicity without decreasing its anti-neuroinflammatory activity, enabling treatment of a wide range of neurodegenerative conditions not previously treatable, including Alzheimer's disease, Parkinson's disease, and amyotrophic lateral sclerosis.

In many aspects, the compound of Formula I inhibits neuroinflammation in the subject. For example, the compound of Formula I can have an $IC_{50}$ of at most about 200 μM, at most about 150 μM, at most about 100 μM, at most about 75 μM, at most about 50 μM, at most about 40 μM, at most about 30 μM, at most about 20 μM, at most about 10 μM, at most about 5 μM, at most about 2.5 μM, or at most about 1 μM for inhibition of LPS-induced nitric oxide overproduction in microglial cells.

The compound of Formula I can also have a low toxicity. For example, the compound of Formula I can have a cellular toxicity $IC_{50}$ of at least about 1 μM, at least about 2.5 μM, at least about 5 μM, at least about 10 μM, at least about 20 μM, at least about 30 μM, at least about 40 μM, at least about 50 μM, at least about 75 μM, at least about 100 μM, at least about 150 μM, at least about 200 μM in microglial cells.

In many aspects, the compound of Formula I diminishes a level of a proinflammatory marker in the subject. In particular, data disclosed herein show that compounds of Formula I are effective for diminishing microglial expression of IL-1β, TNFα, and IL-6, three cytokines that have pronounced effects in promoting inflammation and advancing the pathologies of neurodegenerative diseases. Accordingly, in certain aspects, the compound of Formula I is administered at a dose sufficient to diminish expression of IL-1β in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold. In further aspects, the compound of Formula I is administered at a dose sufficient to diminish expression of TNFα in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold. In additional aspects, the compound of Formula I is administered at a dose sufficient to diminish expression of IL-6 in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold.

In a specific aspect, the compound of Formula I is administered at a dose of about 0.5 μg/kg, about 2 μg/kg, about 20 μg/kg, about 200 μg/kg, about 2 mg/kg, about 20 mg/kg, or about 100 mg/kg. In another aspect, a $C_{MAX}$ of the compound of Formula I in a brain of the subject is about 1-1000 ng/mg. In a further aspect, a brain-to-plasma ratio of the compound of Formula I is at least about 10:1, at least about 25:1, at least about 50:1, at least about 100:1, or at least about 200:1 one hour after the administration.

In an additional aspect, the compound of Formula I is administered orally, buccally, sublingually, rectally, vaginally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intrauterineally, intravitreally, transmucosally, or via an inhaler. In a certain aspect, the compound of Formula I is administered with a pharmaceutically acceptable excipient.

In a further aspect, the compound of Formula I is administered at a dose sufficient to modulate PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, or elongation factor 1-gamma in the subject. In a particular aspect, the compound of Formula I is administered at a dose sufficient to modulate PKM in the subject. In a specific aspect, the PKM is PKM2.

In another aspect, the compound of Formula I is administered at a dose sufficient to increasing a ratio of M2 to M1 microglia in a brain of the subject. In an additional aspect, the compound of Formula I does not cause a toxic stress response in the subject.

In some aspects, a compound of the present disclosure is any one of compounds 1a-5a, 1b-5b, 12a-h, CIAC001, or 19b-f as listed in TABLE 1

**TABLE 1**

| Compound Number | Structure |
|---|---|
| 1a | 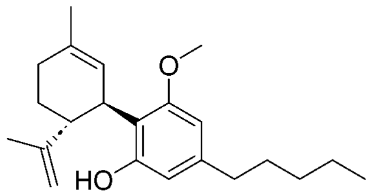 |
| 2a | 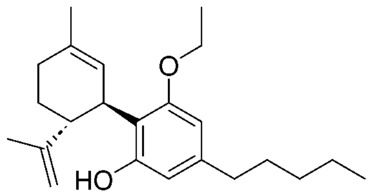 |
| 3a | 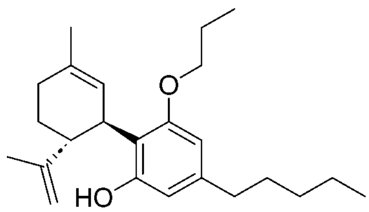 |
| 4a | 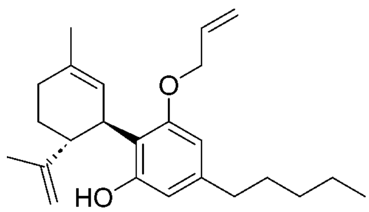 |

(continued)

| Compound Number | Structure |
|---|---|
| 5a | 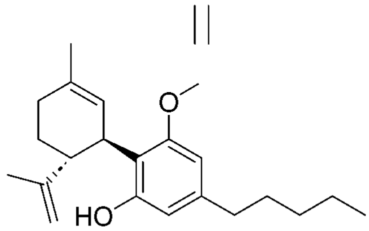 |
| 1b | 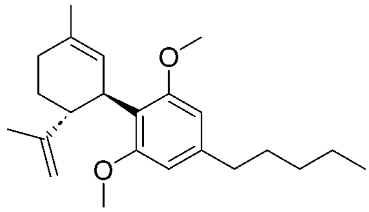 |
| 2b | 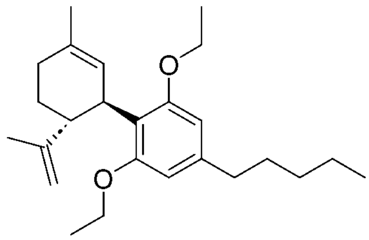 |
| 3b | 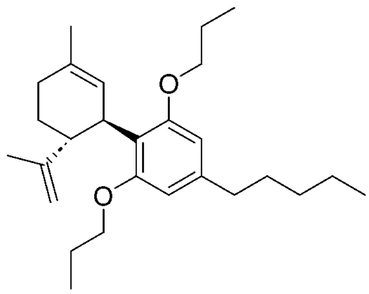 |
| 4b | 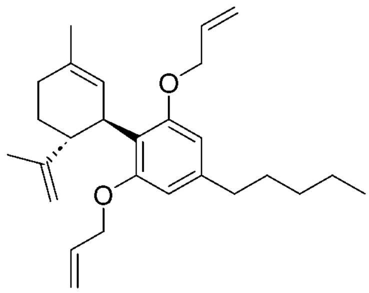 |
| 5b | 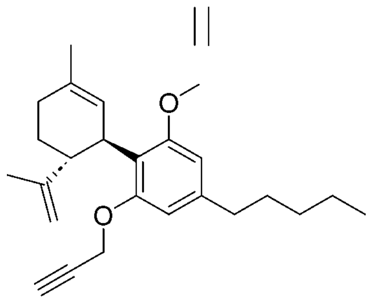 |

(continued)

| Compound Number | Structure |
|---|---|
| 12a | 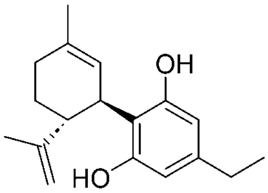 |
| 12b | 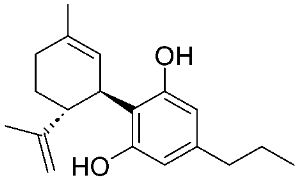 |
| 12c | 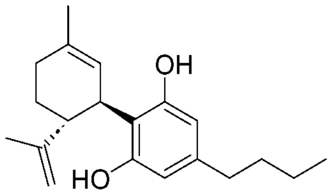 |
| 12d | 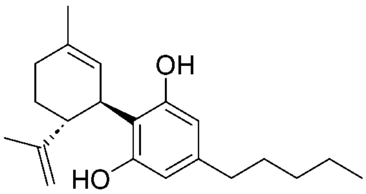 |
| 12e | 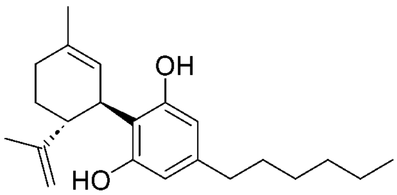 |
| 12f | 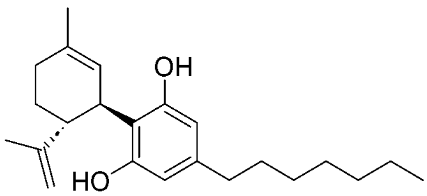 |
| 12g | 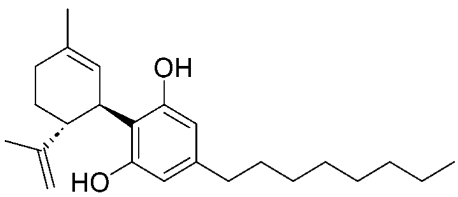 |
| 12h | 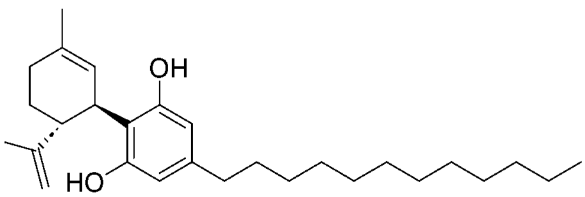 |

(continued)

| Compound Number | Structure |
|---|---|
| 19a (CIAC001) | 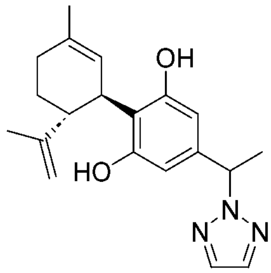 |
| 19b | 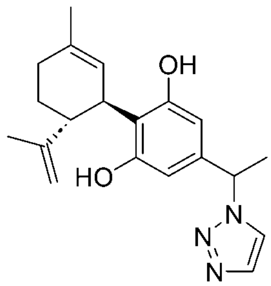 |
| 19c | 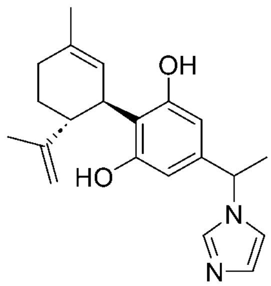 |
| 19d | 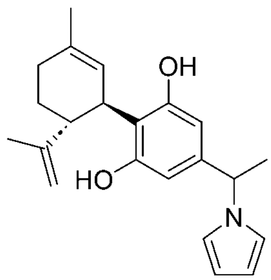 |
| 19e | 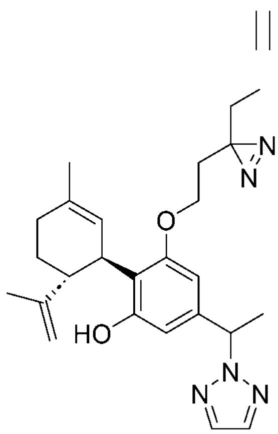 |

(continued)

| Compound Number | Structure |
|---|---|
| 19f | 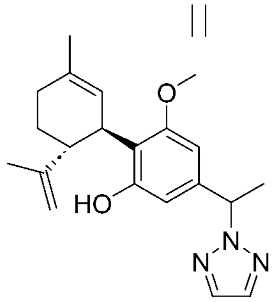 |

## EXAMPLES

The following examples are provided to further illustrate the embodiments of the present invention, but are not intended to limit the scope of the invention. While they are typical of those that might be used, other procedures, methodologies, or techniques known to those skilled in the art may alternatively be used.

## EXAMPLE 1

### Experimental Methods

#### *Cell Cultures*

BV-2 cells and GloSensor 22-F HEK293 cells were cultured in DMEM media supplemented with 10% FBS and 1% penicillin-streptomycin. All cells were incubated at 37 °C with 5% CO2.

#### *Assay Solutions*

0.5 mg/mL 2,3-Diaminonaphthalene: 5 mg of 2,3,2-aminonaphthalene was dissolved in 10 mL of 3M HCl using ultrasound for dissolution, and then vortexed for 1 minute every 30 minutes until completely dissolved. The solution was kept in a light-free environment at -20°C.

3M NaOH: 4 g of NaOH was dissolved in 33 mL of $H_2O$.

4% polyformaldehyde: 40 grams of polyformaldehyde was added to 1 liter of PBS (pH=7.4), heated in a 40°C water bath and dissolved for 1 day.

0.05% Crystal Violet: 50 mg of Crystal Violet was added to 1 L of $H_2O$ and stirred for 1 hour.

0.1 mg/mL LPS: 1 mg of LPS was dissolved in 10 mL of $ddH_2O$.

100 mM CIAC001: 3.69 mg of CIAC001 was dissolved in 100 μL of DMSO.

#### *NO Assay*

BV-2 cells were seeded at a density of 5 X $10^4$ cells per milliliter in 96-well plates. After 24 h incubation, the media was removed and changed to DMEM without FBS and penicillin-streptomycin. LPS (200 ng/mL) and different concentrations of compounds were added. After 24 h treatment, 100 μL of media was transferred to flat black 96-well micro fluor plates, and then, 10 μL of 0.05 mg/mL 2,3-diaminonaphthalene was added and incubated in the dark for 15 min. 5 μL of NaOH (3 M) was subsequently added to each well to quench the reaction. NO inhibition ratio was analyzed by a SYNERGY H1 Microplate Reader (BioTek Instruments, Carlsbad, CA, USA) with excitation at 360 nm and emission at 430 nm. The fluorescence value of the LPS-treated group was set as 100%.

#### *Cytotoxicity Assays*

100 μL of culture medium were transferred to a black 96-well plate for later use. Following removal of old culture medium, 100 μL of 4% paraformaldehyde was added for fixation. Fixation was performed for 5 minutes. Paraformaldehyde was then removed, and 100 μL of 0.05% crystal violet was added. The resultant mixture was incubated for 15 minutes, after which time crystal violet was removed and the mixture was slowly rinsed with water three times. Next, 150 μL of ethanol was added, and the mixture was incubated for 15 minutes. After incubation, the absorbance was measured at

540 nm using a spectrophotometer. Viability (%) = 100 x Fluorescence values of the dosing group/Fluorescence values of the blank group.

***Cell Viability Assays***

Cell viability was determined by crystal violet staining. Briefly, BV-2 cells were treated as indicated in the NO assay. The media was removed, and then 4% paraformaldehyde was added to fix the cells for 5 min. Cells were stained with 100 μL 0.05% crystal violet for 15 min and washed with water 2 times. Next, 150 μL of ethanol was added to each well, and the plate was incubated for 15 min at room temperature. Absorbance at 540 nm was measured using a SYNERGY H1 Microplate Reader.

***qRT-PCR***

Total RNA was extracted from cells or mouse brains using the RNeasy Mini Kit. Isolated RNA was reverse-transcribed into cDNA using an RT2 Easy First Strand cDNA Synthesis Kit following the manufacturer's protocols. PCR amplification was conducted using synthetic primers, SYBR Green, and a TOptical Real-Time qPCR Thermal Cycler (Analytik Jena, Thuringia, Germany). The data were analyzed by the ΔΔCt method, and Rp1711 or Rp127 was used as an internal control.

***Cell Morphology Determination***

BV-2 cells were seeded at a density of 2.5 X $10^5$ cells per milliliter in 6-well plates and incubated for 12 h. Cells were incubated with CIAC001 (10 μM) in the absence or presence of LPS (200 ng/mL) for 24 h. The images of BV-2 cells were taken with an inverted microscope (Ts2-FL, Nikon Corporation, Tokyo, Japan) and analyzed by ImageJ. Statistic data are counted in at least three hundred cells.

***Western Blotting***

The protein samples were separated by SDS-PAGE and transferred to PVDF membranes. The membranes were blocked with 5% nonfat dry milk for 1 h. The membranes were incubated with the corresponding primary antibody at 4 °C overnight and then washed with Tris-buffered saline with 0.1% Tween 20 (TBST) for 5 min each time and incubated for 1 h at room temperature with the secondary antibody-HRP conjugate. After washing with TBST, the membranes were developed using Super-Signal West Pico Chemiluminescent Substrate and visualized by a Tanon-5200 Multi. ImageJ was used for densitometric analysis.

***cAMP Measurement***

GloSensor 22-F HEK293 cells were transfected with plasmids encoding different receptors (CB1 and CB2). After 24 h, HEK293 cells were seeded at a density of 5 X $10^4$ cells per well in 96-well plates and incubated for 8 h; then, the medium was removed, and 200 μL of serum-free DMEM medium containing 2% GloSensor cAMP substrate (Promega) was added. After 2 h incubation, the transfected cells were stimulated with different concentrations of ligands together with 10 mM forskolin. The luminescence was recorded using a Multimode Plate Reader (EnVision, PerkinElmer).

**In Situ Pull-Down Analysis for the Identification of Target Protein**

BV-2 cells were treated with the probe Compound 19e (5 μM) or CIAC001 (20 μM) for 2 h at 37 °C; then, cells were transferred on ice and irradiated with 365 nm UV light for 30 min. Cells were washed with 3 mL PBS twice and lysed by buffer containing 250 mM HEPES (pH = 7.9), 1.5 M KCl, 5 mM EDTA, 0.5% v/v NP-40, and 1% v/v protease inhibitor cocktail. The cell lysate was centrifuged at 4 °C and 12,000g for 10 min, and the protein concentration was adjusted to 1 mg/mL by the BCA assay kit. The click reaction was conducted with 80 μM biotin-$N_3$, 100 μM TBTA, 1 mM TCEP, 1 mM CuSO4, and 5% v/v t-BuOH for 1 h at room temperature. Target proteins were purified by streptavidin magnetic beads, and 50 μL 2X Laemmli buffers were added and incubated at 100 °C for 8 min. The protein samples were separated by SDS-PAGE and then subjected to silver staining. Bands were cut, and the proteins were identified by LC-MS/MS.

**In Vitro Pull-Down Analysis for the Identification of the Target Protein**

BV-2 cells were scraped with PBS; then, cells were lysed with buffer containing 250 mM HEPES (pH = 7.9), 1.5 M KCl, 5 mM EDTA, 0.5% v/v NP-40, and 0.025% v/v protease inhibitor cocktail and incubated on ice for 30 min. The lysate

was diluted to 1 mg/mL protein concentration and incubated with the probe Compound 19f (10 μM) for 2 h. The lysate was subjected to a click reaction as described above and analyzed by SDS-PAGE. The SDS-PAGE gel was subjected to silver staining. Bands were cut, and the proteins were identified by LC-MS/MS.

**Cellular Thermal Shift Assay**

For CETSA experiments, BV-2 cells were seeded at a density of 4 X $10^5$ cells/mL in 10 cm petri dishes. After 24 h, the medium was removed, and cells were washed with cold PBS (3.0 mL) 3 times and harvested by scraping. Cells were then isolated by centrifugation (1000g, 5 min, 4 °C) and resuspended in cell lysis buffer (P0013, Beyotime Biotechnology) with the 1% protease inhibitor cocktail and then incubated on ice for 10 min. The lysates were centrifuged (12,000g, 15 min, 4 °C), and the supernatants were collected. CIAC001 (50 μM) or vehicle control was incubated with BV-2 cell lysate supernatant for 2 h at 20 °C. The lysate supernatants were divided into 60 μL each in microtubes. The lysates were heated at different temperatures (37-72 °C) for 5 min on a Long Gene A200 thermal cycler and cooled down at 25 °C for another 3 min. The samples were stored on ice until centrifugation (20,000g, 4 °C, 20 min) to remove precipitates. The lysate supernatants were denatured in 2X Laemmli sample buffer at 100 °C for 8 min for western blotting with the PKM2 antibody

***Fluorescence Titration***

PKM2 protein (0.5 μM) was titrated with increasing concentrations of CIAC001, and the fluorescence intensity ($\lambda_{ex}$ 280 nm; $\lambda_{em}$ 337 nm) was recorded by a fluorescence spectrophotometer (Cary Eclipse, Agilent Technologies Inc., USA). Appropriate controls were subtracted from the spectra obtained from the samples. The data were fitted by the nonlinear least squares method using Equation 8, derived from equations 1-7.

$$\frac{1}{2}L + P = \frac{1}{2}LP_2 \qquad (1)$$

$$K_D = \frac{[L]^{0.5} \times [P]}{[LP_2]^{0.5}} \qquad (2)$$

$$P_T = [P] + 2[LP_2] \qquad (3)$$

$$L_T = [L] + [LP_2] \qquad (4)$$

$$F = F_0 - F_{PL} \times [LP_2] \qquad (5)$$

$$[LP_2]^3 - (L_T + P_T) \times [LP_2]^2 + \frac{4P_T \times L_T + P_T^2 + K_D^2}{4} \times [LP_2] - \frac{P_T^2 \times L_T}{4} = 0 \qquad (6)$$

Because $[LP_2]^3$ is far less than $(L_T+P_T)x[LP_2]^2$, Equation 6 can be simplified as Equation 7, allowing derivation of Equation 8:

$$-(L_T + P_T) \times [LP_2]^2 + \frac{4P_T \times L_T + P_T^2 + K_D^2}{4} \times [LP_2] - \frac{P_T^2 \times L_T}{4} = 0 \qquad (7)$$

$$F = F_0 - F_{PL} \times [LP_2] = F_0 - F_{PL} \times \frac{\left(L_T \times P_T - \left(\left(\frac{K_D^2}{4} + \frac{P_T^2}{4} + L_T \times P_T\right)^2 - \frac{L_T \times P_T^2 \times (4 \times L_T + 4 \times P_T)}{4}\right)^{0.5} + \frac{K_D^2}{4} + \frac{P_T^2}{4}\right)}{2 \times L_T + 2 \times P_T} \qquad (8)$$

where F is the measured fluorescence; $F_0$ is the initial fluorescence of PKM2 in the absence of CIAC001; FPL is an adjustable parameter for protein-ligand complex molar fluorescence; KD is the dissociation constant; $L_T$ is the total concentration of CIAC001; and $P_T$ is the total PKM2 concentration. The stoichiometry n was fitted according to the equation lg (*F*0/*F* - 1) = -lg (*K*D) + *n* X lg ([CIAC001]).

***Molecular Dynamics Simulations***

CIAC001 and Compound 19b were drawn using GaussView 6 (GaussView) and optimized by Gaussian 09 (Gaussian 09) software through the B3LYP/6-31G (d,p) basis set. The three dimensional structure of the PKM2 tetramer was extracted from the crystal structure (PDB ID: 3ME3), and activators in the structure were removed before docking. The missing hydrogen atoms were added using Maestro at pH 7.0. AutoDock Vina 1.1.2 was used for molecular docking in a box of 94 X 102 X 108 Å3, which completely covered the PKM2 tetramer. During the docking process, the protein was considered rigid and the ligand was regarded as semiflexible. According to their affinities with the PKM2 tetramer, the best docking pose with the best binding affinity to PKM2 was selected out of ten docking poses generated and ranked by AutoDock Vina.

Interactions between PKM2 and CIAC001 were analyzed by LigPlot+ and PyMol (PyMol) software.

The binding of CIAC001 with the PKM2 tetramer was further investigated via molecular dynamics simulations. The best docking pose was selected as the initial structure. The complexes solvated in TIP3P water molecules with 150 mM KCl were built by the CHARMM-GUI webserver. The simulations were performed by the Gromacs 2021.2 program with the CHARMM36m force field. The systems were equilibrated in the isothermal-isobaric (NPT) ensemble at a temperature of 310.15 K for 100 ns. The LINCS algorithm was applied to constrain bonds involving hydrogen atoms.51 The particle-mesh Ewald (PME) summation method was applied to treat long-range electrostatic interactions. The temperature (310.15 K) and pressure (1 atm) were maintained by the Nosé-Hoover Langevin piston method and the Parrinello-Rahman barostat. The rmsd (root-mean-square deviation) analysis was performed through MDAnalysis (FIG. 11A).

***Subcellular Localization of PKM2***

BV-2 cells were treated with 200 ng/mL LPS and 10 μM CIAC001 for 6 h. Cells were washed, harvested with ice-cold PBS, and centrifuged at 800g for 5 min at 4 °C. Cells were lysed in cytoplasmic lysis buffer (10 mM HEPES pH 7.6, 10 mM KCl, 0.5 mM 13-mercaptoethanol, 1.5 mM MgCl2, and 1 mM DTT) plus the protease inhibitor cocktail for 20 min on ice. A final concentration of 0.5% NP40 was added to the lysate and incubated for 2 min on ice. Lysates were centrifuged at 2000g for 10 min at 4 °C, and the supernatants were cytoplasmic lysates. Pellets were washed twice with ice-cold PBS. Nuclear pellets were lysed for 30 min on ice with nuclear lysis buffer (10 mM Tris-HCl pH 7.6, 450 mM NaCl, 2 mM MgCl2, 0.5% NP-40, and 1 mM DTT) plus a protease inhibitor cocktail. Lysates were centrifuged at 12,000g for 10 min at 4 °C, and the supernatants were nuclear lysates. The nuclear lysates were diluted with low-salt buffer (10 mM Tris-HCl pH 7.6 and 2 mM MgCl2) to a final salt concentration of 150 mM in the lysates. The total protein concentrations in the nuclear and cytoplasmic extracts were determined by the BCA assay and then normalized. The lysate supernatants were denatured in 2X Laemmli sample buffer at 100 °C for 8 min for western blotting with the corresponding antibody.

***Cellular ATP Assay***

BV-2 cells were seeded at a density of 5 X $10^5$ cells per well in 6-well plates and incubated for 24 h. BV-2 cells were treated with 10 μM CIAC001 or 10 μM Compound 19b for 2 h. Cellular ATP was measured by an enhanced ATP assay kit (S0027-4, Beyotime Biotechnology Co., Ltd., Shanghai, China).

***Extracellular Lactic Acid Assay***

BV-2 cells were seeded at a density of 5 X $10^5$ cells per well in 6-well plates and incubated for 24 h. BV-2 cells were treated with 10 μM CIAC001 or 10 μM Compound 19b in the absence or presence of 200 ng/mL LPS for 12 h. Culture medium was collected and centrifuged (5000g, 4 °C) for 5 min. The supernates were used for lactic acid contents determination by lactic acid assay kit (A019-2-1, Nanjing Jiancheng Bioengineering Institute).

***Animals***

6-8 week old BALB/c male mice (20-28 g) were purchased from Liaoning Changsheng Biotechnology Co., Ltd. Mice were housed in temperature- (21-24 °C) and light-controlled (12 h light/dark cycle; lights on at 08:00) rooms with standard rodent food and water available ad libitum and allowed to habituate to the holding facility for at least 1 week prior to experimentation.

For Alzheimer's disease behavioral experiments (Morris water maze, open field experiment, novel object recognition, Y-maze, and elevated cross maze), triple transgenic mouse models (B6:129-Psen1tm1Mpm Tg(APPswe, tauP301L)1Lfa/J, 3×Tg) were purchased from the Jax Laboratory in the United States. The wild-type mice (C57BL/6J, WT) were purchased from Beijing Vital River Laboratory Animal Technology Co., Ltd. After 45 days of intraperitoneal

injection of CIAC001, all mice were subjected to subsequent behavioral experiments. In order to minimize the use of mice, the mice are first tested for sufficient physical strength and normal mental states. Only one behavioral experiment is conducted per day based on the following schedule: day 1: open field test; day 2-3: novel object recognition; day 3: elevated plus maze; day 4: Y-maze; day 5-13: Morris water maze.

Triple-transgenic model mice (B6: 129-Psen1tm1Mpm Tg (APPswe, tauP301L) 1Lfa/J, 3×Tg) were purchased from Jax Laboratory, USA; and wild-type mice (C57BL/6J, WT) were purchased from Beijing Vital River Laboratory Animal Technology Co., Ltd., which were grouped according to TABLE 2.

**TABLE 2**

| Group | Age in months at the time of treatment | Treatment time | Dosage of CIAC001 | Mode of administration | Frequency of administration | Number and gender |
|---|---|---|---|---|---|---|
| WT | 13 months | None | None | None | None | 4 males and 9 females |
| WT (high dosage) | 13 months | 45 days | 20 mg/kg | Intraperitoneal injection | 6 times in 1 week | 4 males and 9 females |
| 3×Tg-AD | 13 months | 45 days | 0 mg/kg | Intraperitoneal injection | 6 times in 1 week | 4 males and 9 females |
| 3×Tg-AD (low dosage) | 13 months | 45 days | 0.2 mg/kg | Intraperitoneal injection | 6 times in 1 week | 4 males and 9 females |
| 3×Tg-AD (medium dosage) | 13 months | 45 days | 2 mg/kg | Intraperitoneal injection | 6 times in 1 week | 4 males and 9 females |
| 3×Tg-AD (high dosage) | 13 months | 45 days | 20 mg/kg | Intraperitoneal injection | 6 times in 1 week | 4 males and 9 females |
| Note: WT refers to wild-type mice, i.e., wild-type mice; and 3×Tg-AD refers to triple-transgenic memory AD model mice, i.e., transgene in the accompanying drawings | | | | | | |

***Morphine Withdrawal***

Mice were intraperitoneally injected with an escalating dose of morphine three times daily for 3 days (5, 20, and 40 mg/kg) to induce addiction. On the fourth day, mice were given morphine (40 mg/kg) or vehicle (5 mL/kg). After 3 h, mice were pretreated with different doses of CIAC001, CBD, or vehicle 30 min before naloxone injection (15 mg/kg). The withdrawal was precipitated by naloxone. Each mouse was placed into a transparent plastic box immediately, and the number of jumps was recorded for 15 min.

***Behavioral Sensitization Experiment***

Mice were placed into the test chamber (40 X 40 X 40 $cm^3$) for 60 min to habituate the chamber for three consecutive days. Mice were given morphine (10 mg/kg, i.p.), morphine (10 mg/kg, i.p.) + CIAC001 (0.2 mg/kg, i.p.), or morphine (10 mg/kg, i.p.) + CBD (0.2 mg/kg, i.p.) once daily for 7 days. Locomotor activity was recorded daily for 60 min after the administration of drugs. Next, all the mice were subjected to a 7-day withdrawal period (days 8-14). Subsequently, mice were given morphine (5 mg/kg, i.p.), and locomotor activity was recorded to determine behavioral sensitization.

***Conditional Place Preference Procedures***

Conditional place conditioning (CPP) was conducted using the classic 10-consecutive-day procedure. On days 1 and 2, each mouse was separately placed into the CPP apparatus for 15 min to habituate the apparatus. On day 3 (preconditioning), the time spent in each compartment was recorded to determine any preference for the two compartments. Mice were divided into three groups: vehicle group, 7.5 mg/kg morphine, as well as 7.5 mg/kg morphine + 0.2 mg/kg CIAC001. During the conditioning phase, mice were separately isolated in the compartment for 30 min on 6 consecutive days. Mice were given morphine on days 4, 6, and 8, and vehicle (5 mL/kg) was administered on days 5, 7, and 9. The postconditioning phase was carried out 24 h after the last conditioning session. Each mouse was placed in the apparatus and explored freely, and the time spent in each compartment was recorded for 15 min. The CPP score (s) was calculated using the time spent in the drug-paired compartment minus the time spent in the vehicle-paired compartment.

***Immunofluorescence Staining***

Mice were anesthetized with tribromoethanol following the CPP assay and intracardially perfused with 8 mL saline; the brains were then stripped out and embedded in paraffin. The brain was sectioned coronally at 5 μm thickness on a vibrating microtome. Paraffin-processed tissues were deparaffinized in xylene and rehydrated with a graded alcohol solution. The sections were placed in 0.01 M citrate buffer (pH 6.0) and heated in a microwave oven for hot antigen repair. These sections were incubated with goat serum for 20 min at 37 °C and then with rabbit anti-Iba1 monoclonal antibody at 4 °C overnight. After three washes with PBS, the sections were incubated with an Alexa-488-conjugated secondary antibody for 1 h in the dark. Ultimately, followed by three washes with PBS twice for 5 min each, the sections were counterstained with DAPI and examined under a confocal microscope

***Y-Maze Test***

Mice were tested for short-term memory using a Y-maze task. Briefly, mice were placed into the Y-maze for 10 min once daily to habituate the apparatus for two consecutive days. After daily habituation, the mice were then treated with CIAC001 (20 mg/kg) or THC (10 mg/kg). On the 3rd day, mice were pretreated with CIAC001 or THC 45 min before the Y-maze test. Next, the "new arm" was blocked, and mice had 5 min to freely explore two arms (the "start arm" and the "old arm", **FIG. 8F).** Mice were then removed from the Y-maze and placed into the cages for 2 min. During the test phase, mice were allowed to freely explore all three arms for 2 min, and the time spent in each arm was recorded and analyzed using the Noldus analyzing software. To eliminate olfactory interference, the Y-maze was wiped off with 70% alcohol after each test.

***Open Field Test***

After two days of habitation, each mouse was placed in the open field, and the moving distances within 1 h and the grooming and rubbing numbers within the first 15 min was recorded as vehicle control (pre-test group). Next, each mouse was administered CIAC001 (20 mg/kg; i.p.) once daily for 7 days. 24 h after the last administration, the moving distances and the grooming and rubbing numbers were measured again to compare changes before and after CIAC001 treatment.

***Respiratory Rate Determination***

Mice were intraperitoneally injected with 20 mg/kg CIAC001 or 10 mg/kg THC once daily. After 1 h, the mice were anesthetized with isoflurane by a small animal anesthesia machine (R500IE, RWD Life Science Co) on day 2 and day 5. The respiratory rate of mice was recorded three times within 20 s. The weights of mice were recorded every day.

***Pharmacokinetic Profiles***

CIAC001 and CBD were adminis¬tered at 10 mg/kg (p.o.). Following administration, mice (BALB/c, male, 20-25 g) were sacrificed at 0.5, 1, 2, 3, and 4 h. The blood and brain tissue were collected and extracted with acetonitrile. The concentrations of CIAC001 and CBD were measured by LC-MS.

***General Chemistry Methods and Reagents***

Chemicals and solvents were purchased from Energy Chemical (Shanghai, China) unless otherwise stated. Analytical thin-layer chromatography (TLC) and silica gel were purchased from Qingdao Shuoyuan Silicone Technology Co., Ltd. Flash chromatography was purchased from Biotage. Silica gel column was purchased from Changzhou Santai Technology Co., Ltd. Nuclear magnetic resonance (NMR) spectra were recorded on a Bruker AV-300 spectrometer (300 MHz $^1$H, 75 MHz $^{13}$C) using $CDCl_3$ or $CD_3OD$ solutions. Chemical shifts are expressed in ppm recorded using the residual solvent as the internal reference in all cases ($CDCl_3$: $^1$H 7.26 ppm, $^{13}$C 77.16 ppm; $CD_3OD$: $^1$H 3.31 ppm, $^{13}$C 49.03 ppm). Signal splitting patterns are described as chemical shift, multiplicity (s = singlet, d = doublet, t = triplet, m = multiplet, and br = broad), coupling constant in hertz (Hz), and integration. Low-resolution electrospray (ES) mass spectra were recorded on a Waters QDa mass spectrometer run in the positive and negative modes. The optical rotations of CBD analogues were

measured by polarimeter at 20 °C (Shanghai Jiahang Instrument Co., LTD, Digipol-P610). The enantiopurities of CBD analogues were measured by the OD-H chiral column (CHIRALCEL, ODHOCE-AP039). All compounds were of >95% purity.

### *Morris Water Maze*

The main components of the Morris water maze were a circular water pool measuring about 160 cm in diameter and 50 cm in depth, a platform placed in the water, and a data acquisition system. Computer video analysis software divided the water maze is divided into four quadrants, with each quadrant having different colored and shaped patterns on the walls of the water pool. The platform in the water was a circular piece of organic glass measuring 25 $cm^2$. It was positioned at the center of one of the quadrants, 15 cm away from the edge of the water pool and submerged 1 cm below the water surface. A camera was installed directly above the water pool to record and collect data on the mouse's movement trajectory, speed, and time. During the experiment, curtains were used to cover the surroundings of the water pool, and the water in the pool was maintained at a depth of around 30 cm with a temperature of 22$\pm$1°C. Powdered milk was added to mask the mouse's odor and obstruct its line of sight. The experiments were conducted at roughly the same time each day. The Morris water maze test included place navigation training and spatial exploration tests.

Place navigation training was performed from days 1 to 5. This test involved grasping the tail of the mouse and placing it on the platform to adapt to the environment for 15 seconds. The mouse was then guided to observe the pattern on the pool wall in a specific quadrant, and then gently placed into the water in that quadrant. The maximum swimming time was set to 60 seconds. If the mouse found the platform before 60 seconds, it was immediately removed from the pool. If the mouse had not found the platform after 60 seconds, it was gently guided back to the platform for relearning. Relearning involved keeping the mouse on the platform for 10 seconds and then removing it from the water pool, placing it next to a warm lamp to dry its fur and maintain body temperature, and then putting it back into its cage. The experiment was conducted in three quadrants of the pool each day, with a 60-minute interval between each trial. The time taken for the mouse to find the platform was recorded, representing the mouse's escape latency. Water temperature was maintained at 20°C$\pm$0.5°C during escape latency detection. If a mouse failed to find the platform in a given training session and was unwilling to observe the target pattern on the platform for 10 seconds, the mouse was briefly immersed in water for several seconds before being transferred to the platform for observation.

The spatial exploration test was conducted 24 hours and 72 hours after completion of place navigation training. The test conducted at 24 hours assessed short-term memory, while the test performed at 72 hours assessed long-term memory. The platform was removed from the pool for the spatial exploration test. The mouse was placed in the opposite quadrant of where the platform was previously positioned. Software was then used to record the number of times the mouse crossed the original location of the platform and the time that it stayed in the quadrant where the platform had previously been positioned.

### *Novel Object Recognition*

Novel object recognition involved mice freely exploring within a 40 cm x 40 cm space surrounded by walls. On the first day (namely the training period), two identical objects (same color and shape) were placed symmetrically in the environment. The mouse was placed with its back to the objects and allowed to freely explore for 5 minutes. On the second day, one of the objects was replaced with a novel object (different color and shape). The mouse was placed with its back to the objects and its interactions (touching or approaching) with the two objects were recorded for 5 minutes. The interactions and duration were recorded by a camera positioned above the 40 cm x 40 cm space.

### *Elevated Cross Maze*

The elevated cross maze consisted of two intersecting open arms (25$\times$5$\times$0.5 cm) and two enclosed arms (25$\times$5$\times$16 cm) that were perpendicular to the open arms. A platform (5$\times$5$\times$0.5 cm) was placed in the center of the maze. The entire apparatus was elevated 50 cm above the floor.

At the beginning of the experiment, the mouse was placed in the center area of the elevated cross maze, facing one of the enclosed arms. The mouse was allowed to freely explore the maze for 5 minutes throughout the entire experiment. A camera connected to a computer was used to capture the mouse's movements, and the number of entries and time spent in the open and enclosed arms were recorded. After each mouse's trial, all arms and the center area were cleaned with 75% alcohol and dried with absorbent paper to eliminate any odor from mouse secretions that could interfere with subsequent trials.

***Detection of Long-Term Potentiation (LTP) in Mouse Brain Synapses***

Mouse brains were quickly removed following anesthetization using isoflurane and euthanasia by cervical dislocation and immersed in pre-chilled artificial cerebrospinal fluid (ACSF) for 3 minutes while continuously supplying $O_2$ to the fluid to maintain brain activity. Then, the brain was then quickly fixed with glue onto a platform of a vibratome (with the olfactory bulb facing up and the hindbrain facing down) and placed in well-oxygenated, pre-chilled ACSF. The brain was then sliced at 300 μm per slice with a cutting speed of 0.14 mm/s, starting from the appearance of the hippocampus. The collected brain slices were divided into left and right hemispheres and incubated in an $O_2$-filled water bath at 32°C for 2 hours. Afterwards, computer software used for recording was adjusted to ensure a stable baseline. The incubated brain slices were placed in the center of an electrode, with the brightest line of the CA1 region in the hippocampus spanning diagonally across the baseline, and fixed with a grid and iron block inside the recording device. Each potential in the electrode was stimulated to find the optimal stimulation point. High-frequency stimulation was then performed at the optimal point. Software was then used to record the changes in electrical potential at each electrode point.

***Detection of Tau Pathology, Aβ Pathology, and Synaptic-Related Protein Markers in the Mouse Brain***

The protein samples from the mouse hippocampus were analyzed for Tau5, p-Tau (T231), p-Tau (S202), p-Tau (S404), p-TrkB (Y705), Syn1, Aβ1-42, and Aβ1-16 protein content using Western blot technique.

***Detection of Insulin Signaling Pathway-Related Indicators in Alzheimer's Disease Pathology***

Glucose tolerance test (GTT) procedure: after 16 hours of fasting, mice were weighed, and baseline blood glucose levels were measured using a glucose meter via a tail incision. Then, an intraperitoneal injection of 2 g/kg glucose was administered, and glucose levels were measured at 30, 60, 90, 120, and 150 minutes after glucose injection.

Insulin tolerance test (ITT) procedure: after 6 hours of fasting, mice were weighed, and baseline blood glucose levels were measured using a glucose meter via a tail incision. Then, an intraperitoneal injection of 0.75 U/kg recombinant human insulin was administered, and glucose levels were measured at 30, 60, 90, 120, and 150 minutes after insulin injection.

***Sample Preparation***

Compounds (e.g., CIAC001) were dissolved in 88% saline, 7% Tween 80, and 5% DMSO. The prepared solution was able to be stored at 4°C for 2 weeks.

***Pull Down Analysis***

1 M HEPES (pH 7.9), 1 M KCl, 1 M EDTA, 10% NP40, 1 M $CuSO_4$, and 1M TEPC-HCl were prepared in $ddH_2O$. 80 mM Bition-$PEG_3$-$N_3$ and 100 mM TBTA were prepared in DMSO. Lysate solution was prepared by mixing 12.5 mL HEPES, 7.5 mL KCl, 250 μL NP40 and 250 μL EDTA, and then diluting with $ddH_2O$ to make 50 mL. The lysate solution contained 250 mM HEPES, 150 mM KCl, 0.5% NP40, and 5 mM EDTA.

10 cm dishes of BV-2 cells were cultured for 24 hours with a 1:3 ratio of cell culture to medium. Medium was removed and replaced with 10 mL fresh complete DMEM medium (10% FPS, 1% PS) containing 5 μM CP1 and 20 μM CIAC001. The cells were treated for 2 h and then transferred the culture dish onto ice, and exposed to 365 nm ultraviolet light for 30 minutes. The cells were washed twice with 2 mL of PBS, then collected using a cell scraper. Cell pellets were resuspended in 1 mL of cell lysis buffer and incubated on ice for 30 minutes. After lysis, the lysate was centrifuged at 12,000g for 10 minutes at 4°C, and the supernatant was collected. The protein concentration was adjusted to 1 mg/mL using a BCA assay kit.

A click reaction was then performed by taking 1 mL of protein lysate and adding 1 μL of 80 mM Bition-$PEG_3$-$N_3$, 1 μL of 100 mM TBTA, 1 μL of 1M TCEP, 1 μL of 1M $CuSO_4$, and 50 μL of t-BuOH (final concentrations in the reaction mixture are 80 μM Bition-$PEG_3$-$N_3$, 100 μM TBTA, 1 mM TCEP, 1 mM $CuSO_4$, and 5% v/v t-BuOH). The reaction was performed at room temperature for 1 hour.

After completion of the reaction, 20 μL of streptavidin affinity magnetic beads were washed 3 times with 1 mL PBS, added to the treated lysate, and then incubated at room temperature for 1.5 hours. The beads were then washed 3 times with PBS to remove nonspecific binding. After washing, the supernatant was removed, 50 μL of 2× Laemmli buffer was added, and the mixture was heated to 100°C for 8 minutes. Following separation on an SDS-PAGE gel, staining was performed with a silver staining kit. The gel was excised and target protein was identified using LC-MS.

**EXAMPLE 2**

**General Procedure for the Synthesis of Compounds 1a-5a and 2a-5b**

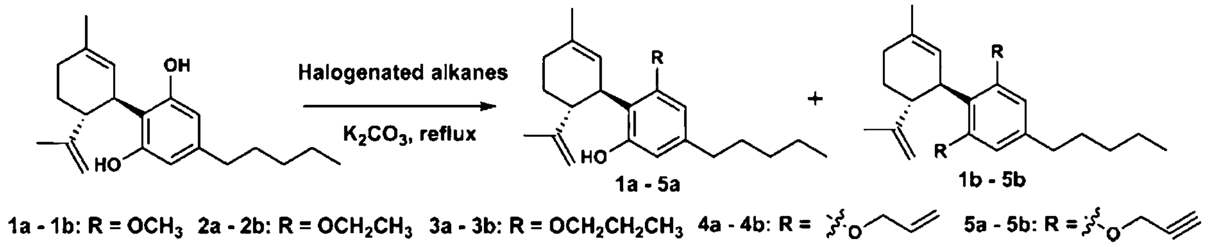


*methyl-4-pentyl-2'-(prop-1-en-2-yl)-1',2',3',4'-tetrahydro-[1,1'-biphenyl]-2-ol* ***(1a)*** *and (1R,2R)-2',6'-Dimethoxy-5-methyl-4'-pentyl-2-(prop-1-en-2-yl)-1,2,3,4-tetrahy-dro-1,1'-biphenyl* ***(1b).*** CBD (0.31 g, 1 mmol) and $CH_3I$ (1.5 mmol, 1.5 equiv) were dissolved in 4 mL of acetone. $K_2CO_3$ (0.14 g, 1 mmol, 1.0 equiv) was crushed into powder and then added to the reaction solution. The solution was stirred at 60 °C overnight. The reaction was quenched by 3 M HCl, and the aqueous phase was extracted by ethyl acetate (2 × 20 mL). The organic phase was washed with brine and dried with $Na_2SO_4$. The crude product was purified by Prep TLC to afford yellow oil. Yield **1a**: yellow oil, 0.085 g, 25.9%, $^1$H NMR (300 MHz, $CDCl_3$): δ = 6.32 (s, 1H), 6.24 (s, 1H), 6.03 (s, 1H), 5.59 (s, 1H), 4.50 (s, 1H), 4.33 (s, 1H), 4.01 (d, *J* = 8.6, 1H), 3.72 (s, 3H), 2.57-2.37 (m, 3H), 2.28-2.09 (m, 2H), 1.80 (s, 5H), 1.68 (s, 3H), 1.63-1.56 (m, 2H), 1.38-1.30 (m, 4H), 0.90 (t, *J* = 6.8, 3H). $^{13}$C NMR (75 MHz, $CDCl_3$): δ = 158.1, 155.7, 147.3, 142.7, 124.5, 115.0, 110.9, 109.5, 103.1, 55.5, 46.7, 36.0, 31.6, 30.9, 30.3, 28.1, 23.7, 22.6, 14.1. $[a]_D^{20}$ = -3 (*c* = 1 mg/mL, $CHCl_3$), ee (%) = 99.0%. MS ($ESI^+$) calcd for $C_{22}H_{32}O_2$ + $Na^+$, 351.24 [M + Na]; found, 351.22. Yield **1b**: yellow oil, 0.075 g, 21.9%, $^1$H NMR (300 MHz, $CDCl_3$): δ 6.35 (s, 2H), 5.22 (s, 1H), 4.45 (d, *J* = 4.7 Hz, 2H), 4.00 (d, *J* = 12.1 Hz, 1H), 3.75 (s, 6H), 2.97-2.86 (m, 1H), 2.59-2.51 (m, 2H), 2.29-1.92 (m, 2H), 1.81-1.73 (m, 3H), 1.68 (s, 3H), 1.62 (s, 4H), 1.39-1.30 (m, 4H), 0.92 (t, *J* = 6.7 Hz, 3H). $^{13}$C NMR (75 MHz, $CDCl_3$): δ 149.6, 141.9, 131.2, 125.9, 118.9, 109.6, 55.9, 45.2, 36.4, 36.1, 31.7, 31.1, 30.7, 29.7, 23.5, 22.6, 19.0, 14.1. $[a]_D^{20}$ = -115 (c = 1 mg/mL, $CHCl_3$), ee (%) = 99.7%. MS ($ESI^+$) calcd for $C_{23}H_{34}O_2$ + $H^+$, 343.22 [M + H]; found, 343.27.

*(1' R,2' R)-6-Ethoxy-5'-methyl-4-pentyl-2'-(prop-1-en-2-yl)-1'2',3',4'-tetrahydro-[1,1'-biphenyl]-2-ol* ***(2a)*** *and (1R,2R)-2',6'-Diethoxy-5-methyl-4'-pentyl-2-(prop-1-en-2-yl)-1,2,3,4-tetrahydro-1,1'-biphenyl* ***(2b).*** The compound was prepared with CBD (0.31 g, 1 mmol) and $CH_3CH_2Br$ (1.5 mmol, 1.5 equiv) according to the **1a** procedure. Yield **2a:** yellow oil, 0.094 g, 27.5%, $^1$H NMR (300 MHz, $CDCl_3$): δ = 6.31 (s, 1H), 6.22 (s, 1H), 6.01 (s, 1H), 5.59 (s, 1H), 4.51 (s, 1H), 4.36 (s, 1H), 4.09-3.84 (m, *J* = 18.4, 17.5, 9.4, 3H), 2.55-2.38 (m, 3H), 2.30-1.98 (m, 2H), 1.87-1.74 (m, 4H), 1.69 (s, 3H), 1.66-1.54 (m, 3H), 1.41-1.28 (m, 7H), 0.90 (t, *J* = 6.6, 3H). $^{13}$C NMR (75 MHz, $CDCl_3$): δ = 157.4, 155.7, 147.2, 142.6, 124.7, 115.1, 111.0, 109.3, 103.8, 63.6, 46.4, 36.0, 31.6, 30.9, 30.3, 23.7, 22.6, 14.9, 14.1. $[a]_D^{20}$ = -40 (c = 1 mg/mL, $CHCl_3$), ee (%) = 98.8%. MS ($ESI^+$) calcd for $C_{23}H_{34}O_2$ + $Na^+$, 365.26 [M + Na]; found, 365.29. Yield 2b: yellow oil, 0.071 g, 19.2%, $^1$H NMR (300 MHz, $CDCl_3$): δ = 6.30 (s, 2H), 5.25 (s, 1H), 4.45 (s, 1H), 4.40 (s, 1H), 4.06-3.84 (m, 5H), 2.93 (dd, *J* = 16.3, 9.1, 1H), 2.56-2.46 (m, 2H), 2.28-1.93 (m, 3H), 1.82-1.73 (m, 2H), 1.70-1.64 (m, 5H), 1.58 (s, 3H), 1.40-1.30 (m, 9H), 0.90 (t, *J* = 6.7, 3H). $^{13}$C NMR (75 MHz, $CDCl_3$): δ = 149.5, 141.7, 130.9, 126.3, 118.9, 109.7, 45.2, 36.4, 35.9, 31.7, 31.0, 30.7, 29.7, 29.5, 23.5, 22.6, 19.1, 15.0, 14.1. $[a]_D^{20}$ = -29 (c = 1 mg/mL, $CHCl_3$), ee (%) = 98.8%. MS ($ESI^+$) calcd for $C_{25}H_{38}O_2$ + $Na^+$, 393.29 [M + Na]; found, 393.31.

*(1'R,2'R)-5'-Methyl-4-pentyl-2'-(prop-1-en-2-yl)-6-propoxy-1',2',3',4'-tetrahydro-[1,1'-biphenyl]-2-ol* ***(3a)*** *and (1R,2R)-5-Meth-yl-4'-pentyl-2-(prop-1-en-2-yl)-2',6'-dipropoxy-1,2,3,4-tetrahydro-1,1'-biphenyl* ***(3b).*** The compound was prepared with CBD (0.31 g, 1 mmol) and $CH_3CH_2CH_2Br$ (1.5 mmol, 1.5 equiv) according to the 1a procedure. Yield **3a:** yellow oil, 0.081 g, 22.7%, $^1$H NMR (300 MHz, $CDCl_3$): δ = 6.31 (s, 1H), 6.23 (s, 1H), 6.04 (s, 1H), 5.60 (s, 1H), 4.53 (s, 1H), 4.39 (s, 1H), 4.05 (d, *J* = 7.8, 1H), 3.92-3.76 (m, 2H), 2.56-2.39 (m, 3H), 2.33-2.01 (m, 2H), 1.85-1.73 (m, 7H), 1.68 (s, 3H), 1.64-1.55 (m, 2H), 1.41-1.29 (m, 4H), 1.03 *(t, J*= 7.4, 3H), 0.90 (t, *J* = 6.7, 3H). $^{13}$C NMR (101 MHz, $CDCl_3$): δ = 149.6, 141.7, 130.7, 126.8, 118.6, 109.6, 107.3, 105.0, 100.0, 44.9, 36.4, 36.2, 31.7, 31.1, 30.8, 29.7, 23.5, 22.9, 22.6, 19.3, 14.1, 10.8. $[a]_D^{20}$ = -28 (c = 1 mg/mL, $CHCl_3$), ee (%) = 99.9%. MS ($ESI^+$) calcd for $C_{24}H_{36}O_2$ + $Na^+$, 379.27 [M + Na]; found, 379.31. Yield **3b:** yellow oil, 0.089 g, 22.1%, $^1$H NMR (300 MHz, $CDCl_3$): δ = 6.30 (s, 2H), 5.26 (s, 1H), 4.51-4.39 (m, 2H), 4.04 (d, *J* = 12.5, 1H), 3.84 (dd, *J* = 12.4, 6.2, 4H), 3.06-2.87 (m, 1H), 2.56-2.44 (m, 2H), 2.33-1.91 (m, 3H), 1.83-1.72 (m, 5H), 1.66 (s, 3H), 1.64-1.60 (m, 3H), 1.58 (s, 2H), 1.38-1.31 (m, 4H), 1.04 (t, *J* = 7.4, 6H), 0.91 (t, *J* = 6.8, 3H). $^{13}$C NMR (75 MHz, $CDCl_3$): δ = 149.6, 141.7, 130.7, 126.7, 118.5, 109.6, 44.9, 36.5, 36.2, 31.7, 31.1, 30.8, 29.6, 23.5, 22.9, 22.6, 19.3, 14.1, 10.8. $[a]_D^{20}$ = -48 (c = 1 mg/mL, $CHCl_3$), ee (%) = 99.9%. MS ($ESI^+$) calcd for $C_{27}H_{42}O_2$ + $Na^+$, 421.32 [M + Na]; found, 421.34.

*(1'R,2'R)-6-(Allyloxy)-5'-methyl-4-pentyl-2'-(prop-1-en-2-yl)-1',2',3',4'-tetrahydro-[1,1'-biphenyl]-2-ol* ***(4a)*** *and (1R,2R)-2',6'-Bis(allyloxy)-5-methyl-4'-pentyl-2-(prop-1-en-2-yl)-1,2,3,4-tetrahy-dro-1,1'-biphenyl* ***(4b).*** The compound was prepared with CBD (0.31 g, 1 mmol) and 3-bromoprop-1-ene (1.5 mmol, 1.5 equiv) according to the **1a** procedure.

Yield 4a: yellow oil, 0.10 g, 28.2%, $^1H$ NMR (300 MHz, $CDCl_3$): δ = 6.33 (s, 1H), 6.23 (s, 1H), 6.04 (m, 2H), 5.60 (s, 1H), 5.39 (dd, *J* = 17.3, 1.6, 1H), 5.25 (dd, *J* = 10.5, 1.4, 1H), 4.54-4.33 (m, 4H), 4.06 (d, *J* = 8.4, 1H), 2.57-2.39 (m, 3H), 2.34-2.01 (m, 3H), 1.85-1.76 (m, 4H), 1.69-1.53 (m, 5H), 1.38- 1.29 (m, 4H), 0.90 (t, *J* = 6.8, 3H). $^{13}C$ NMR (75 MHz, $CDCl_3$): δ = 157.0, 155.8, 147.3, 142.6, 133.8, 124.6, 116.9, 111.1, 109.7, 104.2, 100.0, 69.3, 36.0, 31.5, 30.8, 30.3, 29.7, 23.7, 22.6, 14.1. $[a]_D^{20}$ = -22 (c = 1 mg/mL, $CHCl_3$), ee (%) = 99.8%. MS ($ESI^+$) calcd for $C_{24}H_{34}O_2 + Na^+$, 377.26 [M + Na]; found, 377.27. Yield **4b:** yellow oil, 0.070 g, 18.0%, $^1H$ NMR (300 MHz, $CDCl_3$): δ = 6.35 (s, 2H), 6.12-5.96 (m, 2H), 5.45 (s, 1H), 5.40 (s, 1H), 5.32 (s, 1H), 5.29- 5.21 (m, 2H), 4.53-4.42 (m, 6H), 4.09 (d, *J* = 8.7, 1H), 3.05-2.91 (m, 1H), 2.60-2.48 (m, 2H), 2.28-1.95 (m, 2H), 1.83-1.75 (m, 2H), 1.68 (s, 3H), 1.66-1.55 (m, 5H), 1.33 (m, 4H), 0.92 (t, *J* = 6.8, 3H). $^{13}C$ NMR (75 MHz, $CDCl_3$): δ = 149.4, 141.8, 133.9, 131.0, 126.5, 119.4, 116.4, 109.9, 69.4, 45.1, 36.4, 36.2, 31.6, 31.0, 30.8, 29.6, 23.5, 22.6, 19.3, 14.1. $[a]_D^{20}$ = -47 (c = 1 mg/mL, $CHCl_3$), ee (%) = 99.9%. MS ($ESI^+$) calcd for $C_{27}H_{38}O_2 + Na^+$, 417.29 [M + Na]; found, 417.29.

*(1'R,2'R)-5'-Methyl-4pentyl-2'-(prop-1-en-2-yl)-6-(prop-2-yn-1-yloxy)-1',2',3',4'-tetrahydro-[1,1'-biphenyl]-2-ol* ***(5a)*** *and (1R,2R)-5-Methyl-4'-pentyl-2-(prop-1-en-2yl)-2',6'-bis(prop-2-yn-1-yloxy)-1,2,3,4-tetrahydro-1,1'-biphenyl* ***(5b).*** Yield **5a:** yellow oil, 0.090 g, 25.6%, $^1H$ NMR (300 MHz, $CDCl_3$): δ = 6.36 (s, 1H), 6.31 (s, 1H), 6.07 (s, 1H), 5.59 (s, 1H), 4.62-4.56 (m, 2H), 4.51 (s, 1H), 4.33 (s, 1H), 4.02 (d, *J* = 8.4, 1H), 2.59-2.36 (m, 4H), 2.33-1.98 (m, 2H), 1.85-1.77 (m, 4H), 1.70 (s, 3H), 1.66-1.53 (m, 3H), 1.40-1.28 (m, 4H), 0.90 (t, *J* = 6.7, 3H). $^{13}C$ NMR (75 MHz, $CDCl_3$): δ = 156.3, 155.8, 148.0, 142.7, 124.3, 115.7, 111.2, 110.4, 104.4, 79.0, 74.9, 56.4, 46.6, 35.9, 31.5, 30.8, 30.3, 23.8, 22.6, 14.1. $[a]_D^{20}$ = -23 (c = 1 mg/ mL, $CHCl_3$), ee (%) = 96.0%. MS ($ESI^+$) calcd for $C_{24}H_{32}O_2 + Na^+$, 375.24 [M + Na]; found, 375.22. Yield 5b: yellow oil, 0.095 g, 24.3%, $^1H$ NMR (300 MHz, $CDCl_3$): δ = 6.46 (s, 2H), 5.23 (s, 1H), 4.61 (d, *J* = 2.3, 4H), 4.48-4.42 (m, 2H), 4.01 (d, *J*= 10.5, 1H), 2.97-2.83 (m, 1H), 2.61-2.51 (m, 2H), 2.48 (t, *J* = 2.4, 2H), 2.31-1.92 (m, 2H), 1.81-1.73 (m, 2H), 1.72-1.55 (m, 8H), 1.39-1.30 (m, 4H), 0.91 (t, *J* = 6.8, 3H). $^{13}C$ NMR (75 MHz, $CDCl_3$): δ = 149.2, 141.9, 131.7, 125.3, 120.7, 110.1, 79.2, 74.6, 56.6, 45.4, 36.2, 36.1, 31.5, 30.9, 30.6, 29.7, 29.4, 23.5, 22.6, 19.1, 14.1. $[a]_D^{20}$ = -71 (c = 1 mg/mL, $CHCl_3$), ee (%) = 96.8%. MS ($ESI^+$) calcd for $C_{27}H_{34}O_2 + Na^+$, 413.26 [M + Na]; found, 413.25.

## EXAMPLE 3

**General Procedure for the Synthesis of Compounds 12a-12h**

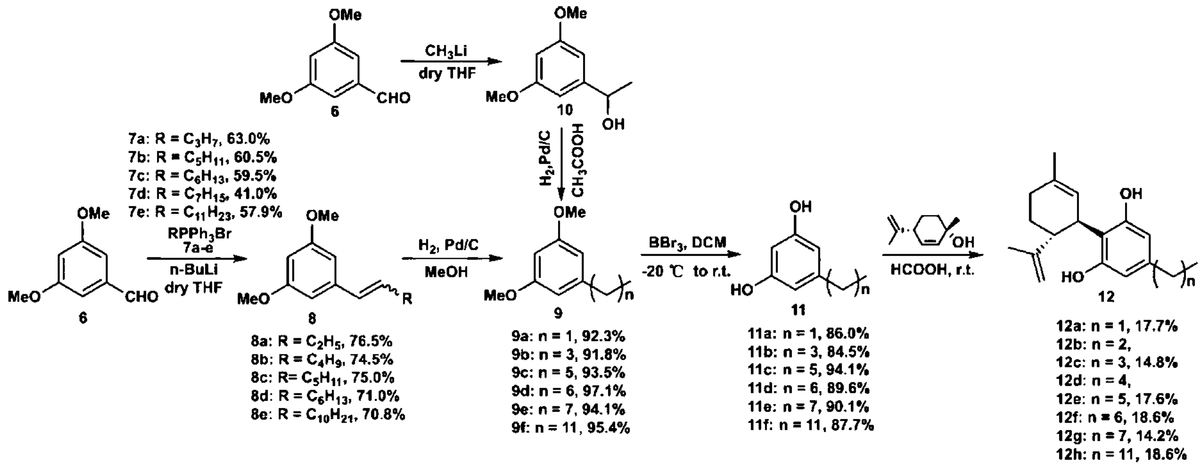


This example covers the synthesis of CBD analogues with alkane side chains of various lengths. Briefly, alkene derivatives **8a-8e** were prepared by triphenylphosphonium bromide analogues **7a-7e** and 3,5-dimethoxybenzaldehyde (6) by the Wittig reaction. Subsequently, alkene derivatives **8a-8e** were hydrogenated by 10% Pd/C to furnish **9b-9f.** Then, the methoxy group from **9b-9f** was removed by $BBr_3$ to yield the phenol analogues **11b-11f.** Especially, phenol analogue **11a** was achieved through a nucleophilic addition with $CH_3Li$, then catalytically hydrogenating with Pd/C, and finally removing phenolic hydroxyl groups. The synthesis of these **12a-12h** was conveniently accomplished by the Friedel-Crafts alkylation reaction between menthadienol and olivetol derivatives **11a- 11f** with linear alkane chains of different lengths.

*Propyl-triphenylphosphonium Bromide (7a).* $Ph_3P$ (2.62 g, 10 mmol) and bromopropane (1.13 g, 10 mmol, 1 equiv) were dissolved in dry toluene (50 mL) and refluxed for 18 h. The reaction solution was cooled to room temperature, and then, 200 mL of ether was added and stirred for 30 min. The precipitate was then filtered, washed with ether, and dried to afford a white powder without further purification. Yield **7a:** 3.49 g, 63.0%, mp = 248-258 °C, $^1H$ NMR (300 MHz, $CDCl_3$): δ = 7.98-7.63 (m, 15H), 3.97-3.73 (m, 2H), 1.74 (s, 2H), 1.35-1.13 (m, 3H).

*Pentyltriphenylphosphonium Bromide (7b).* Compound **7b** was prepared with $Ph_3P$ (11.80 g, 40 mmol) and bromopentane (6.04 g, 40 mmol, 1 equiv) according to the **7a** procedure. Yield **7b:** white powder, 7.48 g, 60.5%, mp = 171-176, $^1$H NMR (300 MHz, $CDCl_3$): δ = 7.89-7.56 (m, 15H), 3.73-3.51 (m, 2H), 1.54 (dd, *J* = 9.0, 5.4 Hz, 4H), 1.23 (dt, *J* = 14.0, 7.0 Hz, 2H), 0.73 (t, *J* = 7.3 Hz, 3H).

*Hexyltriphenylphosphonium Bromide (7c).* Compound **7c** was prepared with $Ph_3P$ (11.80 g, 40 mmol) and bromohexane (6.60 g, 40 mmol, 1 equiv) according to the **7a** procedure. Yield 7c: yellow oil, 7.65 g, 59.5%, $^1$H NMR (300 MHz, $CDCl_3$): δ = 7.79 (m, 15H), 3.89-3.77 (m, 2H), 1.70 (m, 4H), 1.25 (s, 4H), 0.83 (t, *J* = 7.0 Hz, 3H).

*Heptyltriphenylphosphonium Bromide (7d).* The compound was prepared with $Ph_3P$ (11.80 g, 40 mmol) and bromoheptane (7.16 g, 40 mmol, 1 equiv) according to the **7a** procedure. Yield 7d: yellow oil, 6.27 g, 41.0%, $^1$H NMR (300 MHz, $CDCl_3$): δ = 7.88-7.56 (m, 15H), 3.71-3.48 (m, 2H), 1.54 (d, *J* = 3.6 Hz, 4H), 1.27-1.00 (m, 6H), 0.72 (t, *J* = 6.9 Hz, 3H).

*Hendecyltriphenylphosphonium Bromide (7e).* The compound was prepared with $Ph_3P$ (11.80 g, 40 mmol) and bromoundecane (9.40 g, 40 mmol, 1 equiv) according to the **7a** procedure. Yield **7e:** yellow oil, 8.75 g, 57.9%, $^1$H NMR (300 MHz, $CDCl_3$): δ = 7.73 (m, 15H), 3.67 (s, 2H), 1.58 (d, *J* = 3.5 Hz, 4H), 1.18 (d, *J* = 16.0 Hz, 14H), 0.81 (t, *J* = 6.7 Hz, 3H).

*(Z) And (E)-1-(But-1-en-1-yl)-3,5-dimethoxybenzene (8a).* Compound **7a** (0.50 g, 1.3 mmol, 1.3 equiv) was dissolved in 30 mL of dry THF under nitrogen at -30 °C. *n*-BuLi (1.3 mmol, 1.3 equiv) was added dropwise to the solution. The solution was stirred for 30 min at -30 °C, and then 3,5-dimethoxybenzaldehyde **(6)** (1 mmol) was added to the solution and stirred for 16 h at 70 °C. The reaction was quenched by water, and the aqueous phase was extracted by ether (2× 40 mL). The organic phase was washed with brine and dried with $Na_2SO_4$. The crude product was purified by flash chromatography eluting with petroleum ether/EtOAc 20:1 to afford the colorless oil. The product was a mixture of *E*/*Z* isomers, yield: 0.15 g, 76.5%, *E*/*Z* ca. 70/30 according to $^1$H NMR, (*E*)-**8a:** $^1$H NMR (300 MHz, CDCl3): δ = 6.50 (d, *J* = 2.3 Hz, 2H), 6.36-6.34 (m, 1H), 6.37-6.32 (m, 2H), 3.78 (s, 6H), 2.17-2.26 (m, 2H), 1.08 (t, *J* = 8.1 Hz, 3H); $^{13}$C NMR (75 MHz, $CDCl_3$): δ = 161.0, 140.2, 133.4, 129.0, 104.2, 99.3, 55.5, 26.2, 13.8. (*Z*)-**8a:** $^1$H NMR (300 MHz, $CDCl_3$): δ 6.43= (d, *J* = 2.2 Hz, 2H), 6.3-6.24 (m, 2H), 5.65 (m, 1H), 3.80 (s, 6H), 2.34 (m, 2H), 1.08 (t, *J* = 8.1 Hz, 3H); $^{13}$C NMR (75 MHz, $CDCl_3$): δ = 160.7, 139.8, 135.4, 128.4, 107.0, 106.6, 98.9, 55.5, 22.3, 14.6. MS (ESI$^+$) calcd for $C_{12}H_{16}O_2$ + $H^+$, 193.12 [M + H]$^+$; found, 193.04.

*(Z) And (E)-1-(Hex-1-en-1-yl)-3,5-dimethoxybenzene (8b).* Compound **8b** was prepared with compound **7b** (1.71 g, 2.6 mmol, 1.3 equiv) and compound **6** (0.33 g, 2 mmol) according to the **8a** procedure. The product was a mixture of E/Z isomers, yield: 0.33 g, 74.5%, *E*/*Z* ca. 65/35 according to $^1$H NMR, (*E*)-**8b:** $^1$H NMR (300 MHz, $CDCl_3$): δ = 6.49 (d, *J* = 2.2 Hz, 2H), 6.36-6.30 (m, 1H), 6.37-6.12 (m, 2H), 3.78 (s, 6H), 2.15-2.22 (m, 2H), 1.52-1.24 (m, 4H), 0.91 (t, *J* = 7.6 Hz, 3H); $^{13}$C NMR (75 MHz, $CDCl_3$): δ = 161.1, 140.2, 132.0, 129.9, 104.2, 99.3, 55.5, 32.8, 31.7, 22.5, 14.2. (*Z*)-**8b:** $^1$H NMR (300 MHz, $CDCl_3$): δ 6.42= (d, *J* = 2.2 Hz, 2H), 6.36-6.30 (m, 2H), 5.64 (m, 1H), 3.78 (s, 6H), 2.32 (m, 2H), 1.52- 1.24 (m, 4H), 0.91 (t, *J* = 7.6 Hz, 3H); $^{13}$C NMR (75 MHz, $CDCl_3$): δ = 160.7, 139.9, 133.9, 129.9, 128.9, 107.0, 98.9, 55.5, 32.3, 28.7, 22.6, 14.2. MS (ESI$^+$) calcd for $C_{14}H_{20}O_2$ + $H^+$, 221.15 [M + H]$^+$; found, 221.07.

*1-(3,5-Dimethoxyphenyl)-1-heptylene (8c).* Compound 8c was prepared with compound **7c** (1.11 g, 2.6 mmol, 1.3 equiv) and compound **6** (0.33 g, 2 mmol) according to the **8a** procedure. The product was a mixture of E/Z isomers, yield: 0.35 g, 75.0%, *E*/*Z* ca. 63/37 according to $^1$H NMR, $^1$H NMR, (*E*)-8c: $^1$H NMR (300 MHz, $CDCl_3$): δ 6.53 (d, *J* = 2.1 Hz, 2H), 6.38-6.35 (m, 1H), 6.31-6.19 (m, 2H), 3.82 (s, 6H), 2.27-2.17 (m, 2H), 1.54-1.43 (m, 2H), 1.38-1.30 (m, 4H) 0.93 (t, *J* = 7.2 Hz, 3H); $^{13}$C NMR (75 MHz, $CDCl_3$): δ = 161.1, 140.2, 132.1, 129.8, 104.2, 99.3, 55.3, 33.1, 31.6, 29.2, 22.8, 14.3. (Z)-8c: $^1$H NMR (300 MHz, $CDCl_3$): δ = 6.46 (d, *J* = 2.2 Hz, 2H), 6.38-6.30 (m, 2H), 5.73-5.64 (m, 1H), 3.82 (s, 6H), 2.39-2.30 (m, 2H), 1.38-1.30 (m, 4H), 0.93 (t, *J* = 7.2 Hz, 3H); $^{13}$C NMR (75 MHz, $CDCl_3$): δ = 160.7, 138.4, 131.7, 128.8, 126.9, 107.1, 95.8, 55.5, 33.0, 31.8, 29.4, 22.8, 14.3. MS (ESI$^+$) calcd for $C_{15}H_{22}O_2$ + $H^+$, 235.16 [M + H]$^+$; found, 235.09.

*1-(3,5-Dimethoxyphenyl)-1-octylene (8d).* Compound **8d** was prepared with compound **7d** (1.15 g, 2.6 mmol) and compound 6 (0.33 g, 2 mmol) according to the **8a** procedure. The product was a mixture of E/Z isomers (0.35 g, 71.0% yield, *E*/*Z* ca. 56/44 according to $^1$ H NMR. $^1$H NMR, (*E*)-**8d:** $^1$H NMR (300 MHz, $CDCl_3$): δ = 6.53 (d, *J* = 2.1 Hz, 2H), 6.39-6.35 (m, 1H), 6.32-6.19 (m, 2H), 3.82 (s, 6H), 2.25-2.18 (m, 2H), 1.52-1.42 (m, 2H), 1.40-1.25 (m, 6H) 0.90 (t, *J* = 7.0 Hz, 3H); $^{13}$C NMR (75 MHz, $CDCl_3$): δ = 161.1, 140.2, 132.1, 129.8, 104.2, 99.3, 55.5, 33.2, 30.1, 29.3, 29.0, 22.8, 14.3. (*Z*)-**8d:** $^1$H NMR (300 MHz, $CDCl_3$): δ = 6.46 (d, *J* = 2.1 Hz, 2H), 6.39-6.31 (m, 2H), 5.73-5.64 (m, 1H), 3.82 (s, 6H), 2.39-2.32 (m, 2H), 1.52-1.42 (m, 2H), 1.40-1.25 (m, 6H) 0.90 (t, *J* = 7.0 Hz, 3H); $^{13}$C NMR (75 MHz, $CDCl_3$): δ = 160.7, 140.2, 134.4, 128.9, 107.0, 98.9, 55.5, 33.2, 33.0, 29.5, 29.1, 22.8, 14.3. MS (ESI$^+$) calcd for $C_{16}H_{24}O_2$ + $H^+$, 249.08 [M + H]$^+$; found, 248.99.

*1-(3,5-Dimethoxyphenyl)-1-laurylene (8e).* Compound **8e** was prepared with compound **7e** (1.29 g, 2.6 mmol) and compound **6** (0.33 g, 2 mmol) according to the **8a** procedure. The product was a mixture of *E*/*Z* isomers (0.35 g, 70.8% yield, *E*/*Z* ca. 64/36 according to $^1$H NMR. $^1$H NMR, (*E*)-**8e:** $^1$H NMR (300 MHz, $CDCl_3$): δ = 6.54 (d, *J* = 2.1 Hz, 2H), 6.39-6.35 (m, 1H), 6.32-6.19 (m, 2H), 3.82 (s, 6H), 2.25-2.18 (m, 2H), 1.52-1.42 (m, 2H), 1.40-1.25 (m, 14H) 0.90 (t, *J* = 7.0 Hz, 3H); (*E*)-**8e**: $^{13}$C NMR (75 MHz, $CDCl_3$): δ 160.9, 140.0, 131.9, 129.6, 104.0, 99.1, 55.3, 33.0, 31.9, 30.0, 29.6, 29.6, 29.4, 29.3, 28.8, 22.7, 14.1.). (*Z*)-**8e:** $^1$H NMR (300 MHz, $CDCl_3$): δ = 6.45 (d, *J* = 2.1 Hz, 2H), 6.39-6.31 (m, 2H), 5.73-5.64 (m, 1H), 3.82 (s, 14H), 2.39-2.32 (m, 2H), 1.52-1.42 (m, 2H), 1.40-1.25 (m, 6H) 0.91 (t, *J* = 7.0 Hz, 3H); (*Z*)-**8e**: $^{13}$C NMR (75 MHz, $CDCl_3$): δ = 160.5, 139.7, 133.8, 128.7, 106.9, 98.7, 55.3, 33.0, 31.9, 30.0, 29.6, 29.6, 29.4, 29.3, 28.8, 22.7, 14.1. MS

(ESI$^+$) calcd for $C_{20}H_{32}O_2$ + $H^+$, 305.24 [M + H]$^+$; found, 305.10.

*1-(3,5-Dimethoxyphenyl) Ethane (9a).* Compound **10** (1.0 mmol) and 3 mL $CH_3COOH$ were dissolved in 20 mL MeOH, and then, palladium on charcoal (10% w/w) was added to the solution. $H_2$ gas was introduced to the reaction mixture by a balloon at room temperature. The reaction was monitored by TLC. After the reaction was completed, the palladium on charcoal was filtered, and then, the organic phase was concentrated to afford pure product. Yield **9a:** 0.15 g, 92.3% $^1$H NMR (300 MHz, $CDCl_3$): δ = 6.43 (d, *J* = 2.3 Hz, 2H), 6.36 (t, *J* = 2.2 Hz, 1H), 3.83 (s, 6H), 2.65 (q, *J* = 7.6 Hz, 2H), 1.29 (t, *J* = 7.6 Hz, 3H); $^{13}$C NMR (75 MHz, $CDCl_3$): δ = 160.8, 146.8, 105.9, 97.6, 55.2, 29.3, 15.5. MS (ESI$^+$) calcd for $C_{10}H_{14}O_2$ + $H^+$, 195.13 [M + H]$^+$; found, 195.05.

*1-(3,5-Dimethoxyphenyl) Butane (9b).* Compound **8a** (1.0 mmol, 0.19 g) and palladium on charcoal (10% w/w) were added to MeOH (10 mL). $H_2$ gas was introduced to the reaction mixture by a balloon at room temperature. The reaction was monitored by TLC. After the reaction was completed, the palladium on charcoal was filtered, and then the organic phase was concentrated to afford colorless oil. Yield **9b:** 0.18 g, 91.8%, $^1$H NMR (300 MHz, CDCl3): δ = 6.34 (d, *J* = 2.1 Hz, 2H), 6.29 (t, *J* = 2.2 Hz, 1H), 3.77 (s, 6H), 2.62-2.45 (m, 2H), 1.58 (dt, *J* = 15.4, 7.5 Hz, 2H), 1.34 (dq, *J* = 14.5, 7.3 Hz, 2H), 0.91 (t, *J* = 7.3 Hz, 3H); $^{13}$C NMR (75 MHz, $CDCl_3$): δ = 160.9, 145.6, 106.7, 97.7, 55.4, 36.2, 33.6, 22.6, 14.2. MS (ESI$^+$) calcd for $C_{12}H_{18}O_2$ + $H^+$, 195.13 [M + H]$^+$; found, 195.05.

*1-(3,5-Dimethoxyphenyl) Hexane (9c).* Compound **9c** was prepared with **8b** according to the **9b** procedure to afford colorless oil, yield **9c:** 0.21 g, 93.5%, $^1$H NMR (300 MHz, $CDCl_3$): δ = 6.38 (d, *J* = 2.2 Hz, 2H), 6.33 (t, *J* = 2.2 Hz, 1H), 3.81 (s, 6H), 2.62-2.52 (m, 2H), 1.70-1.55 (m, 2H), 1.35 (t, *J* = 8.7 Hz, 6H), 0.92 (t, *J* = 6.7 Hz, 3H); $^{13}$C NMR (75 MHz, $CDCl_3$): δ = 160.9, 145.6, 106.6, 97.7, 55.4, 36.5, 31.9, 31.5, 29.2, 22.8, 14.3. MS (ESI$^+$) calcd for $C_{14}H_{22}O_2$ + $H^+$, 223.16 [M + H]$^+$; found, 223.07.

*1-(3,5-Dimethoxyphenyl) Butane (9d).* Compound **9d** was prepared with **8c** according to the 9b procedure to afford colorless oil, yield **9d:** 0.23 g, 97.1%, $^1$H NMR (300 MHz, $CDCl_3$): δ = 6.37 (d, *J* = 2.2 Hz, 2H), 6.32 (t, *J* = 2.2 Hz, 1H), 3.80 (s, 6H), 2.60-2.53 (m, 2H), 1.68-1.56 (m, 2H), 1.38-1.26 (m, 8H), 0.90 (t, *J* = 6.8 Hz, 3H); $^{13}$C NMR (75 MHz, $CDCl_3$): δ = 160.9, 145.6, 106.7, 97.7, 55.4, 36.5, 32.0, 31.5, 29.5, 29.4, 22.9, 14.3. MS (ESI$^+$) calcd for $C_{15}H_{24}O_2$ + $H^+$, 237.18 [M + H]$^+$; found, 237.09.

*1-(3,5-Dimethoxyphenyl) Octane (9e).* Compound **9e** was prepared with **8d** according to the **9b** procedure to afford colorless oil, yield **9e:** 0.24 g, 94.1%, $^1$H NMR (300 MHz, $CDCl_3$): δ = 6.34 (d, *J* = 2.2 Hz, 2H), 6.29 (t, *J* = 2.2 Hz, 1H), 3.77 (s, 6H), 2.58-2.49 (m, 2H), 1.67-1.52 (m, 2H), 1.28 (m, 10H), 0.88 (t, *J* = 6.7 Hz, 3H); $^{13}$C NMR (75 MHz, $CDCl_3$): δ = 160.7, 145.4, 106.5, 97.5, 55.2, 36.3, 31.9, 31.3, 29.5, 29.4, 29.3, 22.7, 14.1. MS (ESI$^+$) calcd for $C_{16}H_{26}O_2$ + $H^+$, 251.19 [M + H]$^+$; found, 251.10.

*1-(3,5-Dimethoxyphenyl) Dodecane (9f).* Compound **9f** was prepared with **8e** according to the **9b** procedure to afford colorless oil, yield **9f:** 0.29 g, 95.4%, $^1$H NMR (300 MHz, $CDCl_3$): δ = 6.34 (d, *J* = 2.2 Hz, 2H), 6.29 (t, *J* = 2.2 Hz, 1H), 3.77 (s, 6H), 2.59-2.49 (m, 2H), 1.58 (m, 2H), 1.27 (m, 18H), 0.87 (t, *J* = 5.1 Hz, 3H); $^{13}$C NMR (75 MHz, $CDCl_3$): δ = 160.9, 145.6, 106.6, 97.7, 55.4, 36.5, 32.1, 31.5, 29.9, 29.9, 29.8, 29.7, 29.6, 22.9, 14.3. MS (ESI$^+$) calcd for $C_{20}H_{34}O_2$ + $H^+$, 307.26 [M + H]$^+$; found, 307.17.

*1-(3,5-Dimethoxyphenyl) Ethan-1-ol (10).* Compound **6** (10 mmol, 1.66 g) was dissolved in 60 mL of dry THF, and $CH_3Li$ (12 mmol, 1.2 equiv) was dropped into the solution at -20 °C with stirring. After 30 min, the reaction was quenched by saturated aqueous $NH_4Cl$, and the reaction mixture was extracted by EtOAc (2× 50 mL). Then, the organic phase was washed with brine, dried by $Na_2SO_4$, and concentrated to give a faint yellow oil. Yield 10: 1.73 g, 95.12%, $^1$H NMR (300 MHz, $CDCl_3$): δ = 6.52 (d, *J* = 2.2 Hz, 2H), 6.36 (t, *J* = 2.3 Hz, 1H), 4.80 (q, *J* = 6.4 Hz, 1H), 3.79 (s, 6H), 2.53 (s, 1H), 1.47 (d, *J* = 6.5 Hz, 3H); $^{13}$C NMR (75 MHz, $CDCl_3$): δ = 160.9, 148.3, 103.3, 99.2, 70.5, 55.4, 25.1. MS (ESI$^+$) calcd for $C_{10}H_{14}O_3$ + $Na^+$, 205.09 [M + Na]$^+$; found, 204.97.

*1-(3,5-Dihydroxyphenyl) Ethane (11a).* Compound **9a** (2 mmol) was dissolved in 70 mL of dry $CH_2Cl_2$, and $BBr_3$ (6 mmol, 3 equiv, 1 mmol/mL $BBr_3$ in $CH_2Cl_2$) was dropped into the reaction mixture at -20 °C with stirring. After 15 min, the reaction mixture was transferred to room temperature and stirred for 6 h. Water was dropped in the reaction mixture at -20 °C to quench the reaction, the aqueous phase was extracted with EtOAc (2× 30 mL), and then the organic phase was washed with saturated $NaHCO_3$, dried with $Na_2SO_4$, and concentrated to afford colorless oil. Yield 11a: 0.24 g, 86.0%, $^1$H NMR (300 MHz, $CDCl_3$): δ = 6.28 (d, *J* = 5.0 Hz, 2H), 6.20 (t, *J* = 2.1 Hz, 1H), 4.82 (s, 2H), 2.55 (q, *J* = 7.6 Hz, 2H), 1.21 (t, *J* = 7.6 Hz, 3H); $^{13}$C NMR (75 MHz, $CDCl_3$): 8 = 156.6, 147.5, 107.5, 100.1, 28.7, 15.2. MS (ESI$^+$) calcd for $C_8H_{10}O_2$ + $H^+$, 139.07 [M + H]$^+$; found, 138.99.

*1-(3,5-Dihydroxyphenyl) Butane (11b).* Compound **11b** was prepared according to the **11a** procedure. Yield **11b:** colorless oil, 0.28 g, 84.5%, $^1$H NMR (300 MHz, $CDCl_3$): δ = 6.24 (d, *J* = 1.9 Hz, 2H), 6.16 (t, *J* = 2.0 Hz, 1H), 2.38 (t, *J* = 7.7 Hz, 2H), 1.52-1.37 (m, 2H), 1.25 (dq, *J* = 14.3, 7.2 Hz, 2H), 0.84 (t, *J* = 7.3 Hz, 3H); $^{13}$C NMR (75 MHz, $CDCl_3$): δ = 156.2, 146.6, 108.6, 100.5, 35.7, 33.3, 22.5, 14.1. MS (ESI$^-$) calcd for $C_{10}H_{14}O_2$ - $H^+$, 165.10 [M - H]$^-$; found, 165.01.

*1-(3,5-Dihydroxyphenyl) Hexane (11c).* Compound **11c** was prepared according to the **11a** procedure. Yield **11c:** colorless oil, 0.37 g, 94.1%, $^1$H NMR (300 MHz, $CDCl_3$): δ = 6.28 (d, *J*= 2.1 Hz, 2H), 6.20 (t, *J* = 2.2 Hz, 1H), 5.44 (s, 2H), 2.57-2.40 (m, 2H), 1.55 (dd, *J* = 14.7, 6.9 Hz, 2H), 1.34-1.25 (m, 6H), 0.89 (t, *J* = 6.6 Hz, 3H); $^{13}$C NMR (75 MHz, $CDCl_3$): δ = 156.4, 146.6, 108.4, 100.5, 36.0, 31.9, 31.2, 29.2, 22.8, 14.3. MS (ESI$^-$) calcd for $C_{12}H_{18}O_2$ - $H^+$, 193.13 [M - H]$^-$; found,

193.05.

*1-(3,5-Dihydroxyphenyl) Heptane (**11d**).* Compound **11d** was prepared according to the 11a procedure. Yield **11d:** colorless oil, 0.37 g, 89.6%, $^1H$ NMR (300 MHz, $CDCl_3$): δ = 6.26 (d, *J* = 2.2 Hz, 2H), 6.20 (t, *J* = 2.2 Hz, 1H), 4.87 (s, 2H), 2.55-2.44 (m, 2H), 1.67-1.51 (m, 2H), 1.29 (dt, *J* = 10.1, 4.4 Hz, 8H), 0.89 (t, *J* = 6.7 Hz, 3H); $^{13}C$ NMR (75 MHz, $CDCl_3$): δ = 156.2, 146.4, 108.3, 100.3, 35.8, 31.8, 31.1, 29.3, 29.2, 22.7, 14.1. MS ($ESI^-$) calcd for $C_{13}H_{20}O_2$ - $H^+$, 207.15 $[M - H]^-$; found, 207.06.

*1-(3,5-Dihydroxyphenyl) Octane (**11e**).* Compound 6e was prepared according to the **11a** procedure. Yield **11e:** colorless oil, 0.40 g, 90.1%, $^1H$ NMR (300 MHz, $CDCl_3$): δ = 6.25 (d, *J* = 1.9 Hz, 2H), 6.16 (t, *J* = 1.9 Hz, 1H), 5.95 (s, 2H), 2.39 (m, 2H), 1.68-1.48 (m, 2H), 1.34-1.23 (m, 10H), 0.86 (t, *J* = 6.6 Hz, 3H); $^{13}C$ NMR (75 MHz, $CDCl_3$): δ = 156.3, 146.7, 108.52, 100.5, 36.0, 32.1, 31.2, 29.7, 29.6, 29.5, 22.9, 14.3. MS ($ESI^-$) calcd for $C_{14}H_{22}O_2$ - $H^+$, 221.16 $[M - H]^-$; found, 221.08.

*1-(3,5-Dihydroxyphenyl) Dodecane (**11f**).* Compound **11f** was prepared according to the **11a** procedure. Yield **11f:** colorless oil, 0.49 g, 87.7%, $^1H$ NMR (300 MHz, $CDCl_3$): δ = 6.23 (d, *J= 2.0* Hz, 2H), 6.17-6.12 (m, 1H), 5.02 (s, 2H), 2.50-2.37 (m, 2H), 1.58-1.44 (m, 2H), 1.23 (s, 18H), 0.86 (t, *J* = 6.6 Hz, 3H); $^{13}C$ NMR (75 MHz, $CDCl_3$): δ = 156.5, 146.5, 108.4, 100.4, 36.1, 32.1, 31.3, 29.9, 29.9, 29.8, 29.8, 29.6, 22.9, 14.3. MS ($ESI^-$) calcd for $C_{18}H_{30}O_2$ - $H^+$, 277.22 $[M - H]^-$; found, 277.14.

*(1'R,2'R)-4-Ethyl-5'-methyl-2'-(prop-1-en-2-yl)-1',2',3',4'-tetra-hydro-[1,1'-biphenyl]-2,6-diol (**12a**).* Menthadienol (0.91 g, 6 mmol), compound **11a,** and HCOOH (0.28 g, 6 mmol, 1 equiv) were dissolved in 20 mL of $CH_2Cl_2$ with stirring at room temperature for 12 h. After the reaction was completed, the reaction mixture was quenched by saturated $NaHCO_3$, the aqueous phase was extracted by EtOAc (2× 20 mL), and the organic phase was washed with brine and dried with $Na_2SO_4$. The crude product was purified by flash chromatography eluting with petroleum ether/EtOAc 25:1 to give a colorless oil. Yield **12a:** 0.49 g, 17.7%, $^1H$ NMR (300 MHz, $CDCl_3$): δ = 6.33 (m, 2H), 6.11 (s, 1H), 5.57 (s, 1H), 5.30 (s, 1H), 4.66 (s, 1H), 4.57 (s, 1H), 3.94 (d, 1H), 2.58-2.38 (m, 3H), 2.37-2.04 (m, 2H), 1.89-1.78 (m, 5H), 1.71 (s, 3H), 1.18 (t, *J* = 7.6 Hz, 3H); $^{13}C$ NMR (75 MHz, $CDCl_3$): δ = 149.0, 144.3, 140.1, 124.2, 113.9, 111.0, 46.3, 36.8, 30.4, 28.4, 28.4, 23.8, 20.1, 15.1. MS ($ESI^-$) calcd for $C_{18}H_{24}O_2$ - $H^+$, 271.2 $[M - H]^-$; found, 271.2, $[a]_D^{20}$ = -55 (c = 1.0 mg/mL, $CHCl_3$), ee % = 99.9%.

*(1'R,2'R)-5'-Methyl-2'-(prop-1-en-2-yl)-4-propyl-1',2',3',4'-tetra-hydro-(1,1'-biphenyl]-2,6-diol (CBDV, 12b).* Compound **12b** was purchased from Xi'an Resarn Biotechnology Co., Ltd. $^1H$ NMR (300 MHz, $CDCl_3$): δ = 6.24 (m, 2H), 6.02 (s, 1H), 5.59 (s, 1H), 4.74 (s, 1H), 4.64 (d, *J* = 8.6 Hz, 2H), 3.87 (d, *J* = 7.0 Hz, 1H), 2.51-2.34 (m, 3H), 2.33-2.03 (m, 2H), 1.89-1.74 (m, 5H), 1.72-1.52 (m, 5H), 0.92 (t, *J* = 7.3 Hz, 3H); $^{13}C$ NMR (75 MHz, $CDCl_3$): δ = 149.4, 142.8, 140.1, 124.1, 113.8, 110.9, 46.2, 37.6, 37.2, 30.4, 28.4, 24.0, 23.7, 20.5, 13.8. MS ($ESI^-$) calcd for $C_{19}H_{26}O_2$ - $H^+$, 285.19 $[M - H]^-$; found, 285.09. $[a]D^{20}$ = -59 (c = 1.0 mg/mL, $CHCl_3$), ee % = 99.9%.

*(1'R,2'R)-4-Butyl-5'-methyl-2'-(prop-1-en-2-yl)-1',2',3',4'-tetra-hydro-[1,1'-biphenyl]-2,6-diol (12c).* Compound **12c** was prepared according to the **12a** procedure. Yield **12c:** 0.22 g, 14.8% as a colorless oil, $^1H$ NMR (300 MHz, $CDCl_3$): δ = 6.22 (m, 2H), 5.99 (s, 1H), 5.58 (s, 1H), 4.86 (s, 1H), 4.65 (s, 1H), 4.56 (s, 1H), 3.89 (d, *J* = 8.5 Hz, 1H), 2.53-2.33 (m, 3H), 2.35-2.05 (m, 2H), 1.91-1.74 (m, 5H), 1.39-1.16 (m, 7H), 0.93 (t, *J*= 7.3 Hz, 3H); $^{13}C$ NMR (75 MHz, $CDCl_3$): δ = 149.3, 143.0, 140.1, 124.1, 113.7, 110.9, 46.2, 37.1, 35.2, 33.1, 30.4, 28.4, 23.7, 22.3, 20.4, 14.0. MS ($ESI^-$) calcd for $C_{20}H_{28}O_2$ - $H^+$, 299.21; found $[M - H]^-$: 299.16. $[a]_D^{20}$ = -52 (c = 1.0 mg/mL, $CHCl_3$), ee % = 98.5%.

*(1'R,2'R)-5'-Methyl-4-pentyl-2'-(prop-1-en-2-yl)-1',2',3',4'-tetra-hydro-[1,1'-biphenyl]-2,6-diol (CBD, 12d).* Compound **12d** was purchased from Xi'an Resarn Biotechnology Co., Ltd. $^1H$ NMR (300 MHz, $CDCl_3$): δ = 6.24 (m, 2H), 6.02 (s, 1H), 5.59 (s, 1H), 4.77 (s, 1H), 4.68 (s, 1H), 4.58 (s, 1H), 3.88 (d, *J* = 10.2 Hz, 1H), 2.54-2.35 (m, 3H), 2.35-2.03 (m, 2H), 1.92-1.75 (m, 5H), 1.68 (s, 3H), 1.64-1.50 (m, 2H), 1.41-1.23 (m, 4H), 0.91 (t, *J* = 6.8 Hz, 3H); $^{13}C$ NMR (75 MHz, $CDCl_3$): δ = 149.4, 143.1, 140.1, 124.1, 113.8, 110.9, 46.2, 37.2, 35.5, 31.5, 30.7, 30.4, 28.4, 23.7, 22.6, 20.5, 14.1.). MS ($ESI^-$) calcd for $C_{21}H_{30}O_2$ - $H^+$, 313.22 $[M - H]^-$; found, 313.17. $[a]_D^{20}$ experimental = -130 (c = 10.0 mg/mL, EtOH), (lit. $[a]_D^{20}$ = -124 (10.0 mg/mL, EtOH),[58] ee % = 99.9%.

*(1'R,2'R)-4-Hexyl-5'-methyl-2'-(prop-1-en-2-yl)-1',2',3',4'-tetra-hydro-[1,1'-biphenyl]-2,6-diol (12e).* Compound **12e** was prepared according to the **12a** procedure. Yield **12e:** 0.29 g, 17.6% as a colorless oil, $^1H$ NMR (300 MHz, $CDCl_3$): δ = 6.22 (m, 2H), 5.99 (s, 1H), 5.58 (s, 1H), 5.18 (s, 1H), 4.65 (s, 1H), 4.56 (s, 1H), 3.90 (d, *J* = 11.3 Hz, 1H), 2.55-2.36 (m, 3H), 2.33-2.04 (m, 2H), 1.89-1.75 (m, 5H), 1.67 (s, 3H), 1.63-1.49 (m, 2H), 1.34-1.21 (m, 6H), 0.93 (t, *J*= 7.0 Hz, 3H); $^{13}C$ NMR (75 MHz, $CDCl_3$): δ = 149.2, 142.9, 140.0, 124.2, 113.8, 110.9, 58.5, 46.2, 37.0, 35.2, 33.1, 30.4, 28.4, 23.7, 22.4, 20.3, 18.4, 14.0. MS ($ESI^-$) calcd for $C_{22}H_{32}O_2$ - $H^+$, 327.24 $[M - H]^-$; found, 327.19. $[a]_D^{20}$ = -61 (c = 1.0 mg/mL, $CHCl_3$), ee % = 96.2%.

*(1'R,2'R)-4-Heptyl-5'-methyl-2'-(prop-1-en-2 yl)-1',2',3',4'-tetra-hydro-[1,1'-biphenyl]-2,6-diol **(12f).*** Compound **12f** was prepared according to the **12a** procedure. Yield **12f:** 0.25 g, 18.6% as a colorless oil, $^1H$ NMR (300 MHz, $CDCl_3$): δ = 6.23 (m, 2H), 6.02 (s, 1H), 5.59 (s, 1H), 4.90 (s, 1H), 4.67 (s, 1H), 4.57 (s, 1H), 3.95-3.83 (m, 1H), 2.52-2.35 (m, 3H), 2.31-2.02 (m, 2H), 1.90-1.74 (m, 5H), 1.68 (s, 3H), 1.63-1.50 (m, 2H), 1.26 (m, 8H), 0.91 (t, *J* = 7.2 Hz, 3H); $^{13}C$ NMR (75 MHz, $CDCl_3$): δ = 149.3, 143.0, 140.0, 124.2, 113.7, 110.9, 46.2, 37.1, 35.5, 31.8, 31.0, 30.4, 29.3, 29.2, 28.4, 23.7, 22.7, 20.4, 14.1. MS ($ESI^-$) calcd for $C_{23}H_{34}O_2$ - $H^+$, 341.26 $[M - H]^-$; found, 341.27. $[a]_D^{20}$ = -66 (c = 1.0 mg/mL, $CHCl_3$), ee % = 96.2%.

*(1'R,2'R)-5'-Methyl-4-octyl-2'-(prop-1-en-2-yl)-1',2',3',4'-tetra-hydro-[1,1'-biphenyl]-2,6-diol (**12g**).* Compound **12g** was prepared according to the **12a** procedure. Yield **12g:** 0.25 g, 14.2% as a colorless oil. $^1$H NMR (300 MHz, $CDCl_3$): $\delta$ = 6.26 (m, 2H), 6.04 (s, 1H), 5.59 (s, 1H), 4.95 (s, 1H), 4.65 (s, 1H), 4.55 (s, 1H), 3.88 (d, *J* = 11.7 Hz, 1H), 2.53-2.36 (m, 3H), 2.34-2.03 (m, 2H), 1.94-1.77 (m, 5H), 1.68 (s, 3H), 1.62-1.48 (m, 2H), 1.28 (s, 10H), 0.90 (t, *J* = 6.7 Hz, 3H); $^{13}$C NMR (75 MHz, $CDCl_3$): $\delta$ = 149.2, 143.0, 140.1, 124.1, 113.8, 110.9, 46.2, 37.1, 35.6, 31.9, 31.0, 30.4, 29.5, 29.4, 29.3, 28.4, 23.7, 22.7, 20.4, 14.2. MS (ESI$^-$) calcd for $C_{24}H_{36}O_2$ - $H^+$, 355.27 [M - H]$^-$; found, 355.24. $[a]_D^{20}$ = -60 (c = 1.0 mg/mL, $CHCl_3$), ee % = 99.8%.

*(1'R,2'R)-4-Dodecyl-5'-methyl-2'-(prop-1-en-2-yl)-1',2',3',4'-tet-rahydro-(1,1'-biphenyl]-2,6-diol **(12h).*** Compound **12h** was prepared according to the **12a** procedure. Yield **12h:** 0.41 g, 18.6% as a colorless oil, $^1$H NMR (300 MHz, $CDCl_3$): $\delta$ 6.28 (m, 2H), 6.02 (s, 1H), 5.59 (s, 1H), 4.75 (s, 1H), 4.68 (s, 1H), 4.58 (s, 1H), 3.89 (d, *J* = 11.7 Hz, 1H), 2.53-2.36 (m, 3H), 2.34-2.04 (m, 2H), 1.91-1.74 (m, 5H), 1.68 (s, 3H), 1.64-1.49 (m, 2H), 1.40-1.16 (m, 18H), 0.90 (t, *J* = 6.7 Hz, 3H); $^{13}$C NMR (75 MHz, $CDCl_3$): $\delta$ = 149.4, 143.1, 140.1, 124.1, 113.7, 110.9, 46.2, 37.2, 35.5, 32.0, 31.0, 30.4, 29.7, 29.7, 29.6, 29.6, 29.4, 29.3, 28.4, 23.7, 22.7, 20.5, 14.2. MS (ESI$^-$) calcd for $C_{28}H_{44}O_2$ - $H^+$, 411.3 [M - H]$^-$; found, 411.4. $[a]_D^{20}$ = -74 (c = 1.0 mg/mL, $CHCl_3$), ee % = 99.9%.

## EXAMPLE 4

### General Procedure for the Synthesis of CIAC and Compounds 19b-19d

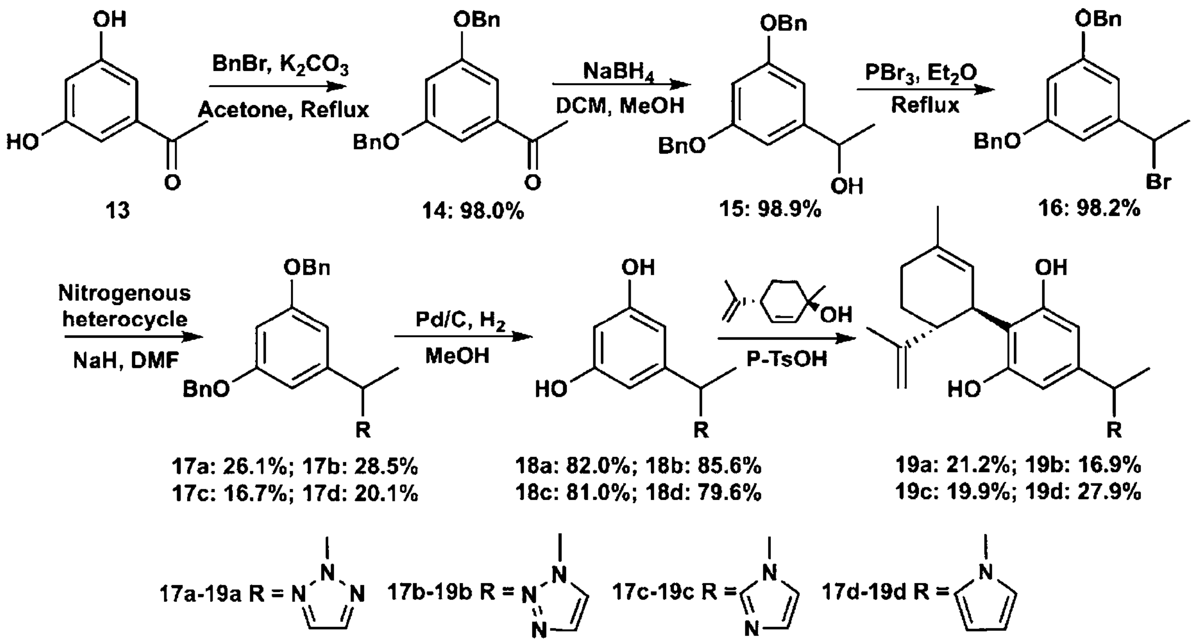


This example covers the synthesis of analogues of Compound 12 with various side chain functionalizations. Briefly, the synthesis of Compounds 19a-19d began with 3,5-dihydroxyacetophenone (13) and benzyl bromide, and then intermediate 14 was reduced to give compound 15 by $NaBH_4$. Replacement of the $\alpha$-OH group of 15 with Br was carried out in the presence of phosphorus tribromide to generate the intermediate 16. Nucleophilic substitution reactions of pyrrole, imidazole, and triazoles with 16 were performed to produce 17a-17d, which were then subjected to benzyl deprotection, leading to 18a-18d. CBD derivatives 19a-19d were obtained from the Friedel-Crafts alkylation reaction of 18a-18d with menthadienol.

*1-(3,5-Bis(benzyloxy)phenyl) Ethan-1-one (14).* Compound **13** (1.52 g, 10 mmol), benzyl bromide (4.28 g, 25 mmol, 2.5 equiv), and $K_2CO_3$ (1.38 g, 10 mmol, 1 equiv) were added to 60 mL acetone. The mixture was refluxed for 24 h; then, 10 mL $Et_3N$ was added to the mixture and refluxed for 30 min. The reaction mixture was diluted with water, the aqueous phase was extracted with EtOAc, and the organic phase was washed with brine and dried with $Na_2SO_4$. The organic phase was concentrated and yielded 14: 3.25 g, 98%. $^1$H NMR (300 MHz, $CDCl_3$): $\delta$ = 7.53-7.33 (m, 10H), 7.24 (d, *J* = 2.3 Hz, 2H), 6.85 (t, *J* = 2.3 Hz, 1H), 5.10 (s, 4H), 2.59 (s, 3H); $^{13}$C NMR (75 MHz, $CDCl_3$): $\delta$ = 197.8, 160.0, 139.1, 136.4, 128.7, 128.2, 127.7, 107.4, 106.9, 70.4, 26.8. MS (ESI$^+$) calcd for $C_{22}H_{20}O_3$ + $H^+$, 333.14 [M + H]$^+$; found, 332.94.

*1-(3,5-Bis(benzyloxy)phenyl) Ethan-1-ol (15).* Compound **14** (3.17 g, 9.5 mmol) was dissolved in 80 mL of

solution [DCM/ MeOH = 20:1 (v/v)], and $NaBH_4$ (0.45 g, 12 mmol, 1.25 equiv) was slowly added to the solution at -20 °C. After 30 min, the reaction mixture was transferred to room temperature and stirred for 1 h. The reaction was quenched by water and extracted by EtOAc (3× 70 mL), and the organic phase was washed with brine and dried with Na2SO4. The organic phase was concentrated, and the yield was **15:** 3.14 g, 98.9%. $^1H$ NMR (300 MHz, $CDCl_3$): δ = 7.51-7.31 (m, 10H), 6.66 (d, *J* = 2.1 Hz, 2H), 6.56 (t, *J* = 2.2 Hz, 1H), 5.05 (s, 4H), 4.83 (q, *J* = 6.4 Hz, 1H), 1.48 (d, *J* = 6.4 Hz, 3H); $^{13}C$ NMR (75 MHz, $CDCl_3$): δ = 160.1, 148.6, 136.9, 128.7, 128.1, 127.7, 104.5, 101.0, 70.5, 70.1, 25.2. MS ($ESI^-$) calcd for $C_{22}H_{22}O_3$ - $H^+$, 333.16 $[M - H]^-$; found, 332.94.

*(((5-(1-Bromoethyl)-1,3-phenylene)bis(oxy))bis(methylene))-dibenzene (16).* Compound **15** (3.01 g, 9 mmol) was dissolved in 200 mL of dry ether, $PBr_3$ (13.5 mmol, 1.5 equiv) was added, and then the reaction mixture was heated to reflux for 5 h. The reaction mixture was quenched by saturated $NaHCO_3$ at -20 °C; the water phase was extracted by EtOAc, and the organic phase was washed with brine and dried with $Na_2SO_4$. The organic phase was concentrated and yielded **16:** 3.50 g, 98.2%. $^1H$ NMR (300 MHz, $CDCl_3$): δ = 7.53-7.32 (m, 10H), 6.75 (d, *J* = 2.1 Hz, 2H), 6.59 (t, *J* = 2.1 Hz, 1H), 5.16 (q, *J* = 6.9 Hz, 1H), 5.07 (s, 4H), 2.05 (d, *J* = 6.9 Hz, 3H); $^{13}C$ NMR (75 MHz, $CDCl_3$): δ = 160.0, 145.5, 136.7, 128.7, 128.2, 127.7, 106.2, 101.8, 70.2, 49.5, 26.8. MS ($ESI^+$) calcd for $C_{22}H_{21}BrO_2$ + $H^+$, 397.07 $[M + H]^+$; found, 396.93.

*(1-(3,5-Bis(benzyloxy)phenyl)ethyl)-2H-1,2,3-triazole (**17a**) and (1-(3,5-Bis(benzyloxy)phenyl)ethyl)-1H-1,2,4-triazole (**17b**).* 2H-1,2,3-Triazole (0.83 g, 12 mmol, 1.5 equiv) was dissolved in dry DMF, and NaH (0.36 g, 15 mmol, 1.87 equiv) was slowly added to the solution at -20 °C. After 30 min, compound **16** (3.17 g, 8 mmol) was added, and then the reaction mixture was stirred at 110 °C for 24 h. After the reaction was completed, the reaction was diluted with saturated $NH_4Cl$, the water phase was extracted with ether, and the organic matter was washed with brine and dried with $Na_2SO_4$. The crude material was purified by flash chromatography eluting with petroleum ether/EtOAc. Yield **17a:** 0.80 g, 26.1%, $^1H$ NMR (300 MHz, CDCl3): δ = 7.62 (s, 2H), 7.46-7.28 (m, 10H), 6.51 (s, 3H), 5.79 (q, *J* = 7.1 Hz, 1H), 4.97 (s, 4H), 1.96 (d, *J* = 7.1 Hz, 3H); $^{13}C$ NMR (75 MHz, $CDCl_3$): δ = 160.2, 143.3, 136.7, 134.2, 128.7, 128.2, 127.7, 105.7, 101.4, 70.2, 64.3, 21.3. MS ($ESI^+$) calcd for $C_{24}H_{23}N_3O_2$ + $H^+$, 408.18 $[M + Na]^+$; found, 408.26. Yield 17b: 0.88 g, 28.5%. $^1H$ NMR (300 MHz, $CDCl_3$): δ = 7.66 (s, 1H), 7.45 (s, 1H), 7.41-7.25 (m, 9H), 6.58 (t, *J*= 2.0 Hz, 1H), 6.50 (d, *J= 2.0* Hz, 2H), 5.73 (q, *J* = 7.0 Hz, 1H), 4.96 (s, 4H), 1.90 (d, *J* = 7.1 Hz, 3H); $^{13}C$ NMR (75 MHz, $CDCl_3$): δ = 160.0, 142.2, 136.3, 133.6, 128.4, 127.9, 127.4, 122.2, 105.6, 101.3, 69.8, 59.7, 21.0. MS ($ESI^+$) calcd for $C_{24}H_{23}N_3O_2$ + $Na^+$, 408.18 $[M + Na]^+$; found, 408.27.

*1-(1-(3,5-Bis(benzyloxy)phenyl)ethyl)-1H-imidazole (**17c**).* Compound **17c** was prepared with 16 (3.57 g, 9 mmol) and 1H-imidazole (0.92 g, 13.5 mmol) according to the **17a** procedure. Yield **17c:** 0.58 g, 16.7%, $^1H$ NMR (300 MHz, $CDCl_3$): δ = 7.59 (s, 1H), 7.45-7.29 (m, 10H), 7.09 (s, 1H), 6.91 (s, 1H), 6.55 (t, *J* = 2.2 Hz, 1H), 6.37 (d, *J* = 2.1 Hz, 2H), 5.25 (q, *J* = 6.9 Hz, 1H), 4.98 (s, 4H), 1.81 (d, *J* = 7.0 Hz, 3H); $^{13}C$ NMR (75 MHz, $CDCl_3$): δ = 160.4, 144.1, 136.5, 136.2, 129.5, 128.7, 128.2, 127.7, 118.1, 105.5, 101.2, 70.2, 56.6, 22.0. MS ($ESI^+$) calcd for $C_{25}H_{24}N_2O_2$ + $H^+$, 385.19 $[M + H]^+$; found, 385.06.

*1-(1-(3,5-Bis(benzyloxy)phenyl)ethyl)-1H-pyrrole (**17d**).* Compound **17d** was prepared with **16** (3.57 g, 9 mmol) and 1H-pyrrole (0.91 g, 13.5 mmol) according to the **17a** procedure. Yield **17d:** 0.69 g, 20.1%, $^1H$ NMR (300 MHz, $CDCl_3$): δ = 7.59-7.39 (m, 10H), 6.88 (t, *J* = 2.0 Hz, 2H), 6.65 (t, *J* = 2.0 Hz, 1H), 6.49 (d, *J* = 2.1 Hz, 2H), 6.34 (t, *J* = 1.9 Hz, 2H), 5.29 (q, *J* = 7.0 Hz, 1H), 5.07 (s, 4H), 1.90 (d, *J* = 7.1 Hz, 3H); $^{13}C$ NMR (75 MHz, $CDCl_3$): δ = 160.1, 146.1, 136.7, 128.6, 128.1, 127.7, 119.5, 108.2, 105.3, 100.6, 70.0, 58.1, 22.0. MS ($ESI^+$) calcd for $C_{26}H_{25}NO_2$ + $Na^+$, 406.19 $[M + Na]^+$; found, 406.04.

*5-(1-(2H-1,2,3-Triazol-2-yl)ethyl)benzene-1,3-diol (18a).* Compound **17a** (0.46 g, 1.2 mmol) and palladium on charcoal (10% w/ w) were added to MeOH (20 mL). $H_2$ gas was introduced to the reaction mixture by a balloon at room temperature. The reaction was monitored by TLC. After the reaction was completed, the reaction mixture was filtered and concentrated to give colorless oil, yield 18a 0.20 g, 82.0%, $^1H$ NMR (300 MHz, $CDCl_3$): δ = 7.58 (s, 2H), 7.12 (s, 2H), 6.15 (d, *J* = 1.9 Hz, 2H), 6.08 (t, *J* = 2.0 Hz, 1H), 5.68 (q, *J* = 7.0 Hz, 1H), 1.87 (d, *J* = 7.1 Hz, 3H); $^{13}C$ NMR (75 MHz, $CDCl_3$): δ = 157.2, 143.3, 134.2, 105.9, 102.8, 64.3, 21.0. MS ($ESI^-$) calcd for $C_{10}H_{11}N_3O_2$ - $H^+$, 204.09 $[M - H]^-$; found, 203.99.

*5-(1-(1H-1,2,4-Triazol-1-yl)ethyl)benzene-1,3-diol (**18b**).* Compound **18b** was prepared according to the procedure described for **18a.** Yield **18b:** 0.17 g, 85.6% as a colorless oil. $^1H$ NMR (300 MHz, MeOD): δ = 9.37 (s, 1H), 9.20 (s, 1H), 7.79 (t, *J* = 2.1 Hz, 1H), 7.75 (d, *J* = 2.1 Hz, 2H), 7.22 (q, *J* = 7.0 Hz, 1H), 6.52 (s, 2H), 3.37 (d, *J* = 7.1 Hz, 3H); $^{13}C$ NMR (75 MHz, MeOD): δ = 160.0, 144.2, 134.3, 124.8, 105.9, 103.4, 61.5, 21.6. MS ($ESI^-$) calcd for $C_{10}H_{11}N_3O_2$ - $H^+$, 204.09 $[M - H]^-$; found, 203.97.

*5-(1-(1H-Imidazole-1-yl)ethyl)benzene-1,3-diol (**18c**).* Compound 18c was prepared according to the procedure described for **18a.** Yield **18c:** 0.21 g, 81.0% as a colorless oil. $^1H$ NMR (300 MHz, MeOD): δ = 7.74 (s, 1H), 7.08 (s, 1H), 6.93 (s, 1H), 6.11 (t, *J*= 2.1 Hz, 1H), 6.06 (d, *J*= 2.1 Hz, 2H), 5.26 (q, *J* = 7.0 Hz, 1H), 1.71 (d, *J* = 7.1 Hz, 3H). MS ($ESI^-$) calcd for $C_{11}H_{12}N_2O_2$ - $H^+$, 203.09 $[M - H]^-$; found, 203.00.

*5-(1-(1H-Pyrrol-1-yl)ethyl)benzene-1,3-diol (**18d**).* Compound **18d** was prepared according to the **18a** procedure. Yield **18d:** 0.36 g, 79.6% as a colorless oil. $^1H$ NMR (300 MHz, MeOD): δ = 6.71 (t, *J= 2.1* Hz, 2H), 6.18-5.98 (m, 5H), 5.10 (q, *J* = 7.0 Hz, 1H), 1.68 (d, *J* = 7.1 Hz, 3H); $^{13}C$ NMR (75 MHz, MeOD): δ = 159.7, 147.9, 120.5, 108.7, 105.7, 102.5, 59.2, 22.5. MS ($ESI^+$) calcd for $C_{12}H_{13}NO_2$ + $K^+$, 242.19 $[M + K]^+$; found, 242.18.

(1'R,2'R)-4-(1-(2H-1,2,3-Triazol-2-yl)ethyl)-5'-methyl-2'-(prop-1-en-2-yl)-1',2',3',4'-tetrahydro-[1,1'-biphenyl]-2,6-diol (**19a,** CIAC001). Compound **18a** (0.10 g, 0.5 mmol) and menthadienol (0.15 g, 1 mmol, 2 equiv) were

dissolved in 20 mL of DCE, p-TsOH (0.02 g, 0.1 mmol, 0.2 equiv) was added, and the reaction mixture was stirred at room temperature. After 2 h, the reaction was quenched by saturated $NaHCO_3$, the water phase was extracted by EtOAc, and the organic phase was washed with brine and dried with $Na_2SO_4$. The crude product was purified by flash chromatography eluting with petroleum ether/EtOAc. Yield **19a:** 0.04 g, 21.2%. $^1H$ NMR (300 MHz, $CDCl_3$): δ = 7.49 (s, 2H), 6.22 (s, 1H), 5.96 (s, 2H), 5.71 (s, 1H), 5.65-5.55 (m, 1H), 5.40 (s, 1H), 4.47 (s, 1H), 4.37 (s, 1H), 3.79 (d, *J* = 9.6 Hz, 1H), 2.35-2.22 (m, 1H), 2.14-1.90 (m, 2H), 1.85-1.75 (m, 3H), 1.72-1.60 (m, 5H), 1.54 (s, 3H); $^{13}C$ NMR (75 MHz, $CDCl_3$): δ = 148.6, 140.6, 140.3, 134.0, 123.9, 116.5, 111.2, 77.4, 63.9, 46.2, 36.7, 30.4, 28.4, 23.8, 20.0. MS ($ESI^-$) calcd for $C_{20}H_{25}N_3O_2$ - $H^+$, 338.19 $[M - H]^-$; found, 338.14. $[a]_D^{20}$ = -56 (c = 1.0 mg/mL, $CHCl_3$), ee % = 99.1%.

*(1'R,2'R)-4-(1-(1H-1,2,4-Triazol-1-yl)ethyl)-5'-methyl-2'-(prop-1-en-2-yl)-1',2',3',4'-tetrahydro-[1,1'-biphenyl]-2,6-diol* ***(19b).*** Compound **19b** was prepared according to the **19a** procedure except that the reaction temperature was 70 °C. Yield **19b:** 0.25 g, 16.9%. $^1H$ NMR (300 MHz, $CDCl_3$): δ = 8.91 (s, 1H), 7.60 (s, 1H), 7.44 (d, *J* = 2.3 Hz, 1H), 6.43-6.33 (m, 2H), 6.18 (s, 1H), 5.67 (dq, *J* = 14.0, 7.0 Hz, 1H), 5.54 (s, 1H), 4.54 (s, 1H), 4.45 (s, 1H), 4.05 (d, *J* = 8.8 Hz, 1H), 2.45 (td, *J* = 9.8, 5.4 Hz, 1H), 2.18 (dt, *J* = 37.9, 17.8 Hz, 2H), 1.92 (t, *J* = 7.5 Hz, 3H), 1.82-1.76 (m, 5H), 1.68 (d, *J* = 13.1 Hz, 3H); $^{13}C$ NMR (75 MHz, CDCl3): δ = 156.6, 148.0, 140.0, 139.0, 133.4, 124.2, 122.8, 117.1, 111.2, 106.2, 77.4, 60.4, 46.5, 30.5, 28.3, 23.8, 20.7, 19.4.) MS ($ESI^-$) calcd for $C_{20}H_{25}N_3O_2$ - $H^+$, 338.19 $[M- H]^-$; found, 338.14. $[a]_D^{20}$ = -68 (c = 1.0 mg/mL, CHCl3), ee % = 99.0%.

(1'R,2'R)-4-(1-(1H-Imidazole-1-yl)ethyl)-5'-methyl-2'-(prop-1-en-2-yl)-1',2',3',4'-tetrahydro-[1,1'-biphenyl]-2,6-diol **(19c).** Compound 19c was prepared according to the **19a** procedure except that the reaction temperature was 70 °C. Yield **19c:** 0.27 g, 19.9%. δ = 8.64 (s, 1H), 8.32 (s, 1H), 7.09 (s, 1H), 6.37 (s, 2H), 5.50 (s, 1H), 5.35 (m, 1H), 4.50 (s, 1H), 4.42 (s, 1H), 4.03 (d, *J* = 7.0, 1H), 2.45 (td, *J* = 10.0, 4.8, 1H), 2.21 (m, 2H), 1.91-1.74 (m, 8H), 1.68 (s, 3H). $^{13}C$ NMR (75 MHz, $CDCl_3$): δ = 156.8, 147.9, 139.9, 137.4, 135.9, 123.7, 111.1, 77.2, 50.7, 30.3, 28.2, 23.7, 20.9, 19.1. MS ($ESI^-$) calcd for $C_{21}H_{26}N_2O_2$ - $H^+$, 337.20 $[M - H]^-$; found, 337.09. $[\alpha]_D^{20}$ = -72 (c = 1.0 mg/mL, $CHCl_3$), ee % = 99.7%.

*(1'R,2'R)-4-(1-(1H-Pyrrol-1-yl)ethyl)-5'-methyl-2'-(prop-1-en-2-yl)-1',2',3',4'-tetrahydro-[1,1'-biphenyl]-2,6-diol* ***(19d).*** Compound **19d** was prepared according to the **19a** procedure. Yield **19b:** 0.30 g, 27.9%. $^1H$ NMR (300 MHz, $CDCl_3$): δ = 6.75 (t, *J* = 2.1 Hz, 2H), 6.28 (s, 1H), 6.19 (t, *J* = 2.1 Hz, 2H), 6.01 (d, *J* = 20.6 Hz, 1H), 5.55 (s, 1H), 5.13 (q, *J* = 7.0 Hz, 1H), 4.83 (s, 1H), 4.65 (dd, *J* = 5.9, 4.2 Hz, 1H), 4.54 (s, 1H), 3.89 (d, *J* = 8.8 Hz, 1H), 2.41 (td, *J* = 10.6, 3.8 Hz, 1H), 2.18 (m, 2H), 1.79 (m, 8H), 1.67 (s, 3H); $^{13}C$ NMR (75 MHz, $CDCl_3$): δ = 148.8, 148.8, 143.5, 143.4, 140.4, 123.7, 119.6, 115.6, 111.1, 107.9, 57.6, 46.1, 36.9, 30.4, 28.3, 23.7, 21.9, 20.2, 20.1. MS ($ESI^-$) calcd for $C_{22}H_{27}NO_2H^+$, 336.20 $[M - H]^-$; found, 336.15. $[a]_D^{20}$ = -77 (c = 1.0 mg/mL, $CHCl_3$), ee % = 99.9%.

## EXAMPLE 5

### Structure-Activity Relationships of Compounds 1a-5a and 1b-5b

This example covers structure-activity relationships of CBD derivatives with various hydroxyl functionalizations. The phenolic hydroxyl groups of CBD play a critical role in bioactivities and cytotoxicity. EXAMPLE 2 covers synthesis of Compounds 1a-5a and 1b-5b by different halogenated alkanes in the presence of $K_2CO_3$ to produce CBD phenolic derivatives.

To quantitively investigate the in vitro anti-neuroinflammatory activities of CBD derivatives, the BV-2 microglial cell line was employed, as these cells retain morphological, phenotypic, and functional properties associated with primary microglia with high fidelity. Microglia are CNS immunocompetent cells. Activation of microglia leads to the release of proinflammatory factors and contributes to the main part of neuroinflammation.

The anti-neuroinflammatory activities of monoalkylated CBD derivatives (Compounds 1a-5a) are reflected by the $IC_{50}$ (half maximal inhibitory concentration) of the compounds inhibiting LPS-induced proinflammatory factor nitric oxide (NO) overproduction in BV-2 cells (TABLE 2), decreased significantly with phenolic hydroxyl monoalkylated substitution. CBD derivatives 1b-5b, where two phenolic hydroxyl groups were alkylated, showed no anti-neuroinflammatory activities (>200 μM, TABLE 3). These findings suggest that the phenolic hydroxyl groups of CBD are essential for its anti-neuroinflammatory activities. In the next structure-activity relationship (SAR) study, phenolic hydroxyl groups will be retained.

**TABLE 3**

| Compound | Antineuroin-flammatory activity (μM)[a] | Cellular toxicity (μM)[b] | Therapeutic index (TI)[c] |
|---|---|---|---|
| **1a** | 37.2 ± 8.8 | 58.2 ± 10.2 | 1.6 ± 0.5 |
| **2a** | 167.3 ± 16.9 | 176.3 ± 7.4 | 1.1 ± 0.1 |
| **3a** | >200 | >200 | n/a[d] |
| **4a** | 59.8 ± 15.6 | 71.5 ± 9.4 | 0.7 ± 0.1 |

(continued)

| Compound | Antineuroin-flammatory activity (μM)[a] | Cellular toxicity (μM)[b] | Therapeutic index (TI)[c] |
|---|---|---|---|
| **5a** | 99.6 ± 16.3 | 65.0 ± 7.9 | 1.2 ± 0.4 |
| **1b** | >200 | >200 | n/a[d] |
| **2b** | >200 | >200 | n/a[d] |
| **3b** | >200 | >200 | n/a[d] |
| **4b** | >200 | >200 | n/a[d] |
| **5b** | >200 | >200 | n/a[d] |

[a]Anti-neuroinflammatory activity was shown by the half-maximal inhibitory concentration ($IC_{50}$) of the CBD analogue inhibiting the LPS-induced proinflammatory factor nitric oxide (NO) in immunocompetent BV-2 cells. [b]Cellular toxicity was indicated by the $IC_{50}$ of the CBD analogue inhibiting BV-2 cellular viability. [c]Therapeutic index (TI) was determined by the following equation: TI = $IC_{50}$ of cellular viability/$IC_{50}$ of NO. [d]Not applicable

## EXAMPLE 6

## Structure-Activity Relationships of Compounds 12a-12h

This example covers the effects of side chain functionalizations on CBD activity. The side chain of CBD is essential for its bioactivities in the SAR study. Therefore, the CBD derivatives Compounds **12a-12h** were designed to interrogate the structure-activity relationships stemming from linear alkane side chains of different lengths. The anti-neuroinflammatory activities of CBD derivatives **12a-12h at** large decreased with the increasing length of the linear alkane chain of CBD derivatives except for **12e** (TABLE 4). Meanwhile, the therapeutic index of CBD derivatives **12a-12h** showed a similar trend.

**TABLE 4**

| Compound | Antineuroin-flammatory activity (μM)[a] | Cellular toxicity (μM)[b] | Therapeutic index (TI)[c] |
|---|---|---|---|
| **12a** | 2.5 ± 0.3 | 22.6 ± 1.6 | 9.1 ± 1.4 |
| **12b** | 5.3 ± 2.9 | 18.3 ± 4.4 | 3.5 ± 2.0 |
| **12c** | 5.0 ± 3.5 | 11.1 ± 2.8 | 2.2 ± 1.6 |
| **12d (CBD)** | 12.3 ± 3.1 | 8.5 ± 0.9 | 0.7 ± 0.2 |
| **12e** | 2.1 ± 0.4 | 19.0 ± 0.5 | 9.0 ± 1.9 |
| **12f** | 42.0 ± 9.3 | 14.1 ± 0.7 | 0.3 ± 0.1 |
| **12g** | 174.4 ± 33.0 | 26.2 ± 5.2 | 0.2 ± 0.1 |
| **12h** | >200 | >200 | n/a[d] |

[a]Anti-neuroinflammatory activity was shown by the half-maximal inhibitory concentration ($IC_{50}$) of the CBD analogue inhibiting the LPS-induced proinflammatory factor nitric oxide (NO) in immunocompetent BV-2 cells. [b]cellular toxicity was indicated by the $IC_{50}$ of the CBD analogue inhibiting BV-2 cellular viability. [c]Therapeutic index (TI) was determined by the following equation: TI = $IC_{50}$ of cellular viability/$IC_{50}$ of NO. [d]Not applicable.

## EXAMPLE 7

## Structure-Activity Relationships of Compounds 19a-19d

Compound **12a,** which has the side chain of the ethyl group, showed the best therapeutic index among CBD derivatives **12a-12h.** Therefore, this compound was further explored with side chain heterocycle functionalizations. Heterocycles are key pharmacophores of many drugs approved for various therapies. As tiazole-substituted side chains can enhance hydrophilicity, it was explored herein whether polar heterocyclic introduction may lead to improved therapeutic effects. Specifically, five-membered nitrogen heterocycles were introduced at the $\alpha$-C position of Compound 12a as outlined in EXAMPLE 4.

Among the four compounds tested, CIAC001, in which 2H-1,2,3-triazole pharmacophore was introduced at $\alpha$-C position, had the most potent anti-neuroinflammatory activity (2.5 ± 0.7 μM) and showed the best therapeutic index (TABLE 5). The anti-neuroinflammatory activity of CIAC001 did not significantly increase compared to Compound 12a. However, the cellular toxicity of CIAC001 was twice less than Compound 12a, which indicates the introduction of 2H-1,2,3-triazole pharmacophore ameliorates cytotoxicity and improves the therapeutic window. Compound 19b, where 1*H*-1,2,3-triazole pharmacophore was introduced instead, exerted about 14.4-fold less anti-neuroinflammatory activity when compared to CIAC001. Considering the subtle structural changes among triazole pharmacophores in 19a and 19b, the results support that the side chain functional group exerts a critical effect on SAR of CBD analogues.

**TABLE 5**

| Compound | Antineuroin-flammatory activity (μM)[a] | Cellular toxicity (μM)[b] | Therapeutic index (TI)[c] |
|---|---|---|---|
| **19a (CIAC001)** | 2.5 ± 0.7 | 57.8 ± 7.6 | 23.1 ± 6.6 |
| **19b** | 35.9 ± 9.6 | 77.1 ± 6.5 | 2.1 ± 0.6 |
| **19c** | 4.4 ± 0.5 | 32.6 ± 3.2 | 7.4 ± 1.2 |
| **19b** | 23.9 ± 5.8 | 154.7 ± 14.3 | 6.5 ± 1.7 |

[a]Anti-neuroinflammatory activity was shown by the half-maximal inhibitory concentration ($IC_{50}$) of the CBD analogue inhibiting the LPS-induced proinflammatory factor nitric oxide (NO) in immunocompetent BV-2 cells. [b]Cellular toxicity was indicated by the $IC_{50}$ of the CBD analogue inhibiting BV-2 cellular viability. [c]Therapeutic index (TI) was determined by the following equation: TI = $IC_{50}$ of cellular viability/IC50 of NO.

**[0191]** In summary, the CBD derivative CIAC001, which showed 4.9-fold increased anti-neuroinflammatory activity and 6.8-fold less cytotoxicity when compared to CBD, was discovered through the SAR investigation of CBD side chains. Meanwhile, the therapeutic index of CIAC001 was improved ~30-fold, which increases its therapeutic window for in vitro cellular studies and in vivo animal investigations. Therefore, CIAC001, which is the most effective and least toxic CBD derivative, was selected as the lead compound for the more in-depth in vitro and in vivo investigations.

## EXAMPLE 8

### In Vitro Anti-neuroinflammatory Activity of CIAC001

This example covers the effects of a representative compound of the present disclosure, CIAC001, on microglia signaling. Activated microglia secrete proinflammatory factors such as IL-1$\beta$, TNF-$\alpha$, and IL-6. CIAC001 concentration-dependently inhibited LPS-induced overexpression of IL-1β (FIG. 1A), TNF-$\alpha$ (FIG. 1B), and IL-6 (FIG. 1C). In contrast, CBD failed to inhibit LPS-induced TNF-$\alpha$ and IL-6 mRNAs overexpression and inhibited IL-1$\beta$ overexpression only at 10 μM. Furthermore, it should be noted that 10 μM CBD even augmented LPS-induced IL-6 mRNA expression. This is not surprising considering 10 μM CBD might cause a toxic stress response.

Microglia alter cell morphological phenotypes in response to stimuli. Most of the untreated BV-2 cells were characterized by small, round cells. The LPS-activated cells had enlarged bodies, and the percentage of amoeboid-shaped microglia increased significantly, while CIAC001 considerably reversed the LPS-induced morphological changes (FIG. 1D). These results indicate that CIAC001 inhibits microglial activation. In addition to the cell morphology analysis, the effect of CIAC001 on M1 and M2 polarization of microglia was further investigated. M1 microglia produce inflammatory mediators and induce neuroinflammation, while M2 microglia release antiinflammatory factors and induce neuroprotection. Expression levels of M1/M2 markers were measured at the mRNA level by qPCR. CIAC001 inhibited LPS-induced increased expression of M1 phenotype markers IL-1β and iNOS and attenuated LPS-induced decreased expression of M2 phenotype marker IL-10 **(FIG. 7).** Collectively, these in vitro cellular assays consistently showed that CIAC001 exhibited potent anti-neuroinflammatory activity, which was worthy of in vivo studies.

## EXAMPLE 9

### Therapeutic Effect of CIAC001 for Morphine Addiction

This example covers the effects of a representative compound of the present disclosure on morphine addiction, withdrawal, and associated pharmacokinetics. Several studies have demonstrated the role of microglia as neuronal modulators of drug reward. Opioids activate microglia and induce proinflammatory factors within the reward circuitries, which increase neuronal excitability and augment mesolimbic dopamine signaling, thus contributing to addiction.

Therefore, it was contemplated herein that pharmacological inhibition of neuro-inflammation has been a feasible strategy for altering the behavioral consequences associated with opioid addiction.

The effect of Compound 19 on opioid withdrawal was investigated by naloxone-precipitated morphine withdrawal behavior **(FIG. 8A).** Naloxone induced frequent jumping after chronic morphine in mice. CIAC001 (2 μg/kg, 20 μg/kg, and 0.2 mg/kg, i.p.) dose-dependently attenuated naloxone-precipitated withdrawal jumps in morphine-dependent mice, while CBD failed to significantly alleviate morphine withdrawal symptoms **(FIG. 2A).** These results suggest that CIAC001 is effective for treating morphine withdrawal treatment and preventing the progression of physical dependence.

Behavioral sensitization, a phenomenon in which locomotor activity increases over time following repeated exposure to psychostimulants, has been a useful model for evaluating the therapeutic efficacy of pharmacological agents for the treatment of drug addiction. Therefore, a morphine-induced behavioral sensitization assay was performed **(FIG. 8B).** Following repeated daily morphine exposure, the mice exhibited increased locomotor activity **(FIG. 2B).** CIAC001 (0.2 mg/kg, i.p.) significantly inhibited the development of morphine-induced behavioral sensitization during the induction period, while the effect of CBD (0.2 mg/kg, i.p.) attenuating morphine-induced behavioral sensitization was not obvious **(FIG. 2B).** After 7 consecutive days (days 1-7) of morphine administration for inducing behavioral sensitization and the following 1 week of withdrawal (days 8-14), mice were challenged with morphine at day 15. Morphine challenge significantly induced the expression of behavioral sensitization in morphine-pretreated mice **(FIG. 2C).** CIAC001 (0.2 mg/ kg, i.p.) but not CBD (0.2 mg/kg, i.p.) decreased the locomotor response to the morphine challenge **(FIG. 2C).** These results show that CIAC001 suppresses the development and expression of morphine-induced behavioral sensitization.

The conditioned place preference (CPP, **FIG. 8C)** is a standard preclinical behavioral model used to measure the rewarding effect of drugs, where animals tend to exhibit a preference (reflected by spending more time in) for the environment paired with the drug. Conditioning with morphine induced the acquisition of CPP in mice, with a significantly higher CPP score than that of the vehicle control **(FIG. 2D).** Administration of CIAC001 (0.2 mg/kg, i.p.) substantially decreased the expression of morphine-induced CPP **(FIG. 2D).** After the CPP behavioral testing on day 7, the experimental mice were sacrificed. Brain regions of the medial prefrontal cortex (mPFC), nucleus accumbens (NAc), and ventral tegmental area (VTA), which are widely implicated in reward and addiction, were dissected. As the most critical pro-inflammatory factor of neuroinflammation,[40,41] the expression of IL-1β was measured. Chronic morphine treatment increased the expression of IL-1β in mPFC **(FIG. 2E),** NAc **(FIG. 2F),** and VTA **(FIG. 2G)** regions, and the morphine-induced IL-1β overexpression in these regions was inhibited by CIAC001 **(FIG. 2E-G).** Moreover, the administration of **CICA001** decreased the expression of Iba1, which is a microglial activation marker, in the mPFC **(FIG. 2H)** but not in the NAc or VTA **(FIG. 9).** These findings indicated that CIAC001 inhibited morphine-induced neuroinflammation and prevented morphine-induced CPP.

Considering the excellent activity of CIAC001 in inhibiting morphine addiction, it was hypothesized that CIAC001 might have great blood-brain barrier (BBB) permeability. CIAC001 and CBD were given orally. The compound concentrations in plasma and the brain were measured. Following oral administration (10 mg/kg, formulation: 5% DMSO/10% Solutol/85% Captisol (20%), p.o.), the brain concentrations of CIAC001 and CBD peaked at 0.3 and 0.5 h, respectively.

TABLE 6 summarizes the pharmacokinetic parameters of CIAC001 and CBD in brain following oral administration at the dose of 10 mg/kg in BLAB/c mice. The brain Cmax of CIAC001 (175.0 ng/mg) was approximately 2 times that of CBD (98.1 ng/mg, **FIG. 10,** TABLE 5). In addition, the half-life ($t_{1/2}$) and mean residence time (MRT) of CIAC001 were 0.8 and 0.9 h, respectively, which were higher than the $t_{1/2}$ (0.5 h) and MRT (0.7 h) of CBD (TABLE 5).

**TABLE 6**

| Parameter | CIAC001 | CBD |
|---|---|---|
| $T_{max}$ (h) | 0.3 | 0.5 |
| $C_{max}$ (ng/g) | 175.0 | 98.1 |
| $t_{1/2}$ (h) | 0.8 | 0.5 |
| $AUC_{0\rightarrow t}$ (h·ng/g) | 172.8 | 66.7 |
| $AUC_{0\rightarrow \infty}$ (h·ng/g) | 191.3 | 85.0 |
| MRT (h) | 0.9 | 0.7 |

TABLE 7 summarizes brain-to-plasma (B/P) ratios of CIAC001 and CBD following oral administration as outlined above. The B/ P ratios of CIAC001 exceeded 200% within the first 3 h after administration, which was much higher than that of CBD (less than 100%, TABLE 6). The area under the curve (AUC) of CIAC001 in the brain (172.8 h ng/g) exceeded that of CBD (66.7 h ng/g) by more than twice. Oral administration of CIAC001 showed better BBB permeability and CNS pharmacokinetics when compared to CBD. The high BBB permeability and the CNS enrichment of CIAC001 may, at least

in part, account for its lower level in the plasma (FIG. 10).

**TABLE 7**

| Time (h) | CIAC001 B/P | CBD B/P |
|---|---|---|
| 0.083 | 207.3 ± 90.7% | 86.4 ± 22.7% |
| 0.25 | 277.8 ± 38.9% | 35.8 ± 10.6% |
| 0.5 | 330.7 ± 38.2% | 93.0 ± 17.6% |
| 1 | 363.0 ± 42.5% | 83.9 ± 14.4% |
| 2 | 412.0 ± 143.8% | n.d.[b] |
| 3 | 576.0 ± 133.7% | n.d.[b] |
| 4 | n.d.[b] | n.d.[b] |

[a]B/P concentration ratios were determined using the following equation: B/P = 100% x Brain concentration/Plasma concentration. [b]Not detected.

Together, these in vivo studies found that CIAC001 ameliorated the morphine-induced withdrawal reaction, behavioral sensitization, and CPP, which demonstrate the therapeutic effect of CIAC001 for morphine addiction.

## EXAMPLE 10

### CIAC001 is a Safe, Non-Addictive Synthetic Cannabinoid

This example covers the safety and rewarding effects of CIAC001. Results from the safety analyses of CIAC001 are shown in FIGS. S2D and 3A-B. Respiratory depression was found in mice upon single administration of THC (10 mg/kg, i.p., **FIG. 3A).** In contrast, CIAC001 (20 mg/kg, i.p.) did not affect the respiratory rate **(FIG. 3A).** Moreover, a Y maze test was performed to investigate the effect of THC or CIAC001 on spatial learning and memory in mice **(FIG. 8E).** Compared to the vehicle control, mice treated with THC (10 mg/kg, i.p.) spent significantly less time on the new arm **(FIG. 3B).** In contrast, CIAC001 (20 mg/kg, i.p.) treatment did not affect the time mice spent on the new arm **(FIG. 3B),** indicating no apparent potential for memory decline or cognitive impairment.

To directly investigate the behaviorally rewarding effect of CIAC001, a CPP assay was performed **(FIG. 8F).** As shown in **FIG. 3C,** mice treated with CIAC001 (20 mg/kg, i.p.) did not show any effect on CPP after 6 consecutive days of training, which implies that CIAC001 lacks a rewarding effect. An open-field assay was performed after CIAC001 (20 mg/kg, i.p.) administration for seven consecutive days **(FIG. 8D).** No apparent differences in the motor activity **(FIG. 3D)** as well as the grooming and rubbing behaviors **(FIG. 3E)** were observed for mice before the administration of CIAC001 and the mice treated with CIAC001 (20 mg/kg, i.p.), which indicated that CIAC001 did not cause stress to mice. Consistent with these observations, the body weights of CIAC001-treated mice did not change during the drug treatment compared to the vehicle control **(FIG. 3F).** It should be noted that the dose of CIAC001 (20 mg/kg) used for in vivo safety evaluation experiments was 100 times the maximal dose (0.2 mg/kg CIAC001) used for preventing morphine addiction. Hence, CIAC001 is a safe, non-addictive synthetic cannabinoid compared to THC.

## EXAMPLE 11

### Target Fishing and the Verification of PKM2 as a Direct Target for CIAC001

In order to fish out the molecular target responsible for CIAC001 inhibiting neuro-inflammation, the photoaffinity probe Compound 19e was synthesized. The workflow chart for protein target identification is shown in **FIG. 4A.** The photoaffinity probe Compound 19e bear a photoreactive diazirine group and a terminal alkynyl group on the same molecule. Upon UV irradiation, the photoreactive group was activated, generating extremely reactive chemical species which formed a new covalent bond between the photoreactive group and the macromolecular binding partners of Compound 19e. BV-2 cells were incubated with Compound 19e (5 M) and different concentrations of CIAC001 (0, 20 M) for 2 h, and then, the live cells were irradiated at 365 nm by UV light for 30 min. Subsequently, the cells were lysed, and a copper-catalyzed alkyne-azide cycloaddition "click" reaction was utilized for the conjugation to the biotin. The resulting mixtures were subjected to streptavidin pull-down. The samples were separated by SDS-PAGE and visualized by silver staining **(FIG. 4B).** The bands enriched by Compound 19e were competitively inhibited by CIAC001 (20 M). In order to limit

the impact of modifications on CIAC001, the non-covalent small-molecule probe Compound 19f without the photoreactive crosslinking moiety was also used for target identification. Normalized cell homogenates were incubated with Compound 19f. The labeling was done through a "click" reaction between the ethynyl group of Compound 19f and Biotin-$PEG_3$-$N_3$, followed by streptavidin beads-based affinity enrichment **(FIG. 4A).** Silver staining of Compound 19f captured proteins in polyacrylamide gel showed similar patterns as that of Compound 19e. The bands captured by Compound 19e or Compound 19f were cut for proteolysis into peptides, followed by proteomic analysis by LC-MS/MS. The proteins that bind to CIAC001 are shown in FIG. 4C. The possible target protein at the intersection of Compound 19e-based in situ capturing and Compound 19f based in vitro pull-down was selected for subsequent protein target verification.

PKM (pyruvate kinase M112, approx. 58 kDa for the monomer), which is one of the representative binding partners of CIAC001 probes **(FIG. 4C),** has been recently recognized as a critical determinant of promoting the pro-inflammatory response. Western blotting using the corresponding antibodies was used to first verify whether PKM directly binds with CIAC001. As shown in **FIG. 5A,** CIAC001 directly binds with PKM2 rather than PKM1 in situ and in vitro. Indeed, the expression level of PKM2 was much higher than that of PKM1 in the immunocompetent microglial BV2 cells. Moreover, the binding of Compound 19e to PKM2 could be competitively inhibited by high concentrations of free CIAC001 **(FIG. 5A).** Furthermore, a cellular thermal shift assay (CETSA) was performed to confirm whether PKM2 was the binding target of CIAC001. Briefly, the CETSA assay involves the incubation of cell lysates with or without CIAC001, heating to denature and precipitate proteins, as well as the removal of cell debris and aggregates from the soluble protein fraction. The remaining soluble protein was detected through quantitative western blotting. CIAC001 binding increased the thermal stabilization of PKM2, as reflected by the shift of its melting temperature ($T_m$) with $\Delta T_m$ of $3.9 \pm 1.3$ °C **(FIG. 5B).** In contrast, Compound 19b failed to affect the $T_m$ of PKM2, which demonstrates the specificity of the interaction of CIAC001 with PKM2. In order to quantitively characterize the interaction of CIAC001 with PKM2, fluorescence titration of the purified PKM2 by CIAC001 was performed **(FIG. 5C).** CIAC001 binding caused the quenching of PKM2 intrinsic fluorescence. A stoichiometry of $0.54 \pm 0.07$ and a dissociation constant KD of $2.2 \pm 0.2$ μM were derived for the CIAC001-PKM2 interaction **(FIG. 5C).** In silico docking was performed to identify the binding site of CIAC001 with PKM2, which adopts a tetrameric quaternary structure to be active for the pyruvate kinase activity. In agreement with the measured stoichiometry of $0.54 \pm 0.07$, CIAC001 was found to bind at the interaction interface between two PKM2 monomers **(FIG. 5D)** and promote tight protein binding through hydrophobic interactions with the surrounding residues **(FIG. 5E).** Further molecular dynamics simulations showed that the binding of CIAC001 increased the number of hydrogen bonds between the dimers in the tetrameric form **(FIG. 11).** However, Compound 19b bound to the monomer PKM2, far away from the dimerization interface **(FIGS. 5D, F).**

Tetrameric PKM2 promotes ATP production via oxidative phosphorylation. Consistently, CIAC001 was found to increase the ATP level, but Compound 19b showed no effect **(FIG. 6A),** which supports CIAC001 activating PKM2. Together, all these results indicate that the PKM2 protein is a direct target of CIAC001. PKM2 exists in a dynamic population of different oligomeric states. LPS treatment decreased the PKM2 tetramer but increased the PKM2 monomer in cells **(FIG. 6B).** CIAC001 was found to reverse the LPS-induced decline of the PKM2 tetramer and inhibit the LPS-induced rise of the PKM2 monomer **(FIG. 6B).** Monomeric PKM2 translocates into the nucleus, where it functions as a transcriptional coactivator and boosts the inflammatory response.[45] As expected, LPS treatment significantly increased the content of PKM2 in the nucleus **(FIG. 6C).** CIAC001 completely reversed the LPS-induced translocation of nuclear PKM2 (FIG. **6C).** Monomeric PKM2 has been reported to function as a glycolysis promotor and promote the Warburg effect.[46] Indeed, exposure of microglial BV-2 cells to LPS increased lactate dehydrogenase A (LDHA) mRNA **(FIG. 12)** and lactate in the culture **(FIG. 6D).** CIAC001 treatment significantly inhibited LPS-induced LDHA mRNA overexpression **(FIG. 12)** as well as lactate release **(FIG. 6D),** while Compound 19b did not block LPS-induced lactate increase **(FIG. 6D),** which indicates that CIAC001 can reverse PKM2-mediated metabolic reprogramming.

Hif-1$\alpha$ is directly regulated by PKM2 and controls the expression of IL-1$\beta$.[47] Chronic morphine treatment increased the expression of Hif-1$\alpha$ in mPFC **(FIG. 6E).** Administration of CIAC001 was found to inhibit chronic morphine-induced Hif-1$\alpha$ overexpression **(FIG. 6E).** The above results imply that CIAC001 had a role in preventing morphine-induced addiction is at least mediated by the PKM2-Hif-1$\alpha$-IL-1$\beta$ signaling axis.

Cannabinoid receptors (CB1 and CB2) have been reported as the main targets for most cannabinoids.[48] To test the possible interaction of CIAC001 with CB1/CB2, a cAMP assay for Gi-coupled GPCR signaling was performed. In contrast to CBD, which has slight CB1/CB2 receptor activity, CIAC001 had no apparent CB1/CB2 activity (**FIG. 13**, TABLE 8), which clearly rules out the possibility of CB1/CB2 as the target for CIAC001. All together, these results demonstrate that PKM2 is a specific target for CIAC001.

TABLE 8

| Ligands | CB1 Receptor | | CB2 Receptor | |
|---|---|---|---|---|
| | $EC_{50}$ (μM) | $\Delta E_{max}$ (%) | $EC_{50}$ (μM) | $\Delta E_{max}$ (%) |
| WIN 55,212-2 Mesylate | 3.9 ± 0.3 | 79.0 ± 8.1 | 0.15 ± 0.1 | 48.7 ± 4.6 |
| CBD | 1.5 ± 0.2 | 13.3 ± 0.1 | $3.5x10^{-3}$ ± 1.1 | 30.2 ± 0.9 |
| CIAC001 | n.d.[a] | n.d.[a] | n.d.[a] | n.d.[a] |
| [a]: Not detected | | | | |

In order to identify the molecular target responsible for CIAC001 inhibiting neuroinflammation, target fishing led to the identification of PKM2 as the target protein. CIAC001 is bound at the interaction interface between two PKM2 monomers and the stabilized PKM2 tetramer. The transition between PKM2 monomers and tetramers is tightly regulated.[44,53] CIAC001 reversed the LPS-induced decline of the PKM2 tetramer and the inhibited LPS-induced rise of the PKM2 monomer. Monomeric PKM2 translocates into the nucleus and mediates inflammation by affecting inflammation-related gene expression. CIAC001 inhibited LPS-induced translocation of nuclear PKM2 and blocked LPS-induced inflammatory factors and the Warburg effect. This study provides evidence that PKM2 is a target of CIAC001. However, it should be noted that no studies have been conducted on the broader implications of PKM2 regarding the effects of phytocannabinoids on neuro-inflammation. Whether PKM2 would be a broad antiinflammatory target of cannabinoids deserves further exploration.

## EXAMPLE 12

### Synthesis of Compounds 19e-19f

(1'R,2'R)-4-(1-(2H-1,2,3-triazol-2-yl)ethyl)-5'-methyl-2'-(prop-1-en-2-yl)-6-(2-(3-(prop-2-yn-1-yl)-3H-diazirin-3-yl)ethoxy)-1',2',3',4'-tetrahydro-[1,1'-biphenyl]-2-ol (Compound 19e). CIAC001 (0.10 g, 0.3 mmol), Ph3P (0.09 g, 0.36 mmol, 1.2 equiv.) and 2-(3-(but-3-yn-1-yl)-3H-diazirin-3-yl)ethan-1-ol (0.04 g, 0.36 mmol, 1.2 equiv.) were dissolved in 10 mL dry THF. Diisopropyl azodicarbo (0.20 g, 0.36 mmol) was dissolved in 2 mL THF and dropped into the reaction mixture. The reaction mixture was stirred at room temperature overnight. The reaction was diluted with water, extracted with ether and washed with brine. The crude product was purified by flash chromatography eluting with petroleum ether/EtOAc. Yield Compound 19e: 34.19 mg, 25.6%, 1H NMR (300 MHz, CDCl3) δ = 7.57 (s, 2H), 6.81 (d, J = 2.3, 2H), 5.79 (q, J = 7.1, 1H), 5.12 (s, 1H), 4.48 (s, 1H), 4.39 (s, 1H), 3.48 (d, J = 10.3, 1H), 2.68 -2.54 (m, 1H), 2.15 (s, 9H), 1.91 (d, J = 7.1, 3H), 1.85 -1.58 (m, 6H), 1.53 (s, 3H); 13C NMR (75 MHz, CDCl3) δ = 168.7, 149.9, 147.3, 139.6, 134.1, 133.2, 128.8, 128.8, 123.9, 111.3, 63.2, .45.4, 38.4, 30.3, 28.6, 23.4, 20.9, 20.8, 19.4. MS (ESI-) calcd for C27H33N5O2-H+: 458.26[M -H]-; found: 458.22.

(1'R,2'R)-4-(1-(2H-1,2,3-triazol-2-yl)ethyl)-5'-methyl-2'-(prop-1-en-2-yl)-6-(prop-2-yn-1-yloxy)-1',2',3',4'-tetrahydro-[1,1'-biphenyl]-2-ol (Compound 19f). CIAC001 (168.5 mg, 0.5 mmol) and 3-bromopropyne (0.6 mmol, 1.2 equiv.) were dissolved in 15 mL acetone, and then K2CO3 (71.38 mg, 0.6 mmol, 1.2 equiv.) was added to the mixture with stirring at room temperature. After 4 h, the reaction mixture was diluted with water and extracted with EtOAc, and the organic phase was washed with brine. The crude product was purified by flash chromatography eluting with petroleum ether/EtOAc. Yield Compound 19f: 51.87 mg, 27.5%, 1H NMR (300 MHz, CDCl3) δ = 7.64 (d, J = 3.0 Hz, 2H), 6.42 (d, J = 13.5 Hz, 1H), 6.23 (d, J = 6.6 Hz, 1H), 6.14 (s, 1H), 5.75 (q, J = 7.0 Hz, 1H), 5.53 (s, 1H), 4.50 (s, 1H), 4.35 (s, 1H), 4.02 (s, 1H), 3.80 -3.61 (m, 2H), 2.41 (s, 1H), 2.20 (m, 2H), 2.07 -1.99 (m, 5H), 1.96 (m, 3H), 1.85 - 1.75 (m, 6H), 1.63 (s, 3H), 1.27 (t, J = 5.2 Hz, 3H); 13C NMR (75 MHz, CDCl3) δ = 149.0, 134.0, 123.9, 117.6, 111.2, 100.0, 82.6, 69.3, 64.1, 62.9, 32.9, 32.3, 30.1, 26.5, 13.3. MS (ESI-) calcd for C23H27N3O2-H+: 376.21 [M -H]-; found: 376.09.

## EXAMPLE 13

### CIAC001 Improves Learning, Memory, and Anxiety

This example covers the effect of CIAC001 on learning, memory, and anxiety. First, wild-type and transgene (Alzheimer's disease model) mice were administered vehicle, cannabidiol, or CIAC001 and were subjected to Morris water maze and Y-maze tests. The results of these tests are shown in **FIGS. 14A-F** (Morris water maze), **FIGS. 14G-I** (Open field experiments), **FIGS. 14J** (New object recognition experiments) and **FIGS. 14K-L** (Y-maze). These results show that CIAC001 improves learning and memory abilities of Alzheimer's disease mice at a lower dose than cannabidiol.

The wild-type and transgene mice were also subjected to high plus maze and open field assays to assess the effect of CIAC001 on Alzheimer's disease-induced anxiety. The results of these analyses are summarized in **FIGS. 15M-N** (high plus maze assay). In these assays, CIAC001 restored arm entry activity and grid traversal, showing that CIAC001 suppresses Alzheimer's disease induced anxiety.

## EXAMPLE 14

### Mechanism of CIAC001 Alzheimer's Disease Treatment

To probe the mechanism of CIAC001 action in Alzheimer's disease treatment, the transgene mice were subjected to *in vitro* potential of hippocampus measurements. The results of these analyses (summarized in **FIGS. 15A-B)** demonstrated that CIAC001 increased hippocampal potential by almost 500%.

Western blot analyses were also performed on multiple Alzheimer's disease markers in transgene mice administered 0, 0.2, 2, or 20 mg/kg CIAC001 **(FIGS. 15C-D).** These analyses showed that CIAC001 inhibits Tau and TrkB phosphorylation, and reduces Syn1 and Aβ levels.

Although the invention has been described with reference to the presently preferred embodiment, it should be understood that various modifications can be made without departing from the spirit of the invention. Accordingly, the invention is limited only by the following claims.

## Claims

1. A method of treating or preventing opioid addiction, withdrawal, or overdose in a subject in need thereof comprising administering a compound of Formula I:

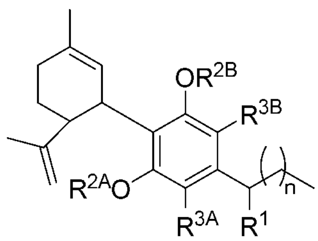

(I)

or a salt or solvate thereof to the subject, wherein:
$R^1$ is selected from -H,

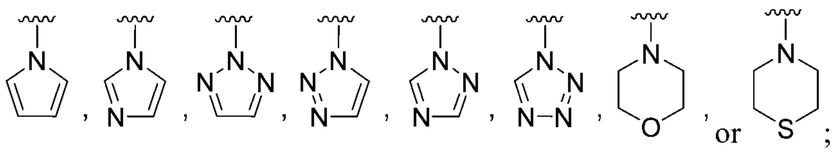

, , , , , , , or ;

$R^{2A}$ and $R^{2B}$ are each independently selected from -H, $C_1$-$C_4$ alkyl, $C_1$-$C_4$ alkenyl, or $C_1$-$C_4$, wherein $R^{2A}$ and $R^{2B}$ are each optionally substituted by diazirine;
$R^{3A}$ and $R^{3B}$ are each independently selected from -H, halogen, -COOH, or $-NH_2$; and subscript n is 0 to 12; thereby treating or preventing the opioid addiction, withdrawal, or overdose in the subject.

2. The method of claim 1, wherein the compound of Formula I is a compound of Formula Ia:

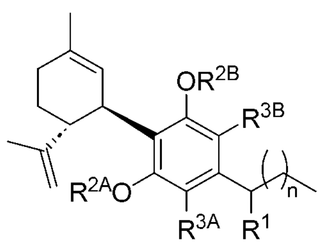

(Ia).

**3.** The method of claim 1, wherein $R^1$ is selected from H,

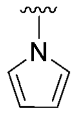, 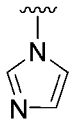, 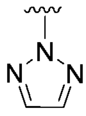 or 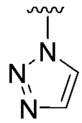.

**4.** The method of claim 3, wherein $R^1$ is

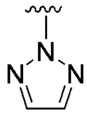.

**5.** The method of claim 1, wherein $R^{2A}$ and $R^{2B}$ are each independently selected from -H or $C_1$-$C_4$ alkyl.

**6.** The method of claim 5, wherein $R^{2A}$ and $R^{2B}$ are each independently selected from -H or $-CH_3$.

**7.** The method of claim 6, wherein $R^{2A}$ and $R^{2B}$ are each -H.

**8.** The method of claim 1, wherein $R^{3A}$ and $R^{3B}$ are each independently selected from -H, - Cl,-Br, or -COOH.

**9.** The method of claim 8, wherein $R^{3A}$ and $R^{3B}$ are each -H.

**10.** The method of claim 1, wherein n is 0 or 1.

**11.** The method of claim 1, wherein the compound of Formula I is

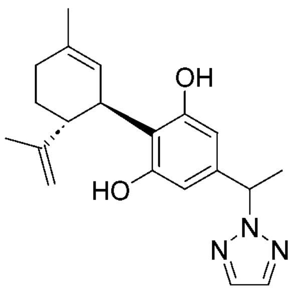.

**12.** The method of claim 1, wherein the method comprises treating or preventing the opioid addiction.

**13.** The method of claim 1, wherein the method comprises treating or preventing the opioid withdrawal.

**14.** The method of claim 1, wherein the compound of Formula I is administered at a dose of about 0.5 μg/kg, about 2 μg/kg, about 20 μg/kg, about 200 μg/kg, about 2 mg/kg, about 20 mg/kg, or about 100 mg/kg.

**15.** The method of claim 1, wherein a $C_{MAX}$ of the compound of Formula I in a brain of the subject is about 1-1000 ng/mg.

**16.** The method of claim 1, wherein a brain-to-plasma ratio of the compound of Formula I is at least about 10:1, at least about 25:1, at least about 50:1, at least about 100:1, or at least about 200:1 one hour after the administration.

**17.** The method of claim 1, wherein the opioid is fentanyl, buprenorphine, codeine, dextromoramide, dihydrocodeine, enkephalin, heroin, hydrocodone, hydromorphone, meperidine, methadone, morphine, nicomorphine, opium, oxycodone, oxymorphone, pentazinine, pentazocine, a derivative thereof, a precursor thereof, or pharmacologically acceptable salt or solvate thereof.

**18.** The method of claim 17, wherein the opioid is morphine.

**19.** The method of claim 1, wherein the subject exhibits a symptom of opioid addiction selected from withdrawal reactions, behavioral sensitization, or conditioned place preference.

**20.** The method of claim 1, wherein the compound of Formula I is administered orally, buccally, sublingually, rectally, vaginally, intravenously, intra-arterially, intraosseously, intramuscularly, intracerebrally, intracerebroventricularly, intrathecally, subcutaneously, intraperitoneally, intraocularly, intranasally, transdermally, epidurally, intracranially, percutaneously, intrauterineally, intravitreally, transmucosally, or via an inhaler.

**21.** The method of claim 1, wherein the compound of Formula I is administered with a pharmaceutically acceptable excipient.

**22.** The method of claim 1, further comprising administering an opioid antagonist to the subject.

**23.** The method of claim 22, wherein the opioid antagonist is naloxone or naltrexone.

**24.** The method of claim 1, wherein the subject is addicted to the opioid.

**25.** The method of claim 1, wherein the compound of Formula I is administered at a dose sufficient to inhibit opioid induced overexpression of IL-1β, TNFα, IL-6, or a combination thereof in the subject.

**26.** The method of claim 1, wherein the compound of Formula I is administered at a dose sufficient to modulate PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, or elongation factor 1-gamma in the subject.

**27.** The method of claim 26, wherein the compound of Formula I is administered at a dose sufficient to modulate PKM in the subject.

**28.** The method of claim 27, wherein the PKM is PKM2.

**29.** The method of claim 1, wherein the compound of Formula I is administered at a dose sufficient to increase a ratio of M2 to M1 microglia in a brain of the subject.

**30.** The method of claim 1, wherein the compound of Formula I does not cause a toxic stress response in the subject.

**31.** A method of modulating PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, elongation factor 1-gamma in a subject in need thereof comprising administering a compound of Formula I:

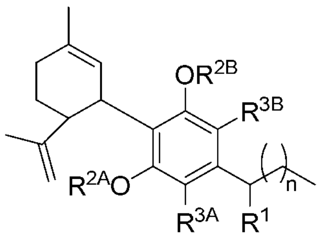

(I)

or a salt or solvate thereof to the subject, wherein:

$R^1$ is selected from -H,

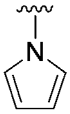 , 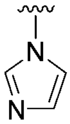 , 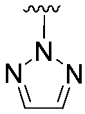 , 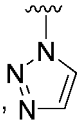 , 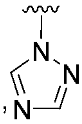 , 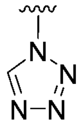 , 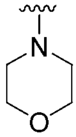 , or 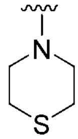 ;

$R^{2A}$ and $R^{2B}$ are each independently selected from-H, $C_1$-$C_4$ alkyl, $C_1$-$C_4$ alkenyl, or $C_1$-$C_4$, wherein $R^{2A}$ and $R^{2B}$ are each optionally substituted by diazirine;
$R^{3A}$ and $R^{3B}$ are each independently selected from -H, halogen, -COOH, or -$NH_2$; and subscript n is 0 to 12; thereby modulating PKM, 60 kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, elongation factor 1-gamma in the subject.

**32.** The method of claim 31, wherein the method comprises modulating PKM in the subject.

**33.** The method of claim 32, wherein the PKM is PKM2.

**34.** A method of increasing a ratio of M2 to M1 microglia in a brain of a subject comprising administering a compound of Formula I to the subject:

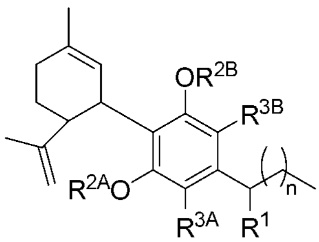 (I)

or a salt or solvate thereof to the subject, wherein:

$R^1$ is selected from -H,

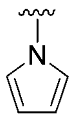 , 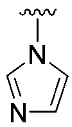 , 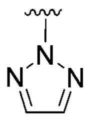 , 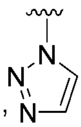 , 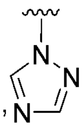 , 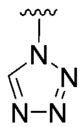 , 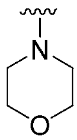 , or 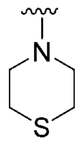 ;

$R^{2A}$ and $R^{2B}$ are each independently selected from-H, $C_1$-$C_4$ alkyl, $C_1$-$C_4$ alkenyl, or $C_1$-$C_4$, wherein $R^{2A}$ and $R^{2B}$ are each optionally substituted by diazirine;
$R^{3A}$ and $R^{3B}$ are each independently selected from -H, halogen, -COOH, or -$NH_2$; and subscript n is 0 to 12; thereby increasing M2 to M1 microglia ratios in the brain of the subject.

**35.** A method of treating a neurodegenerative disease or disorder in a subject in need thereof comprising administering a compound of Formula I:

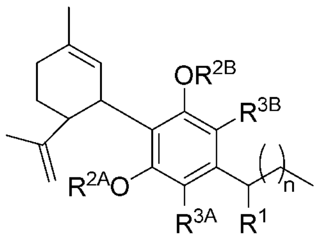 (I)

or a salt or solvate thereof to the subject, wherein:

$R^1$ is selected from -H,

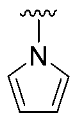, 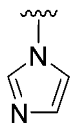, 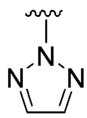, 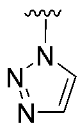, 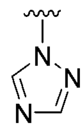, 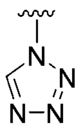, 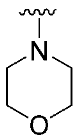, or 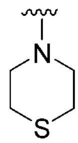;

$R^{2A}$ and $R^{2B}$ are each independently selected from -H, $C_1$-$C_4$ alkyl, $C_1$-$C_4$ alkenyl, or $C_1$-$C_4$, wherein $R^{2A}$ and $R^{2B}$ are each optionally substituted by diazirine;
$R^{3A}$ and $R^{3B}$ are each independently selected from -H, halogen, -COOH, or $-NH_2$; and subscript n is 0 to 12; thereby treating the disease or disorder in the subject.

**36.** The method of claim 35, wherein the compound of Formula I is a compound of Formula Ia:

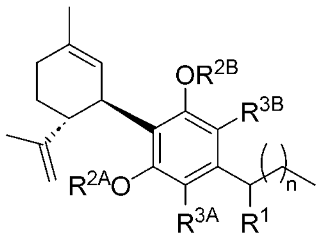
(Ia).

**37.** The method of claim 35, wherein $R^1$ is selected from H,

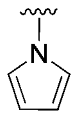, 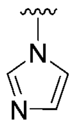, 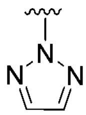 or 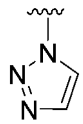.

**38.** The method of claim 37, wherein $R^1$ is

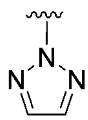.

**39.** The method of claim 35, wherein $R^{2A}$ and $R^{2B}$ are each independently selected from -H or $C_1$-$C_4$ alkyl.

**40.** The method of claim 39, wherein $R^{2A}$ and $R^{2B}$ are each independently selected from -H or $-CH_3$.

**41.** The method of claim 40, wherein $R^{2A}$ and $R^{2B}$ are each -H.

**42.** The method of claim 35, wherein $R^{3A}$ and $R^{3B}$ are each independently selected from -H, - Cl,-Br, or -COOH.

**43.** The method of claim 42, wherein $R^{3A}$ and $R^{3B}$ are each -H.

**44.** The method of claim 35, wherein n is 0 or 1.

**45.** The method of claim 35, wherein the compound of Formula I is

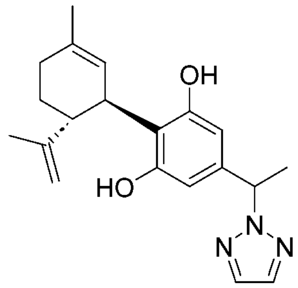

.

**46.** The method of claim 35, wherein the disease or disorder is selected from Alzheimer's disease, Parkinson's disease, or amyotrophic lateral sclerosis.

**47.** The method of claim 35, wherein the compound of Formula I inhibits neuroinflammation in the subject.

**48.** The method of claim 35, wherein the compound of Formula I comprises an $IC_{50}$ of at most about 200 μM, at most about 150 μM, at most about 100 μM, at most about 75 μM, at most about 50 μM, at most about 40 μM, at most about 30 μM, at most about 20 μM, at most about 10 μM, at most about 5 μM, at most about 2.5 μM, or at most about 1 μM for inhibition of LPS-induced nitric oxide overproduction in microglial cells.

**49.** The method of claim 35, wherein the compound of Formula I comprises a cellular toxicity $IC_{50}$ of at least about 1 μM, at least about 2.5 μM, at least about 5 μM, at least about 10 μM, at least about 20 μM, at least about 30 μM, at least about 40 μM, at least about 50 μM, at least about 75 μM, at least about 100 μM, at least about 150 μM, at least about 200 μM in microglial cells.

**50.** The method of claim 35, wherein the compound of Formula I is administered at a dose sufficient to diminish expression of IL-1β in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold.

**51.** The method of claim 35, wherein the compound of Formula I is administered at a dose sufficient to diminish expression of TNFα in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold.

**52.** The method of claim 35, wherein the compound of Formula I is administered at a dose sufficient to diminish expression of IL-6 in microglial cells of the subject by at least about 1.5-fold, at least about 2-fold, at least about 2.5-fold, at least about 3-fold, at least about 4-fold, at least about 5-fold, at least about 6-fold, at least about 8-fold, at least about 10-fold, at least about 15-fold, or at least about 20-fold.

**53.** The method of claim 35, wherein the compound of Formula I is administered at a dose of about 0.5 μg/kg, about 2 μg/kg, about 20 μg/kg, about 200 μg/kg, about 2 mg/kg, about 20 mg/kg, or about 100 mg/kg.

**54.** The method of claim 35, wherein a $C_{MAX}$ of the compound of Formula I in a brain of the subject is about 1-1000 ng/mg.

**55.** The method of claim 35, wherein a brain-to-plasma ratio of the compound of Formula I is at least about 10:1, at least about 25:1, at least about 50:1, at least about 100:1, or at least about 200:1 one hour after the administration.

**56.** The method of claim 35, wherein the compound of Formula I is administered orally, buccally, sublingually, rectally, vaginally, intravenously, intra-arterially, intraosseously, intramuscularly, intracerebrally, intracerebroventricularly, intrathecally, subcutaneously, intraperitoneally, intraocularly, intranasally, transdermally, epidurally, intracranially, percutaneously, intrauterine ally, intravitreally, transmucosally, or via an inhaler.

**57.** The method of claim 35, wherein the compound of Formula I is administered with a pharmaceutically acceptable excipient.

**58.** The method of claim 35, wherein the compound of Formula I is administered at a dose sufficient to modulate PKM, 60

kDa heat shock protein, alpha-enolase, elongation factor 1-1 alpha, RNA helicase, heat shock protein 90-beta, ubiquitin carboxyl-terminal hydrolase, T-complex protein 1 subunit, alpha, phosphoglycerate kinase 1, elongation factor Tu serine, elongation factor Tu cysteine, peptidase inhibitor, clade B member 6a, or elongation factor 1-gamma in the subject.

**59.** The method of claim 58, wherein the compound of Formula I is administered at a dose sufficient to modulate PKM in the subject.

**60.** The method of claim 59, wherein the PKM is PKM2.

**61.** The method of claim 35, wherein the compound of Formula I is administered at a dose sufficient to increase a ratio of M2 to M1 microglia in a brain of the subject.

**62.** The method of claim 35, wherein the compound of Formula I does not cause a toxic stress response in the subject.

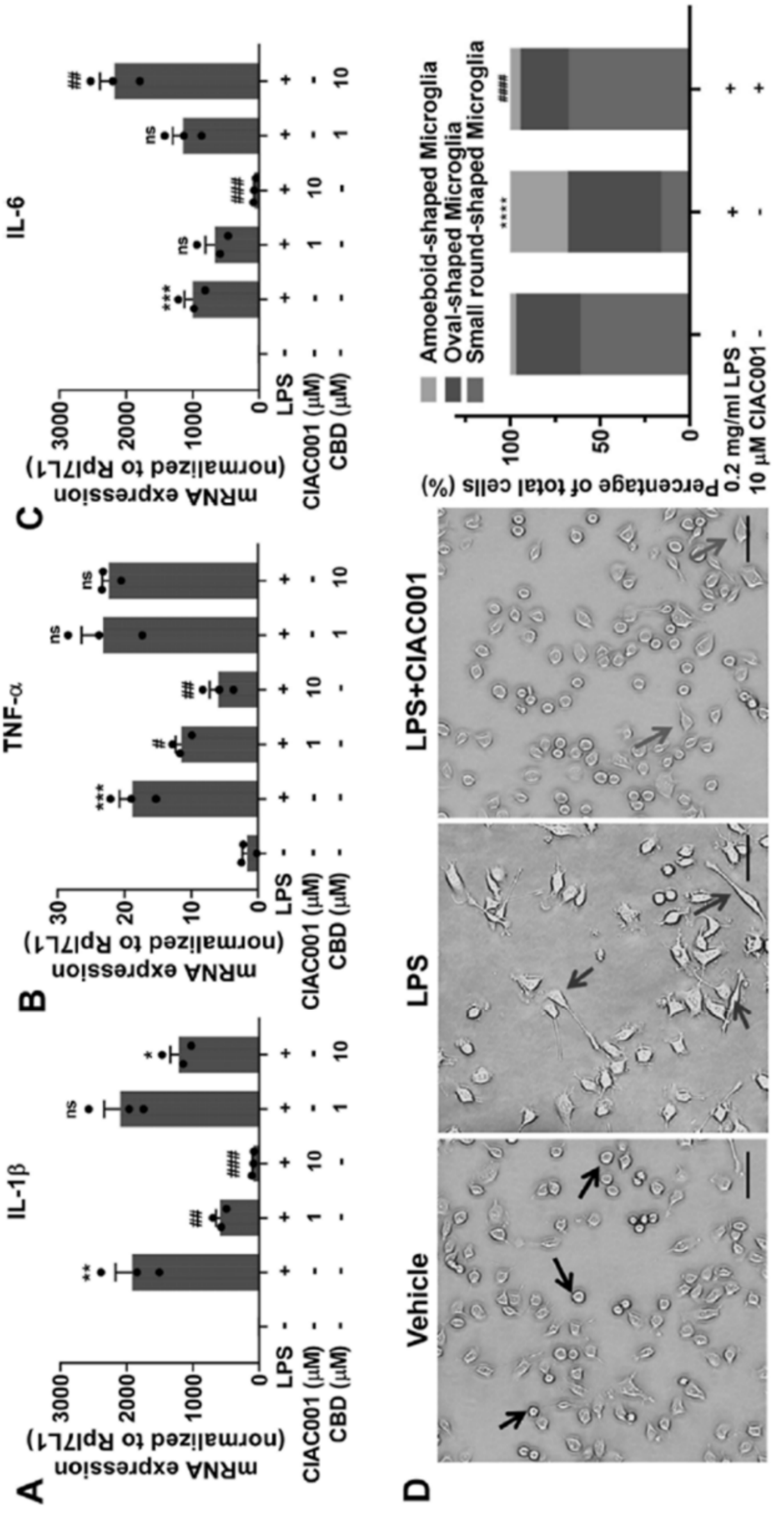
A
IL-1β
mRNA expression (normalized to Rpl7L1)
3000
2000
1000
0
LPS - + + + + +
CIAC001 (μM) - - 1 10 - -
CBD (μM) - - - - 1 10
** ## ### ns *
B
TNF-α
mRNA expression (normalized to Rpl7L1)
30
20
10
0
LPS - + + + + +
CIAC001 (μM) - - 1 10 - -
CBD (μM) - - - - 1 10
*** # ## ns ns
C
IL-6
mRNA expression (normalized to Rpl7L1)
3000
2000
1000
0
LPS - + + + + +
CIAC001 (μM) - - 1 10 - -
CBD (μM) - - - - 1 10
*** ns ### ns ##
D
Vehicle
LPS
LPS+CIAC001
Amoeboid-shaped Microglia
Oval-shaped Microglia
Small round-shaped Microglia
Percentage of total cells (%)
100
50
0
0.2 mg/ml LPS - + +
10 μM CIAC001 - - +
**** ####

FIG. 1

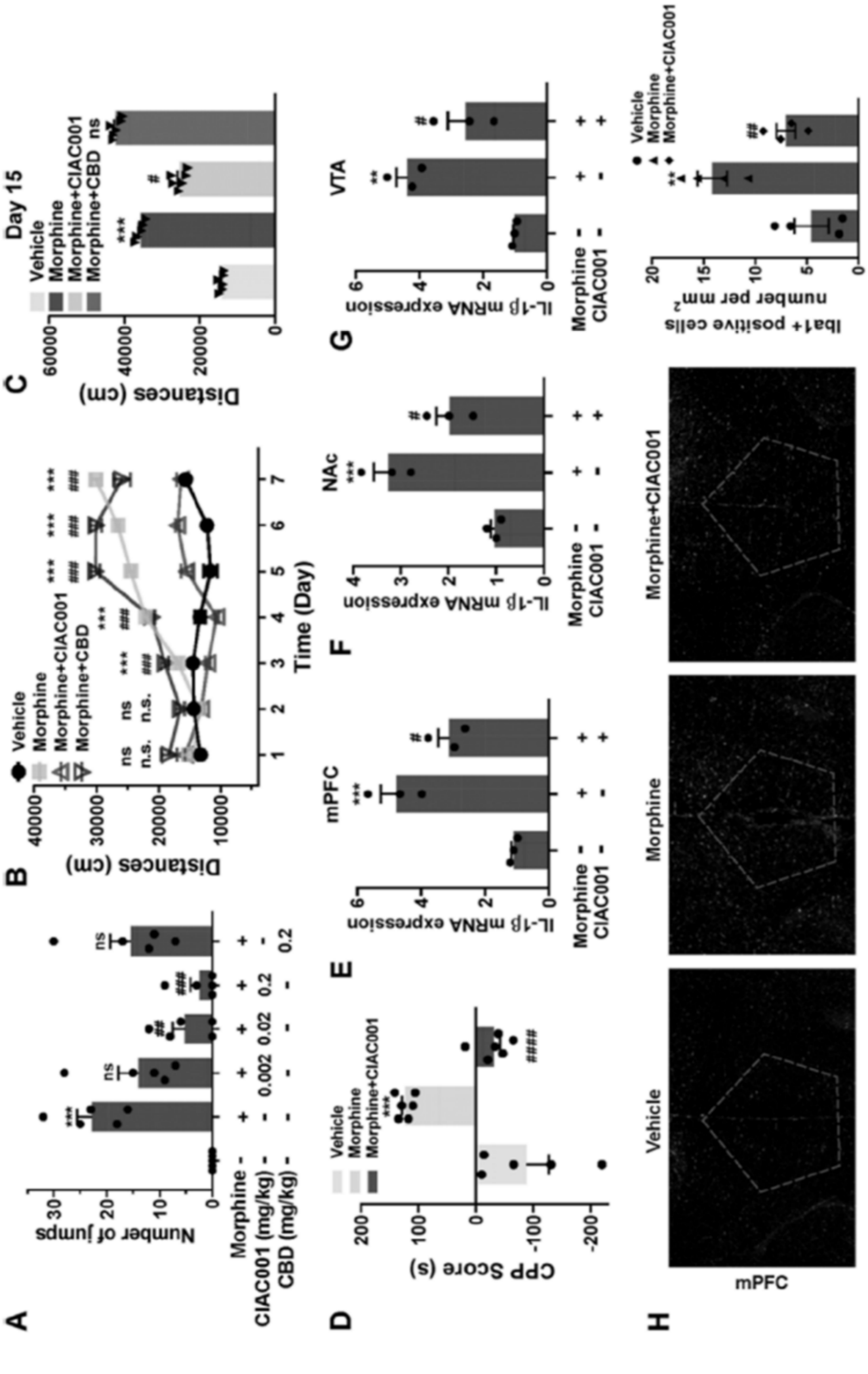
A
Number of jumps
Morphine
CIAC001 (mg/kg)
CBD (mg/kg)
B
Vehicle
Morphine
Morphine+CIAC001
Morphine+CBD
Distances (cm)
Time (Day)
C
Day 15
Distances (cm)
D
CPP Score (s)
E
mPFC
F
NAc
G
VTA
IL-1β mRNA expression
Iba1+ positive cells number per mm²
H
Vehicle
Morphine
Morphine+CIAC001
mPFC

FIG. 2

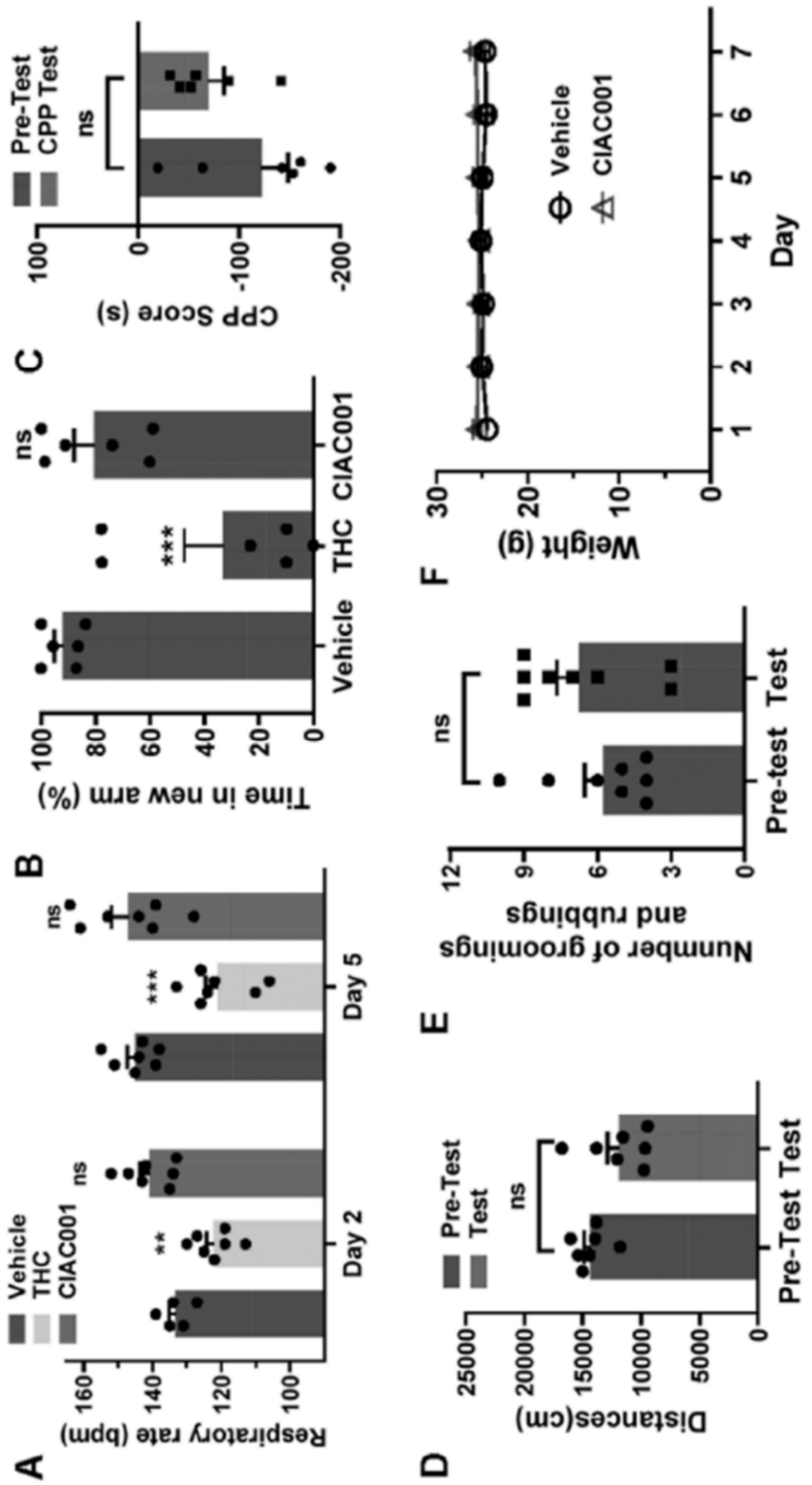
A
Respiratory rate (bpm)
160
140
120
100
Vehicle
THC
CIAC001
**
ns
***
ns
Day 2
Day 5
B
Time in new arm (%)
100
80
60
40
20
0
***
ns
Vehicle
THC
CIAC001
C
CPP Score (s)
100
0
-100
-200
Pre-Test
CPP Test
ns
D
Distances(cm)
25000
20000
15000
10000
5000
0
Pre-Test
Test
ns
Pre-Test
Test
E
Nunmber of groomings
and rubbings
12
9
6
3
0
ns
Pre-test
Test
F
Weight (g)
30
20
10
0
Vehicle
CIAC001
1
2
3
4
5
6
7
Day

FIG. 3

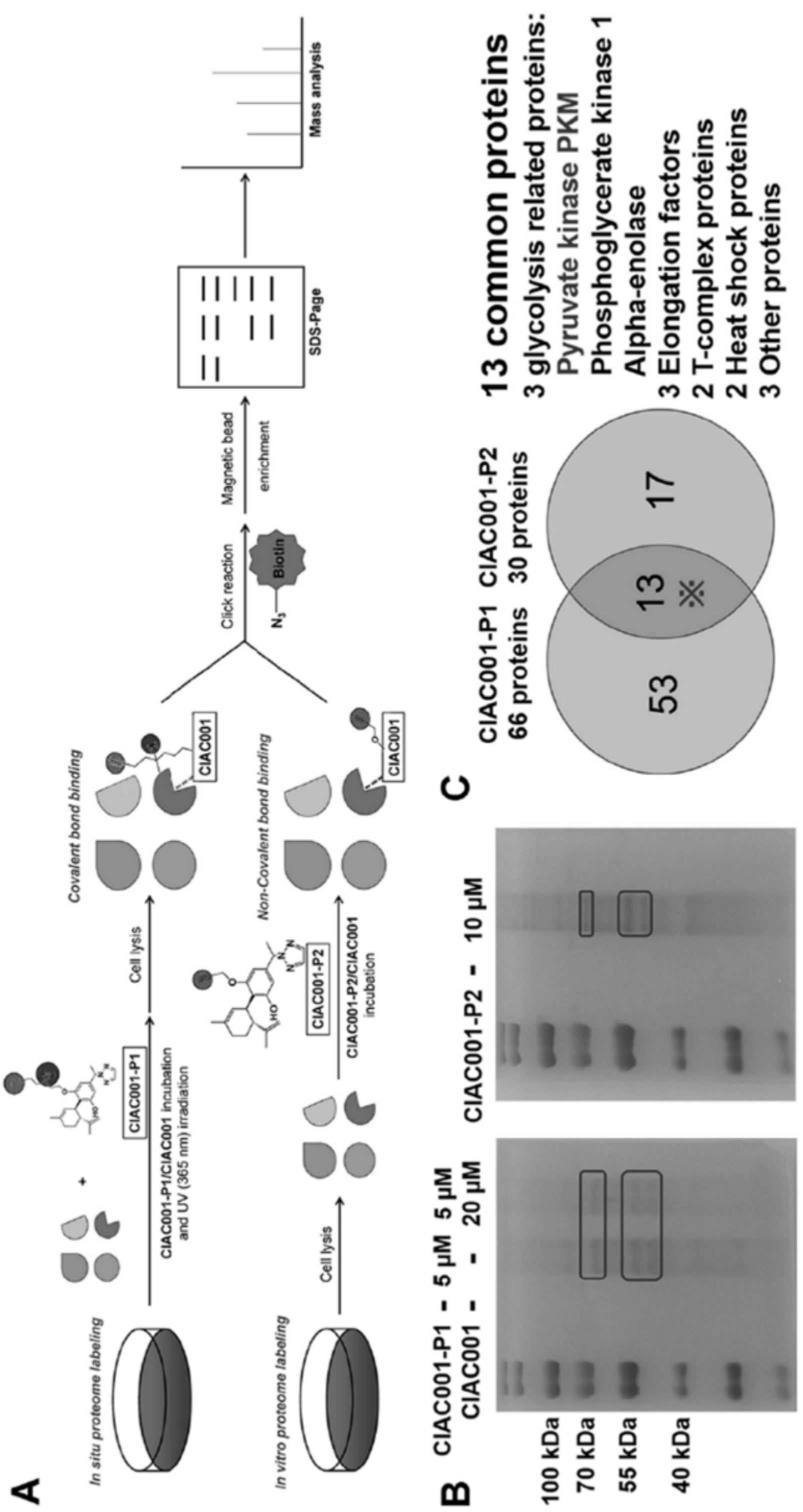
A
In situ proteome labeling
CIAC001-P1
CIAC001-P1/CIAC001 incubation and UV (365 nm) irradiation
Cell lysis
Covalent bond binding
CIAC001
In vitro proteome labeling
Cell lysis
CIAC001-P2
CIAC001-P2/CIAC001 incubation
Non-Covalent bond binding
CIAC001
Click reaction
Biotin
Magnetic bead enrichment
SDS-Page
Mass analysis
B
CIAC001-P1 - 5 μM 5 μM
CIAC001 - - 20 μM
CIAC001-P2 - 10 μM
100 kDa
70 kDa
55 kDa
40 kDa
C
CIAC001-P1 66 proteins
CIAC001-P2 30 proteins
53
13
17
13 common proteins
3 glycolysis related proteins:
Pyruvate kinase PKM
Phosphoglycerate kinase 1
Alpha-enolase
3 Elongation factors
2 T-complex proteins
2 Heat shock proteins
3 Other proteins

FIG. 4

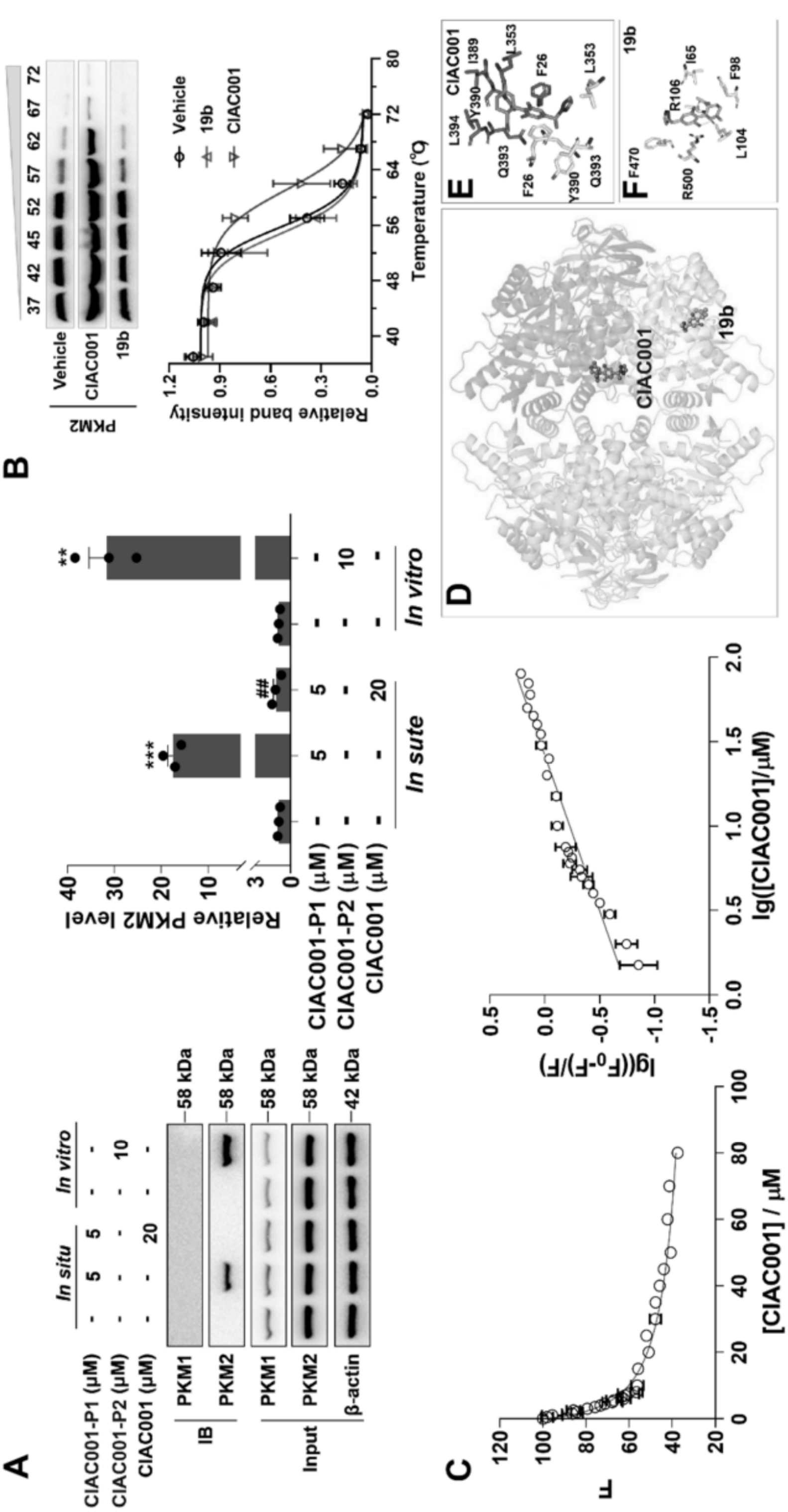
A
CIAC001-P1 (μM)
CIAC001-P2 (μM)
CIAC001 (μM)
In situ
In vitro
IB
PKM1
PKM2
Input
β-actin
58 kDa
42 kDa
Relative PKM2 level
In sute
In vitro
B
Temperature (℃)
Vehicle
CIAC001
19b
Relative band intensity
Temperature (℃)
C
F
[CIAC001] / μM
lg((F0-F)/F)
lg([CIAC001]/μM)
D
E
F

FIG. 5

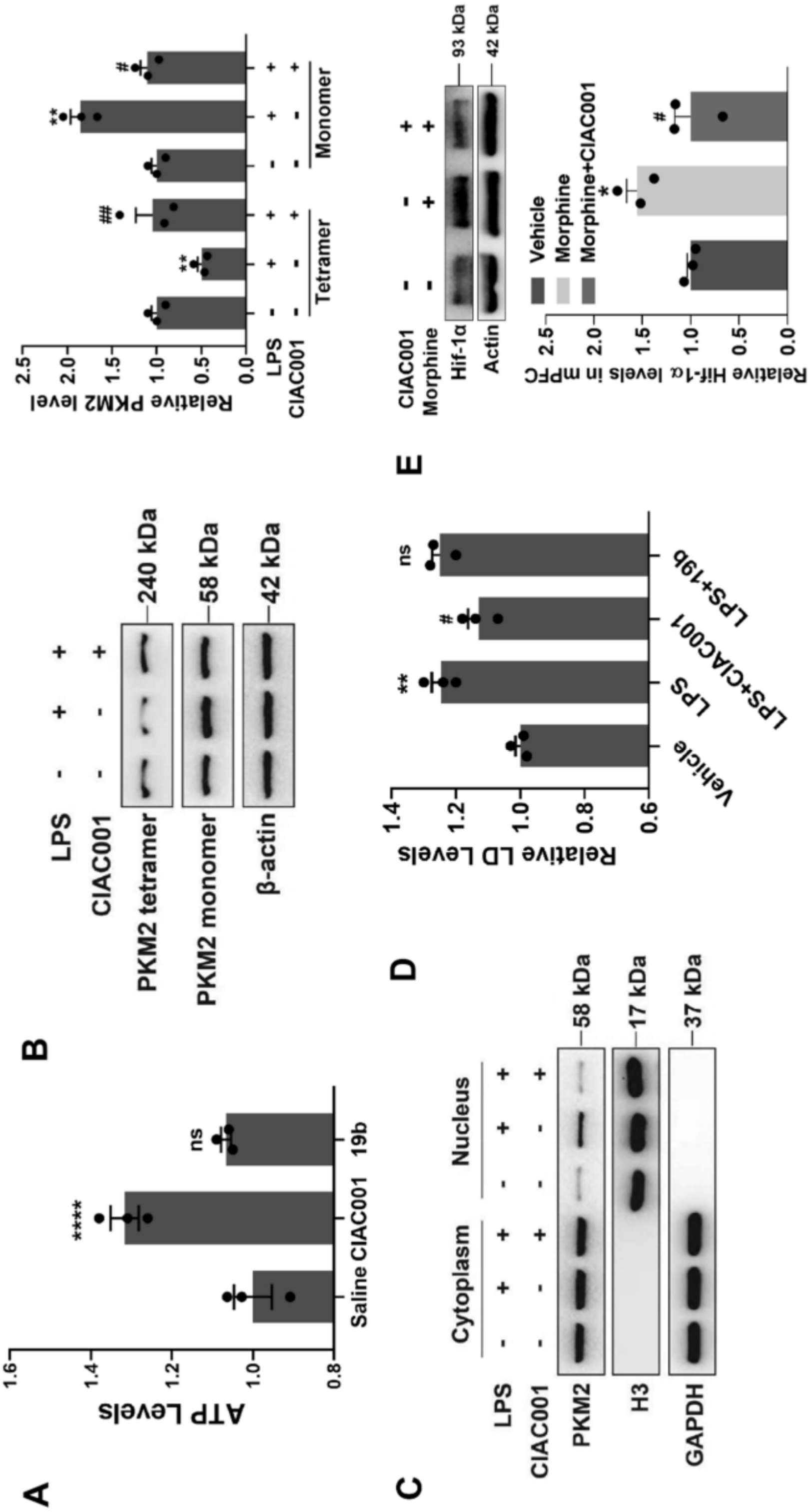
A
ATP Levels
Saline
CIAC001
19b
****
ns
B
LPS
CIAC001
PKM2 tetramer
PKM2 monomer
β-actin
240 kDa
58 kDa
42 kDa
Relative PKM2 level
Tetramer
Monomer
C
Cytoplasm
Nucleus
PKM2
H3
GAPDH
58 kDa
17 kDa
37 kDa
D
Relative LD Levels
Vehicle
LPS
LPS+CIAC001
LPS+19b
E
Morphine
Hif-1α
Actin
93 kDa
42 kDa
Vehicle
Morphine
Morphine+CIAC001
Relative Hif-1α levels in mPFC

FIG. 6

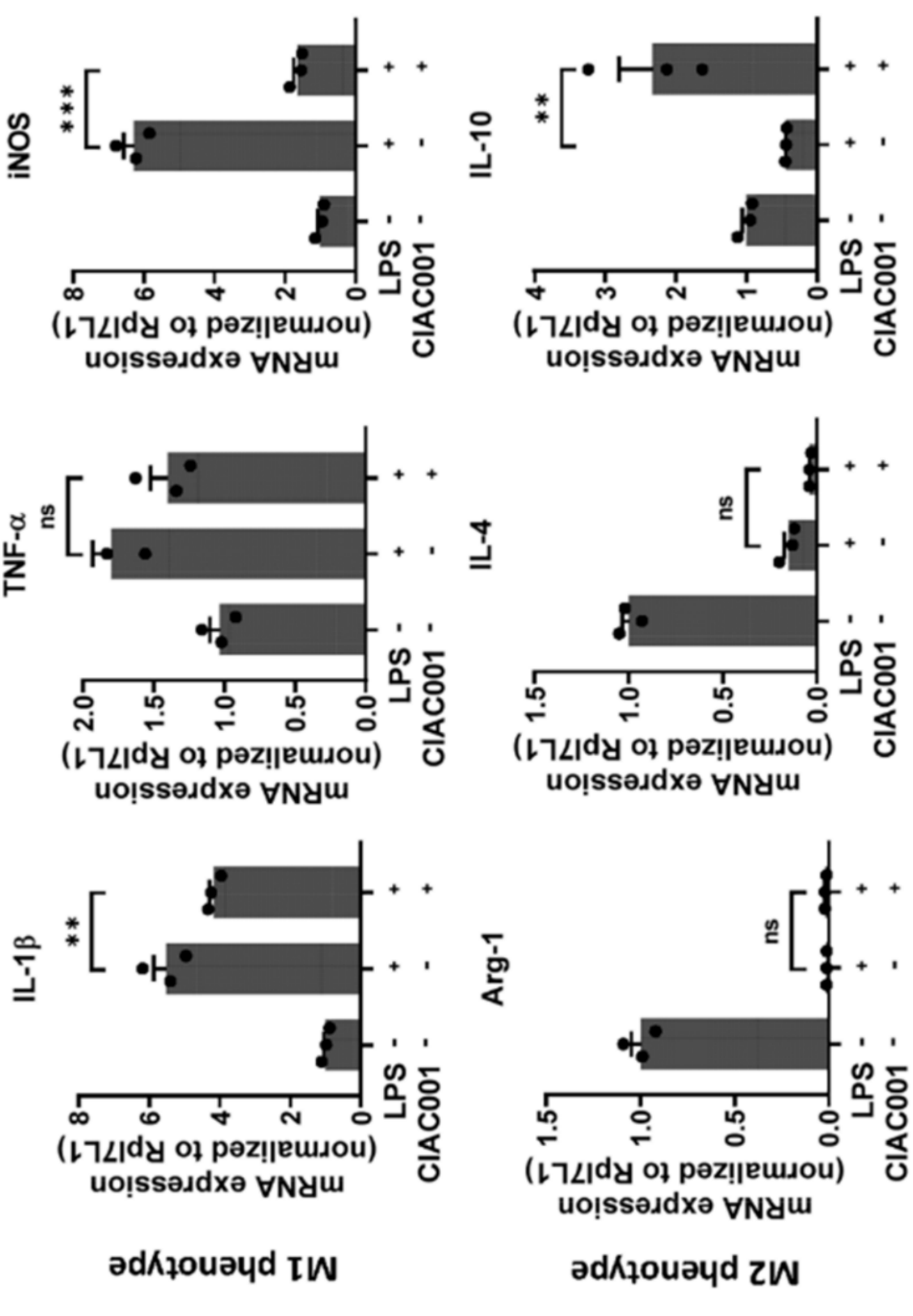
M1 phenotype
M2 phenotype
IL-1β
TNF-α
iNOS
Arg-1
IL-4
IL-10
mRNA expression (normalized to Rpl7L1)
LPS
CIAC001
ns
**
***

FIG. 7

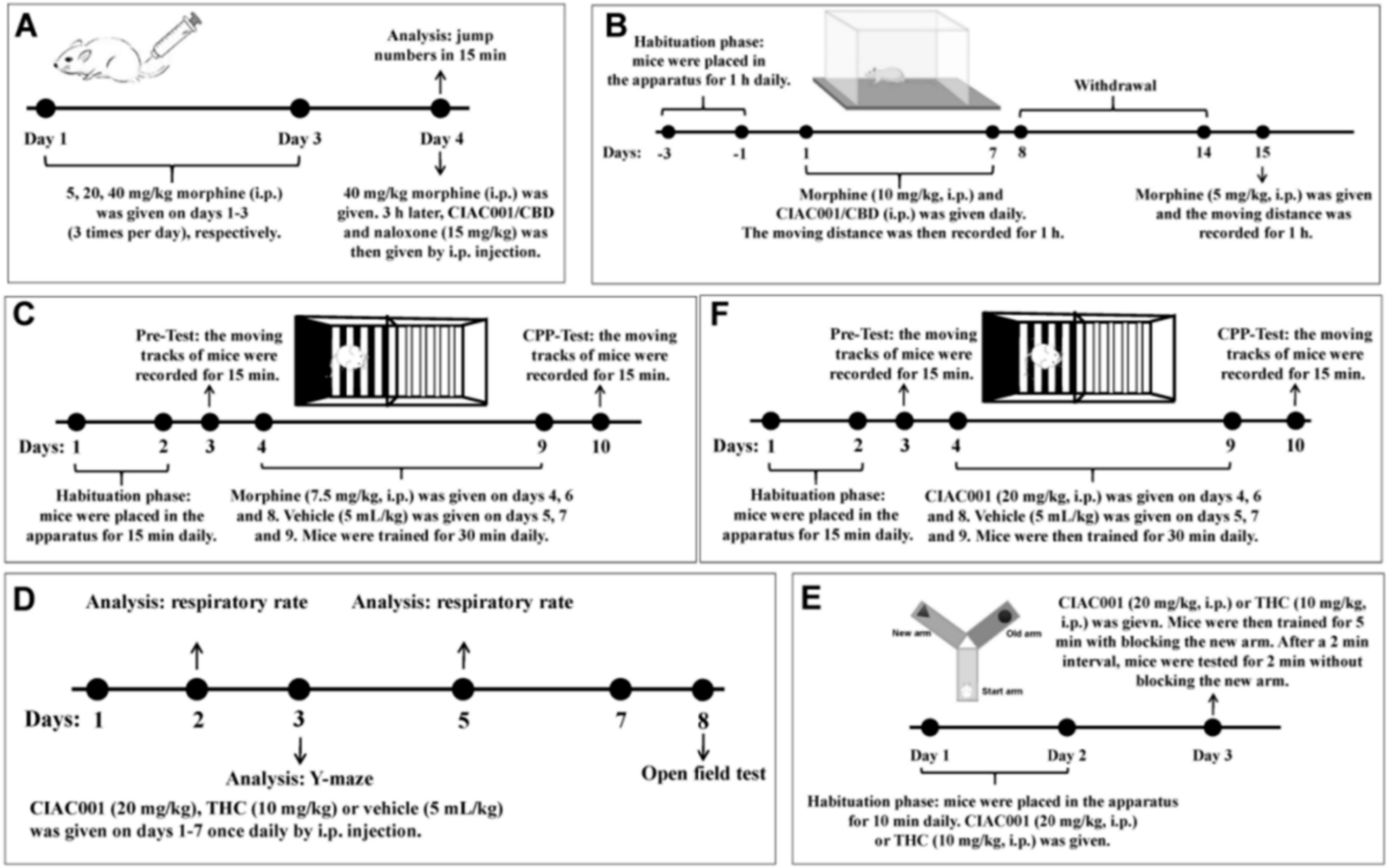
A
Analysis: jump numbers in 15 min
Day 1
Day 3
Day 4
5, 20, 40 mg/kg morphine (i.p.) was given on days 1-3 (3 times per day), respectively.
40 mg/kg morphine (i.p.) was given. 3 h later, CIAC001/CBD and naloxone (15 mg/kg) was then given by i.p. injection.
B
Habituation phase: mice were placed in the apparatus for 1 h daily.
Withdrawal
Days: -3 -1 1 7 8 14 15
Morphine (10 mg/kg, i.p.) and CIAC001/CBD (i.p.) was given daily. The moving distance was then recorded for 1 h.
Morphine (5 mg/kg, i.p.) was given and the moving distance was recorded for 1 h.
C
Pre-Test: the moving tracks of mice were recorded for 15 min.
CPP-Test: the moving tracks of mice were recorded for 15 min.
Days: 1 2 3 4 9 10
Habituation phase: mice were placed in the apparatus for 15 min daily.
Morphine (7.5 mg/kg, i.p.) was given on days 4, 6 and 8. Vehicle (5 mL/kg) was given on days 5, 7 and 9. Mice were trained for 30 min daily.
F
Pre-Test: the moving tracks of mice were recorded for 15 min.
CPP-Test: the moving tracks of mice were recorded for 15 min.
Days: 1 2 3 4 9 10
Habituation phase: mice were placed in the apparatus for 15 min daily.
CIAC001 (20 mg/kg, i.p.) was given on days 4, 6 and 8. Vehicle (5 mL/kg) was given on days 5, 7 and 9. Mice were then trained for 30 min daily.
D
Analysis: respiratory rate
Analysis: respiratory rate
Days: 1 2 3 5 7 8
Analysis: Y-maze
Open field test
CIAC001 (20 mg/kg), THC (10 mg/kg) or vehicle (5 mL/kg) was given on days 1-7 once daily by i.p. injection.
E
New arm
Old arm
Start arm
CIAC001 (20 mg/kg, i.p.) or THC (10 mg/kg, i.p.) was gievn. Mice were then trained for 5 min with blocking the new arm. After a 2 min interval, mice were tested for 2 min without blocking the new arm.
Day 1
Day 2
Day 3
Habituation phase: mice were placed in the apparatus for 10 min daily. CIAC001 (20 mg/kg, i.p.) or THC (10 mg/kg, i.p.) was given.

FIG. 8

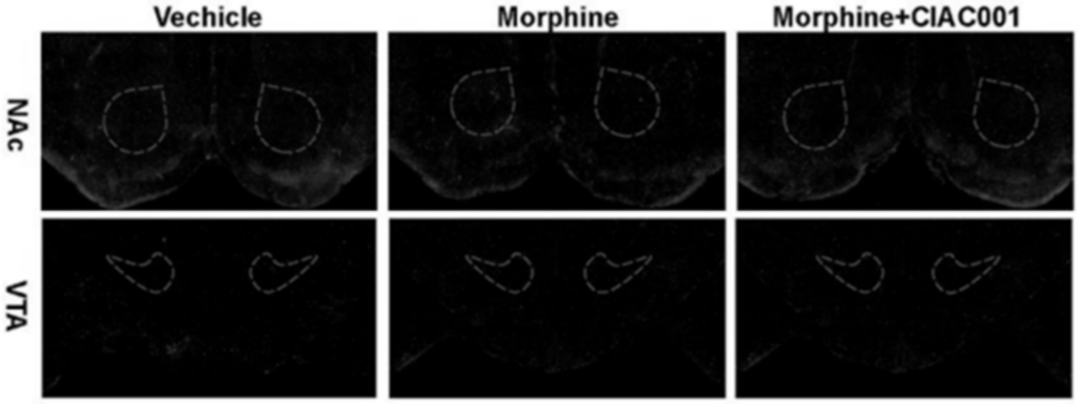


FIG. 9A

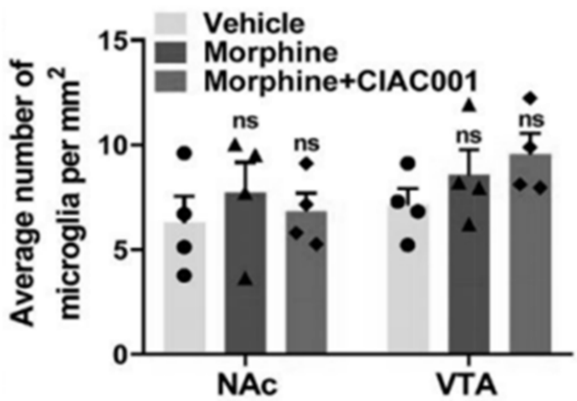


FIG. 9B

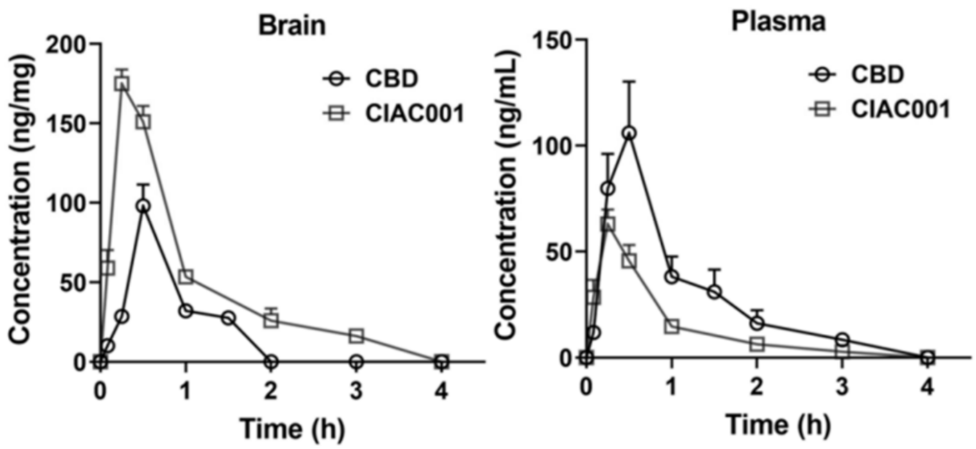


FIG. 10

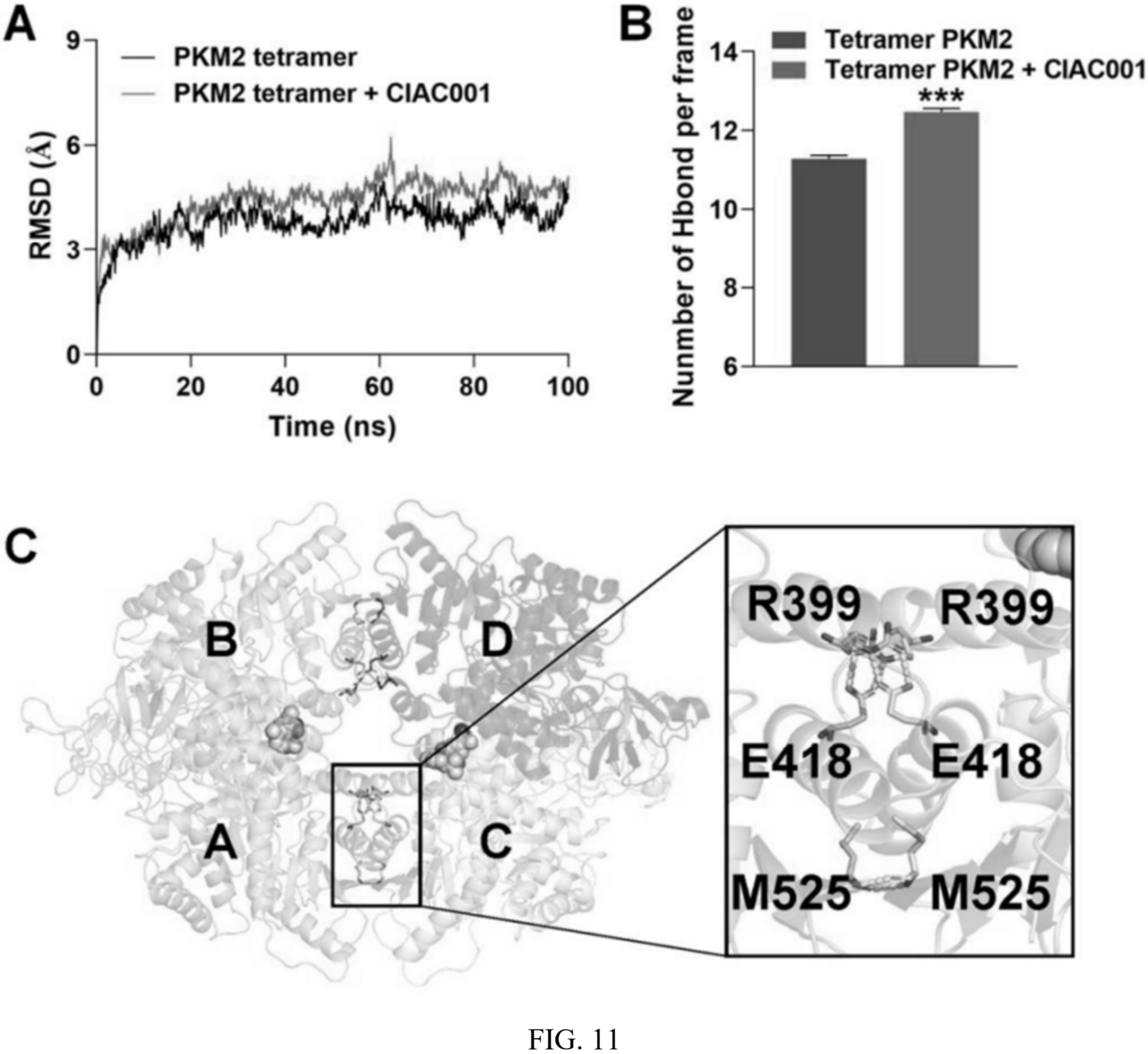
A
9
6
3
0
0
20
40
60
80
100
RMSD (Å)
Time (ns)
PKM2 tetramer
PKM2 tetramer + CIAC001
B
Tetramer PKM2
Tetramer PKM2 + CIAC001
***
14
12
10
8
6
Nunmber of Hbond per frame
C
B
D
A
C
R399
R399
E418
E418
M525
M525

FIG. 11

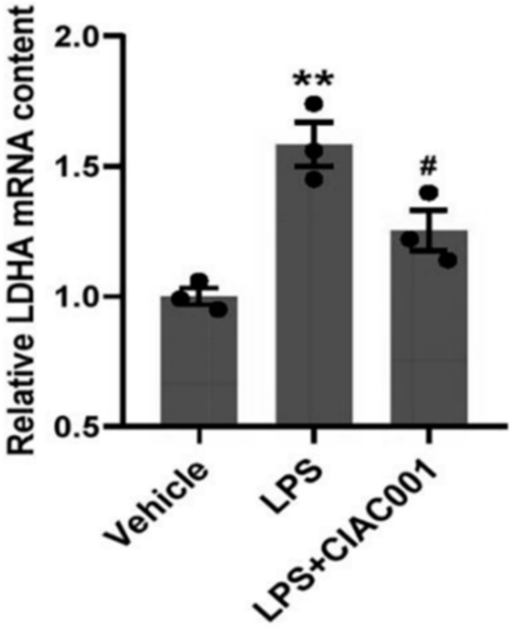
Relative LDHA mRNA content
2.0
1.5
1.0
0.5
**
#
Vehicle
LPS
LPS+CIAC001

FIG. 12

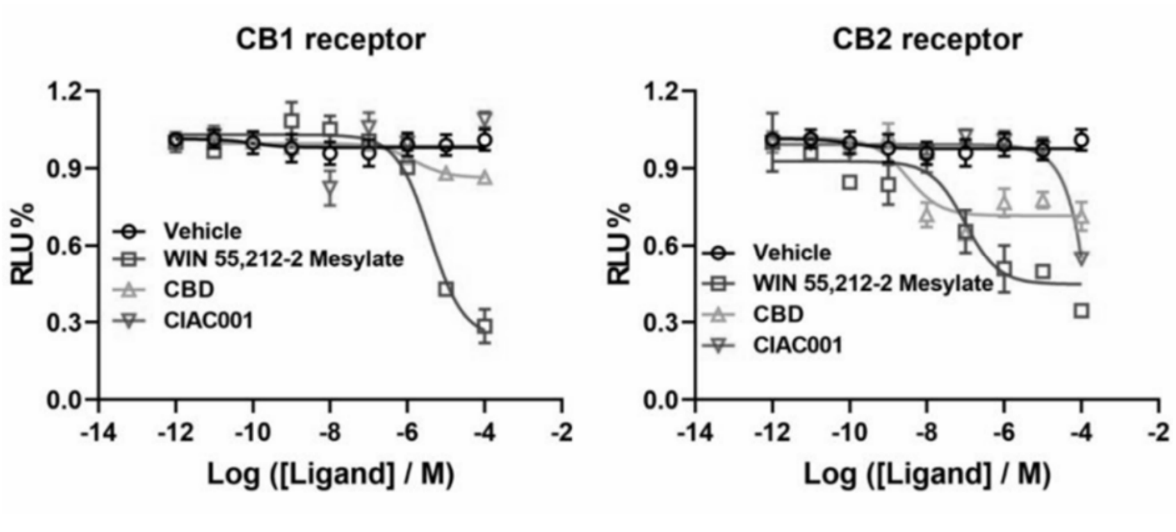
CB1 receptor
CB2 receptor
RLU%
1.2
0.9
0.6
0.3
0.0
Vehicle
WIN 55,212-2 Mesylate
CBD
CIAC001
-14
-12
-10
-8
-6
-4
-2
Log ([Ligand] / M)

FIG. 13

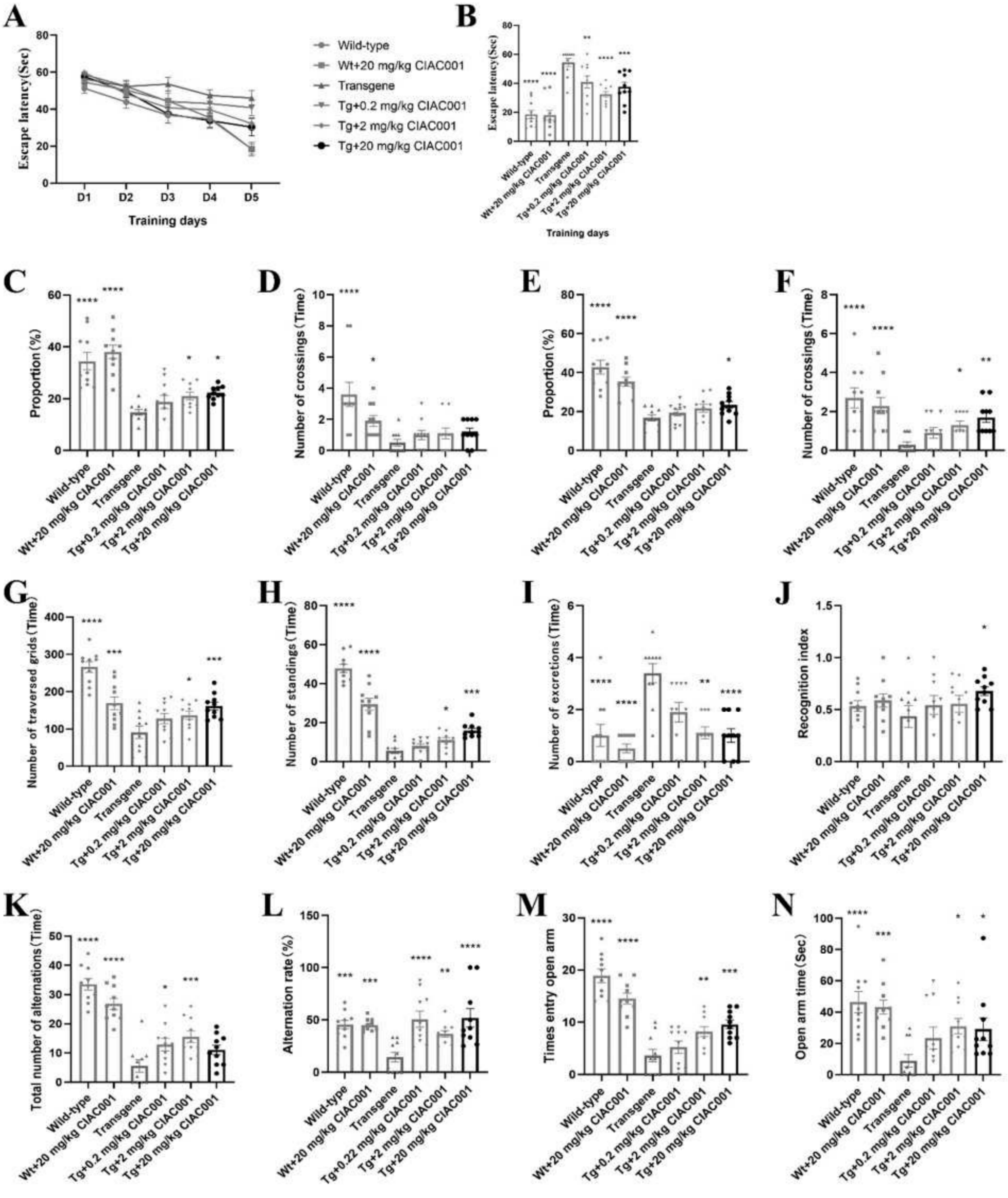
A
Escape latency(Sec)
Training days
D1
D2
D3
D4
D5
Wild-type
Wt+20 mg/kg CIAC001
Transgene
Tg+0.2 mg/kg CIAC001
Tg+2 mg/kg CIAC001
Tg+20 mg/kg CIAC001
B
Escape latency(Sec)
Training days
C
Proportion (%)
D
Number of crossings (Time)
E
Proportion (%)
F
Number of crossings (Time)
G
Number of traversed grids (Time)
H
Number of standings (Time)
I
Number of excretions (Time)
J
Recognition index
K
Total number of alternations (Time)
L
Alternation rate (%)
Tg+0.22 mg/kg CIAC001
M
Times entry open arm
N
Open arm time (Sec)

FIG. 14

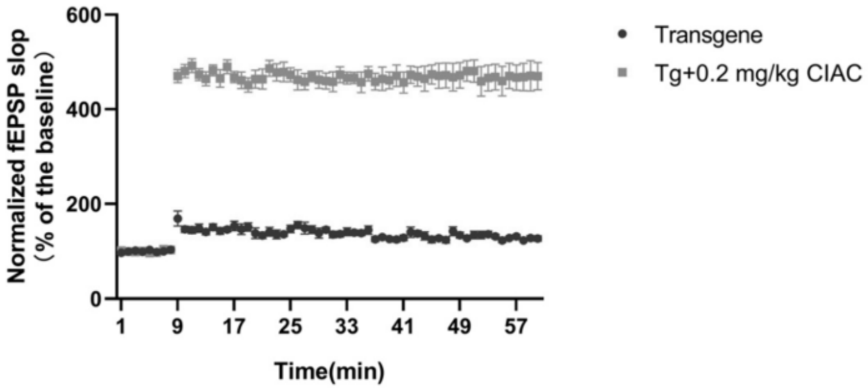


FIG. 15A

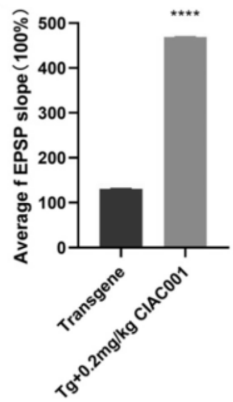


FIG. 15B

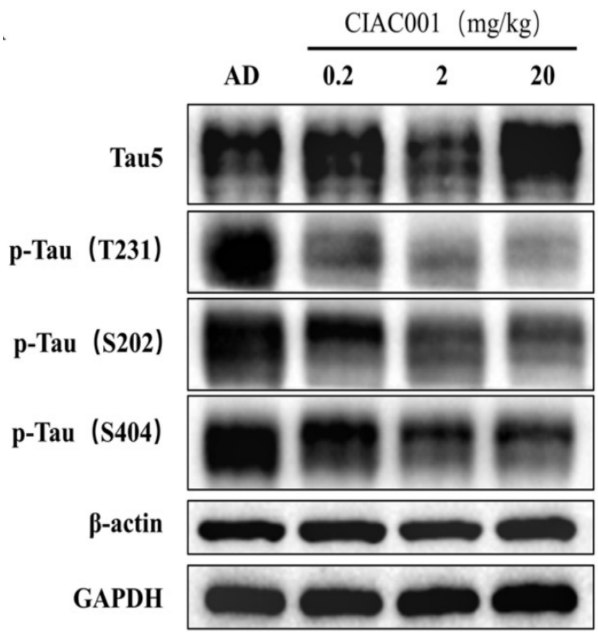


FIG. 15C

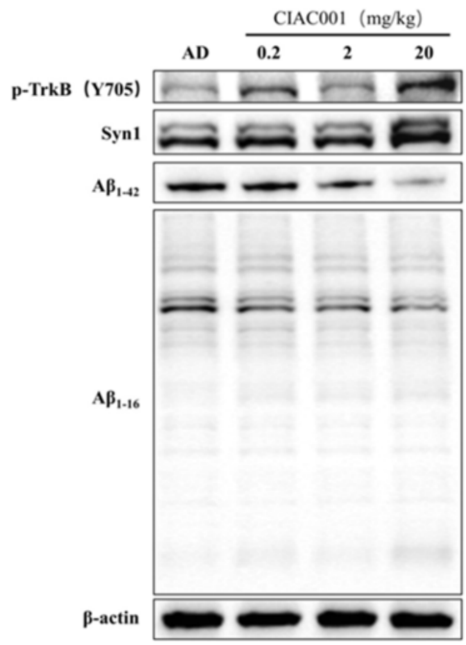


FIG. 15D

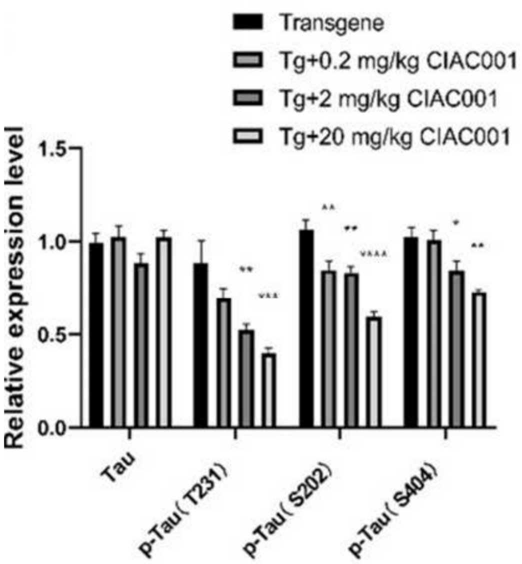


FIG. 15E

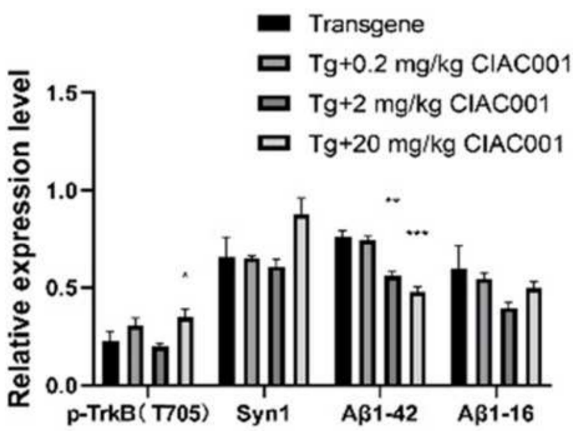


FIG. 15F

**INTERNATIONAL SEARCH REPORT**

International application No.
**PCT/CN2024/090415**

**A. CLASSIFICATION OF SUBJECT MATTER**

A61K 31/4192(2006.01)i; C07D 207/325(2006.01)i; C07D 233/60(2006.01)i; C07D 249/04(2006.01)i; A61P25/30(2006.01)i; A61P 25/28(2006.01)i; A61P 25/22(2006.01)i; A61P25/00(2006.01)i; A61P29/00(2006.01)i

According to International Patent Classification (IPC) or to both national classification and IPC

**B. FIELDS SEARCHED**

Minimum documentation searched (classification system followed by classification symbols)

IPC: A61K; C07D; A61P

Documentation searched other than minimum documentation to the extent that such documents are included in the fields searched

Electronic data base consulted during the international search (name of data base and, where practicable, search terms used)

CNABS, CNTXT, DWPI, WPABS, ENTXT, STNext, ISI Web of Science, PUBMED, 百度学术, Baidu Scholar, 中国期刊全文数据库, Chinese Journal Full-text Database, 读秀学术, Duxiu Scholar: 阿片, 成瘾, 戒断, 吗啡, 大麻二酚, 胶质细胞, 神经退行性疾病, 阿尔茨海默, 极化, 转化, 丙酮酸激酶, 抗炎, addictive, withdrawal, Opioids, morphine, CBD, Alzheimer, microglial, M1, M2, polarization, phenotype, conversion, neurodegenerative, inflammation, PKM, CIAC001, 2649154-82-3

**C. DOCUMENTS CONSIDERED TO BE RELEVANT**

| Category* | Citation of document, with indication, where appropriate, of the relevant passages | Relevant to claim No. |
|---|---|---|
| PX | JIN S. et al. "Cannabidiol Analogue CIAC001 for the Treatment of Morphine-Induced Addiction by Targeting PKM2" *Journal of Medicinal Chemistry,* Vol. vol. 66, 02 August 2023 (2023-08-02), pages 11498-11516 | 1-34 |
| PY | JIN S. et al. "Cannabidiol Analogue CIAC001 for the Treatment of Morphine-Induced Addiction by Targeting PKM2" *Journal of Medicinal Chemistry,* Vol. vol. 66, 02 August 2023 (2023-08-02), pages 11498-11516 | 35-62 |
| PX | CN 117338773 A (CHANGCHUN INSTITUTE OF APPLIED CHEMISTRY, CHINESE ACADEMY OF SCIENCES) 05 January 2024 (2024-01-05) claims 1-10 | 31-33, 35-62 |
| X | CN 112898190 A (CHANGCHUN INSTITUTE OF APPLIED CHEMISTRY, CHINESE ACADEMY OF SCIENCES) 04 June 2021 (2021-06-04) description, paragraph [0206] | 34 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 August 2024** | **22 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/090415**

**C. DOCUMENTS CONSIDERED TO BE RELEVANT**

| Category* | Citation of document, with indication, where appropriate, of the relevant passages | Relevant to claim No. |
|---|---|---|
| A | CN 112898190 A (CHANGCHUN INSTITUTE OF APPLIED CHEMISTRY, CHINESE ACADEMY OF SCIENCES) 04 June 2021 (2021-06-04)<br>claims 1-10, and description, paragraphs [0005] and [0016]-[0018] | 1-33, 35-62 |
| Y | 李程 等 (LI, Cheng et al.). "Warburg效应与神经退行性疾病的关系 (Non-official translation: The Relationship between the Warburg Effect and Neurodegenerative Diseases)"<br>*神经损伤与功能重建 (Neural Injury and Functional Reconstruction),*<br>Vol. 13, No. (3), 31 March 2018 (2018-03-31),<br>pages 149-152 | 35-62 |
| A | 潘三强 (PAN, Sanqiang). "胶质细胞与药物成瘾 (Glial Cells and Drug Addiction)"<br>*暨南大学学报(医学版) (Journal of Jinan University (Natural Science & Medicine Edition)),*<br>Vol. 32, No. (6), 31 December 2011 (2011-12-31),<br>pages 562-566 | 1-30 |
| A | MARTIRE S. et al. "Bioenergetic Impairment in Animal and Cellular Models of Alzheimer's Disease: PARP-1 Inhibition Rescues Metabolic Dysfunctions"<br>*Journal of Alzheimer's Disease,* Vol. vol. 54, 31 December 2016 (2016-12-31),<br>pages 307-324 | 35-62 |
| A | US 20200271672 A1 (UNIVERSITÉ LAVAL) 27 August 2020 (2020-08-27)<br>claims 45-64 | 1-62 |

Form PCT/ISA/210 (second sheet) (July 2022)

**INTERNATIONAL SEARCH REPORT**

International application No.
**PCT/CN2024/090415**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-62**
because they relate to parts of the international application that do not comply with the prescribed requirements to such an extent that no meaningful international search can be carried out, specifically:

The technical solutions of claims 1-62 comprise a method for treating or preventing a disease, and therefore said claims do not comply with PCT Rule 39.1(iv). The search report is provided on the basis that the designation of the subject matter thereof is a corresponding pharmaceutical use.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/090415**

| Patent document cited in search report | | | Publication date (day/month/year) | Patent family member(s) | | | Publication date (day/month/year) |
|---|---|---|---|---|---|---|---|
| CN | 117338773 | A | 05 January 2024 | None | | | |
| CN | 112898190 | A | 04 June 2021 | None | | | |
| US | 20200271672 | A1 | 27 August 2020 | CA | 3056085 | A1 | 16 August 2018 |
| | | | | WO | 2018145211 | A1 | 16 August 2018 |
| | | | | US | 11662352 | B2 | 30 May 2023 |
| | | | | US | 2023408532 | A1 | 21 December 2023 |

Form PCT/ISA/210 (patent family annex) (July 2022)